# EUROPEAN PATENT APPLICATION

(11) **EP 4 019 539 A1**
(43) Date of publication of application: **29.06.2022**
(21) Application number: 20383142.5
(22) Date of filing: 22.12.2020
(51) Int. Cl.: C07K 16/00, C12N 15/62, C12N 15/70, A61P 35/00, C07K 14/21, C07K 14/24, C07K 16/28

(54) **RECOMBINANT BACTERIUM AND USES THEREOF**

(71) Applicant: Consejo Superior de Investigaciones Científicas (CSIC), 28006 Madrid (ES)
(72) Inventor: ASENSIO CALAVIA, Alejandro, 28049 Madrid (ES); ÁLVAREZ GONZÁLEZ, Beatriz, 28049 Madrid (ES); FERNÁNDEZ HERRERO, Luis Ángel, 28049 Madrid (ES)
(74) Representative: Pons

(57) **Abstract**

The invention relates to a recombinant bacterium and uses thereof. In particular, it relates to the use of recombinant bacteria to translocate cargo proteins into the cytosol of target cells. Said recombinant bacteria comprises a T3 secretion system under the control of a genetic regulatory circuit and a targeting module which allow the recombinant bacteria to target specific cells, adhere to these and inject their cargo into the cytosol of the target cells.

## Description

The invention relates to a recombinant bacterium and uses thereof. In particular, it relates to the use of recombinant bacteria to translocate cargo proteins into the cytosol of target cells.

### BACKGROUND ART

The revolution that the synthetic biology field has experienced in the last decade has allowed living organisms to be rationally programmed as biomachines for harnessing health, environmental or agricultural global problems. The unique and dynamic possibilities of biological entities make them interesting therapeutic agents to address disease states. Microbiome organisms that reside in our bodies, and specifically bacteria, have a tremendous potential to impact our health status. The design and incorporation of engineered functional modules in a safe bacterial chassis by synthetic biology methods enables a la carte design of sophisticated new therapies.

The major uses of such programmed biomachines have been drug delivery to the cytosol of cells/organisms, vaccination and immunotherapy. The document WO2012012872 discloses the use of recombinant bacterium expressing an antigen that is translocated to the cytosol of a host organism, wherein said antigen is fused with a translocation domain from a Type III secretion system (T3SS). The delivery of the antigen elicits an immune response from the host.

Another example of such biomachines is describe in the document US201540037A1 where bacterial minicells comprising the T3SS machinery are used to secrete a compound of interest. Said minicells are used as a vaccine delivery system, specifically for the treatment of cancer and inflammatory diseases.

The document WO20181294A4 discloses engineered microorganisms that produce one or more anti-cancer effector molecules with effects that range from the immune activation and priming to immune augmentation, T cell expansion and stromal modulation.

Finally, the document WO2008110653 discloses microorganisms engineered to produce, secrete and inject single-domain recombinant antibodies into eukaryotic cells.

Said microorganisms comprise a T3SS system that allows the injection of the antibodies into the cytosol of the eukaryotic cells.

Despite the recent advances, most attempts to use engineered microorganisms for the delivery of effector molecules as vaccines or for immunotherapy rely on attenuated pathogenic bacteria that express an injector system (such as T3SS). Said bacteria, despite being attenuated, may lead to undesirable immune responses. To counteract these problems, bacteria where replication has been abolished or minicells derived from replication defective bacteria have been used. However, said bacteria and/or minicells do not totally avoid undesirable immune responses due to pathogenesis; additionally, they are easily eliminated from the host system due to the lack of replication.

In an attempt to partially solve the above problems, nonpathogenic microorganisms have been engineered to assemble fully working delivery systems of effector compounds. Albeit a step in the right direction, these systems lack a tight genetic control of expression and production of the delivery system, leading to leak expression of the delivery system and, by consequence, leaky delivery of the effector proteins. Furthermore, these systems do not allow the targeted delivery of the effectors compounds to the cells/organisms of interest.

### SUMMARY OF THE INVENTION

The present invention relates to novel recombinant microorganisms that allow the targeted delivery of cargo proteins to eukaryotic cells with a tight control of the expression and production of the delivery machine in the microorganisms, thereby avoiding leaky delivery of the cargo proteins and solving the disadvantages presented by the previous systems known in the art. Said microorganisms are, therefore, suitable for use in the treatment of several diseases, including but not limited to immunodeficiencies, cancer and inflammatory diseases, as well as for use in vaccination and drug delivery.

Therefore, a first aspect of the present invention relates to a recombinant gram-negative bacterial strain comprising: (i) a type III protein secretion system (T3SS); (ii) at least one cargo component, wherein the cargo component comprises a secretion signal (SS) region recognized by the T3SS system and a cargo protein; (iii) an inducible genetic regulatory circuit to regulate the transcription of (i) and (ii); and (iv) at least one synthetic adhesin (SA) driving adhesion of the recombinant bacterial strain to a target cell, wherein the strain is capable of adhering specifically the target cell and subsequently injecting the cargo protein into said cell.

The terms "recombinant bacterium" or "recombinant microorganism" as used herein refer to a bacterium or microorganism that has been genetically altered or engineered; such genetic engineering may comprise the inclusion of one or more foreign nucleic acids into the bacterium or microorganism. The terms "recombinant" and "engineered" are considered equivalent within this context i.e. when referring to a bacterium or microorganism.

The term "gram-negative bacteria" as used herein refers to the standard meaning of the term i.e. bacteria that do not retain the crystal violet stain used in the gram-staining method of bacterial differentiation, as accepted in the field of bacterial taxonomy (Boone D.R. et al. Bergey's Manual^{®} of Systematic Bacteriology. Springer, New York, NY).

In some embodiments of the invention the bacterial strain of the invention is a nonpathogenic bacterial strain.

The term "nonpathogenic bacterial strain" as used herein refers to bacteria that are not capable of causing disease or harmful responses in a host. In some embodiments, nonpathogenic bacteria do not contain toxic lipopolysaccharides (LPS). In some embodiments, the nonpathogenic bacteria are commensal bacteria. In some embodiments, the nonpathogenic bacteria are probiotic bacteria.

Non-pathogenic bacteria may be genetically engineered to enhance or improve desired biological properties, e.g., survivability or probiotic properties.

The terms "commensal" or "commensal bacteria" as used herein refers to all types of bacteria living on or in an individual without causing a pathologic reaction. According to this definition the term comprises all commensal bacteria despite their potential to be pathogenic.

The term "probiotic" as used herein refers to live, non-pathogenic microorganisms, e.g., bacteria, which can confer health benefits to a host organism that contains an appropriate amount of the microorganism. In some embodiments, the host organism is a mammal. Some species, strains, and/or subtypes of non-pathogenic bacteria are currently recognized as probiotic bacteria. Examples of probiotic bacteria include, but are not limited to certain strains belonging to the genus *Bifidobacteria*, *Escherichia* and *Lactobacillus*, such as *Bifidobacterium bifidum, Enterococcus faecium, Escherichia coli* strain Nissle 1917, *Lactobacillus acidophilus, Lactobacillus bulgaricus, Lactobacillus paracasei* and *Lactobacillus plantarum.* As stated above, these bacteria may be engineered to enhance their probiotic properties.

In some embodiments of the invention the nonpathogenic, gram-negative bacteria strain is selected from a genus group consisting of: *Enterobacter, Pseudomonas, Bacteroides, Escherichia* and *Lactococcus.* In some embodiments the nonpathogenic gram-negative bacterial strain is selected from a species group consisting of: *Bacteroides fragilis, Bacteroides subtilis, Bacteroides thetaiotaomicron.* In some preferred embodiments of the invention the nonpathogenic gram-negative bacterial strain is *Escherichia coli.* In certain specific embodiments of the invention the *E. coli* strain is selected from the group consisting of: *Escherichia coli* K-12 MG1655, *Escherichia coli* Nissle 1917, *Escherichia coli* ABU 83972, *Escherichia coli* DSM17252 and *Escherichia coli* Colinfant AO 34/86.

The bacterial strain of the invention comprises a bacterial secretion system or injectosome. Bacterial secretion systems are protein complexes present on the cell membranes of pathogenic bacteria; some of these bacteria use the secretion systems to secrete their virulence factors (mainly proteins) to invade their host cells. Generally, the virulence factors can be secreted through two different processes. The first process is a one-step mechanism in which the virulence factors from the cytoplasm of bacteria are transported and delivered directly through the cell membrane into the host cell. The second process involves a two-step activity in which the virulence factors are first transported out of the inner cell membrane, then deposited in the periplasm, and finally through the outer cell membrane into the host cell.

The system used in the present invention is the type 3 secretion system or T3SS.

The T3SS is a bacterial nanomachine comprising a protein complex capable of translocating proteins into eukaryotic host cells in a one-step export mechanism across the bacterial and eukaryotic membranes. The core components of the T3SS are shared with the bacterial flagellum. The T3SS is a large transmembrane complex that bridges the space to the target cell with a hollow extracellular needle and consists of (i) an extracellular needle formed by helical polymerization of a small protein and terminated by a pentameric tip structure, (ii) a series of membrane rings that span both bacterial membranes and embed (iii) the export apparatus, formed by five highly conserved hydrophobic proteins thought to gate the export process, and (iv) a set of essential cytosolic components, also termed "sorting platform", which cooperate in substrate selection and export. The set of essential cytosolic T3SS components form a highly dynamic interface, in which the components permanently exchange between the T3SS and the cytosol.

The T3SS is present in some of the most virulent bacteria, including but not limited to bacterial strains of *Shigella, Salmonella, Vibrio, Burkholderia, Yersinia, Chlamydia, Pseudomonas,* and enteropathogenic and enterohemorrhagic *E. coli strains.* In some embodiments of the invention the T3SS of the invention is thus selected from the group consisting of the T3SS of: *Pseudomonas aeruginosa, Yersinia spp., Salmonella spp., Shigella spp., Chlamydia spp., P. syringae, Rhizobium spp.* and enteropathogenic and enterohemorrhagic *E. coli* strains.

In some embodiments the bacterial strain of the invention comprises nucleotide sequences encoding the polypeptide components of the T3SS of an enteropathogenic or enterohemorrhagic *E. coli* strain. In some specific embodiments, the nucleotide sequences are selected from the group consisting of: EspD (UniProt B7UM93), EspB (UniProt Q05129), EspA (UniProt B7UM94), EscF (UniProt B7UM90), EscC (UniProt B7UMB3), Escl (UniProt B7UMB0), EtgA (UniProt B7UMB7), EscJ (UniProt B7UMB1), EscD (UniProt B7UM96), EscR (UniProt B7UMC1), EscS (UniProt B7UMC0), EscT (UniProt B7UMB9), EscU (UniProt B7UMB8), EscV (UniProt B7UMA7), EscL (UniProt B7UMC2), EscN (UniProt B7UMA6), EscO (UniProt B7UMA5), SepQ (UniProt B7UMA3), EscK (UniProt B7UMC3), SepL (UniProt B7UM95), SepD (UniProt B7UMB2), CesL (UniProt B7UMA8), EscP (UniProt B7UMA4), EscG (UniProt B7UM89), EscE (UniProt B7UMC5), CesAB (UniProt B7UMC4), CesD (UniProt B7UMB4), CesD2 (UniProt B7UM91), CesF (UniProt B7UMA1), CesT (UniProt P21244), orf4 (UniProt Q9ZNF0), Ler (UniProt B7UMC6), escQ (UniProt Q7DB73), escG (UniProt Q9AJ07), espA (UniProt O33976), espB (UniProt Q8GC70), espD (UniProt 088127) and any variants or fragments thereof, in any combination.

In some specific embodiments of the invention the nucleotide sequences encoding the polypeptide components of the T3SS consist of: EspD (UniProt B7UM93), EspB (UniProt Q05129), EspA (UniProt B7UM94), EscF (UniProt B7UM90), EscC (UniProt B7UMB3), Escl (UniProt B7UMB0), EscJ (UniProt B7UMB1), EscD (UniProt B7UM96), EscR (UniProt B7UMC1), EscS (UniProt B7UMC0), EscT (UniProt B7UMB9), EscU (UniProt B7UMB8), EscV (UniProt B7UMA7), EscL (UniProt B7UMC2), EscN (UniProt B7UMA6), EscO (UniProt B7UMA5), SepL (UniProt B7UM95), SepD (UniProt B7UMB2), CesL (UniProt B7UMA8), EscP (UniProt B7UMA4), EscG (UniProt B7UM89), EscE (UniProt B7UMC5), CesAB (UniProt B7UMC4), CesD (UniProt B7UMB4), CesD2 (UniProt B7UM91), CesT (UniProt P21244), orf4 (UniProt Q9ZNF0) and escQ (UniProt Q7DB73).

As used herein the term "nucleotide sequence" refers to an oligonucleotide sequence or polynucleotide sequence, and variants, homologues, fragments and derivatives thereof. The nucleotide sequence may be DNA or RNA of genomic or synthetic or recombinant origin, and it may be double-stranded or single-stranded, whether representing the sense or antisense strand. Generally, the term "nucleotide sequence" as used herein refers to DNA; said DNA may be recombinant DNA i.e. it may have been prepared by use of recombinant DNA techniques.

The term "identity" or "sequence identity" as used herein refers to the degree of similarity between two nucleotide or amino acid sequences obtained by aligning the two sequences, i.e. the proportion of identical nucleotide or amino acids between two compared nucleotide sequences or polypeptides/proteins along their full-length sequence. Depending on the number of common residues between the aligned sequences, a different degree of identity, expressed as a percentage, will be obtained. The degree of identity between two amino acids or nucleotides sequences may be determined by conventional methods, for example, by standard sequence alignment algorithms known in the state of the art, such as BLAST [Altschul S.F. et al. Basic local alignment search tool. J Mol Biol. 1990 Oct 5; 215(3): 403-10]. The BLAST programs, for example, BLASTN, BLASTX, and T BLASTX, BLASTP and TBLASTN, are in the public domain at The National Center for Biotechonology Information (NCBI) website. The skilled person in the art understands that mutations in the nucleotide sequence of genes that lead to conservative amino acid substitutions at non-critical positions for the functionality of the protein are evolutionarily neutral mutations which do not affect the protein's global structure or functionality.

The term "nucleotide variant" as used herein refers to any nucleotide sequence having at least 85% sequence identity with a reference nucleotide sequence. Thus, the variant nucleotide has a nucleotide sequence with 85, 86, 87, 88, 89, 90, 91, 92, 93, 94, 95, 96, 97, 98, 99 or 100% sequence identity with the reference nucleotide sequence.

The term "polypeptide variant" as used herein refers to refers to a polypeptide wherein at least one amino acid residue differs from the reference amino acid sequence of said polypeptide; the difference may result from deletion, insertion, non-conservative or conservative substitution, or combinations thereof. Generally, the term refers to any polypeptide sequence having at least 85% sequence identity with a reference polypeptide sequence. Thus, the variant polypeptide has a polypeptide sequence with 85, 86, 87, 88, 89, 90, 91, 92, 93, 94, 95, 96, 97, 98, 99 or 100% sequence identity with the reference polypeptide sequence.

The variant polypeptide is preferably a functional equivalent that shows the same biological activity as the reference polypeptide. However, its physical and chemical properties may be modified as necessary. The terms peptide, polypeptide and protein are used interchangeably in the context of the present invention.

The nucleotide sequences that encode the polypeptide components of the T3SS of the invention may be organized in operons in the bacterial strain of the invention. The term "operon", as used herein, refers to a functional cluster of adjacent genes under the control of single regulatory signal, preferably a promoter. The term "promoter", as used herein, refers to a DNA region which contains an initial binding site for RNA polymerase and facilitates the transcription of a particular gene downstream thereof. That is, a promoter is an untranslated nucleotide sequence, upstream of a coding region, to which RNA polymerase binds to initiate the transcription of a gene, and is typically located near the genes it regulate, on the same strand and upstream (towards the 5' region of the sense strand).

Thus, in some embodiments, the nucleotide sequences that encode the polypeptide components of the T3SS of the invention comprise a promoter that drives transcription of the operon, wherein said promoter is a functional *E. coli* as disclosed in the state of the art (by way of non-limiting example, those listed in: http://parts.igem.org/Promoters/Catalog; https://biocyc.org/group?id=:ALL-PROMO TERS&orgid=ECOLI; https://ecocyc.org/site-search.shtml; http://margalit.huji.ac.il/ promec/ index.html).

In some embodiments, the nucleotide sequence encodes a promoter selected from the group consisting of: Ptac (IGEM: BBa_K864400), pR (IGEM: BBa_R0051), pPepT, FF+20, Pvgb (IGEM: BBa_K1555003), plldR(IGEM: BBa_K1847007), CadA (IGEM: BBa_K174015), CadC (IGEM: BBa_K112410), ArsS/R (IGEM: BBa_M50085), ttrRS (IGEM: BBa_K895007), hmp (IGEM: BBa_K216005), PnorV (IGEM: BBa_K1153000), OxyR (IGEM: BBa_K362001), SoxR/S (IGEM: BBa_K554003), Pbad (IGEM: BBa_10500), Ptet (IGEM: BBa_J01101), XylS (IGEM: BBa_J61051)

In some preferred embodiments the promoter is the Tac promoter (Ptac) (IGEM: BBa_K864400) and the nucleotide sequence encoding it consists of SEQ ID NO: 6.

The bacterial strain of the invention comprises at least one cargo component, from here onwards the cargo component of the invention, wherein the at least one cargo component comprises a secretion signal (SS) region recognized by the T3SS system and a cargo protein.

The term "cargo protein" a used herein refers to any proteins that are transported or translocated trough the T3SS into the cytosol of the target cells and thus includes any (natural) effector proteins of T3SS as defined herein.

The nucleotide sequences that encode the at least one cargo component comprise (i) a first nucleotide sequence encoding a secretion signal (SS) region which the T3SS system recognizes in order to export any effector proteins comprising said SS, fused to (ii) a second nucleotide sequence encoding a cargo protein. While the SS are only well-defined and empirically characterized in some of the T3SS effector proteins, they are usually located near the N-terminal end of the effector proteins and comprise the first 15-130 amino acids of said proteins. The SS region of the cargo component of the invention is located near or at the N-terminal end of the effector T3SS protein and comprises a number of amino acid residues of 1-100 (inclusive). The SS region of the cargo component of the invention may be selected from the group consisting of any of the SS regions of any natural T3SS effector proteins; in some specific embodiments, the SS region of the T3SS of the invention is any of the SS regions of any natural T3SS effector proteins of an enteropathogenic or enterohemorrhagic *E. coli* strain.

In some embodiments the nucleotide sequences that encode the cargo component of the invention comprise a nucleotide sequence encoding a secretion signal (SS) region selected from the group consisting of: Tir30 (SEQ ID NO: 7), Tir100 (SEQ ID NO:8) and EspF20 (SEQ ID NO:9) or variants or fragments thereof.

The term "protein fragment" as used herein refers to proteins having one or more (e.g., several) amino acids absent from the amino and/or carboxyl terminus of the amino acid sequence when compared to the reference amino acid sequence, wherein the fragment has the same activity as the reference protein.

The nucleotide sequences encoding the cargo component of the invention may further comprise a nucleotide sequence encoding a chaperone. The term "chaperone' as used herein refers to a protein that assists the cargo protein being translocated through the T3SS into the target cell cytosol, usually through the recognition of a specific binding site in the amino acid sequence of the cargo protein being translocated. Therefore, the nucleotide sequences encoding the cargo component of the invention may also comprise a nucleotide sequence encoding the binding site of the chaperone. In such cases, said nucleotide sequence is comprised within the nucleotide sequence encoding the SS i.e. the first nucleotide sequence. In some embodiments, the nucleotide sequences encoding the at least one cargo component comprise both a nucleotide sequence encoding a chaperone and a nucleotide sequence encoding the binding site for said chaperone.

The chaperone protein may be any suitable protein known in the art, and must be compatible with the chosen translocation domain. Preferably the chaperone is a type III secretion system chaperone. More preferably, the chaperone is the Tir chaperone CesT (UniProt P21244; SEQ ID NO: 10) and the nucleotide sequence encoding the secretion signal comprises the binding site for CesT present in the Translocated Intimin Receptor (Tir) (UniProt B7UM99), as previously described (Little DJ et al., 2018 PLoS Pathog. Aug 17; 14(8)).

The at least one cargo component of the invention comprises a cargo protein. The cargo protein may be selected from the group consisting of: a) Antibody fragments; b) Cytotoxins; c) Effector proteins of T3SS; d) Proteins inducing cell death; e) Prodrug converting enzymes; f) Immunogenic antigens; and g) Genetic reprogramming factors.

The term "antibody fragment" or "antibody fragments" as used herein, refers an a naturally occurring antibody which lacks on or more domains or one or more amino acids. Typically, an antibody fragment contains the entire antigen binding or variable region thereof of such naturally occurring antibody. Examples of antibody fragments include any antibody that lacks the or has no Fc portion. Examples of antibody fragments include also Fab, Fab', F(ab')2, Fv and scFv fragments; diabodies; triabodies; tetrabodies; minibodies; antibodies consisting essentially of a single, two or three immunoglobulin domain(s) such as Domain Antibodies^{™}; single-chain antibodies; bispecific, trispecific, tetraspecific or multispecific variants of any of the above. The term "antibody fragment" or "antibody fragments" as used herein also refers to camelid antibodies (e.g., from camels or llamas such as nanobodies) and derivatives thereof. Antibody fragments and the various techniques for their production are well known in the art (Holliger and Hudson, 2005; Glover and Humphreys, 2004). The term "antibody fragment" or "antibody fragments" as used herein, comprises human, humanized, primatized and chimeric antibody fragments.

The term "nanobody(ies)" as used herein refers to small sized entities (15 kDa) formed solely by the antigen binding region of the heavy chain (VH fragment) of immunoglobulins. Said nanobodies are mainly produced after immunizing animals of the Camelidae family (also called VHH fragments), such as camels, dromedaries, llamas, and alpacas; and also, of the shark family (also called VNAR fragments), which have the particularity of having antibodies which naturally lack the light chain (VL fragment) and recognize the antigen by the heavy chain variable domain. Nevertheless, the nanobodies derived from these sources may require a humanization process for their therapeutic application. Another potential source for obtaining similar single domain antibodies is from human antibodies by separating the VH and VL domains of the variable region and introducing amino acid substitutions found in camelid VHHs (Riechmann, L. et al 1999 J Immunol Methods 231(1-2): 25-38). Nanobodies present advantages such as a production cost reduction with respect to whole antibodies, stability and the reduction of immunogenicity.

The term "cytotoxins" as used herein refers to a compound which is capable of causing necrosis or apoptosis to a cell which is affected by the compound. In a preferred embodiment the cytotoxin is an anti-cancer cytotoxin. Examples thereof include enzyme genes capable of converting a nontoxic prodrug into a substance with cytotoxicity (for example, thymidine kinase catalyzing monophosphorylation of GCV so as to induce cellular death) and genes of various toxins directly damaging cells (for example, diphtheria toxin, tetanus toxin, cholera toxin). Further examples include antibiotics, tubulin polymerization inhibitors, alkylating agents that bind to and destroy DNA, and agents that destroy the functions or protein synthesis of essential cellular proteins such as protein kinases, phosphatase, topoisomerase, enzymes, and cyclins. The term "cytotoxins" also includes fragments of said cytotoxins, whose definition is identical to the previously describe protein fragment, as well as variants of said cytotoxins.

In some embodiments of the present invention the cargo protein, fragment or variant thereof is a cytotoxin selected from the group consisting of: thymidine kinase (UniProt Q9QNF7), Exotoxin A (UniProt A0AOH2Z8B0), TccC3 (UniProt Q8GF97), Diphteria toxin (UniProt Q6KE85), Cholera toxin (UniProt P01555 and P01556), Heat-labile enterotoxin (UniProt P06717 and P32890), Pertussis toxin (UniProt P04977, P04978, P04979, POA3R5, P04981), C2 (UniProt A5I4T4), Botulinum neurotoxin (UniProt P0DPI1, P10844), Tetanus toxin (UniProt P04958), Shiga toxin (UniProt Q9FBI2, Q7BQ98), Shiga-like toxin (UniProt P08026, P69179), Shiga-like toxin 2 (UniProt P09385, P09386), Ribonuclease alpha-sarcin (UniProt P00655) and Ricin (UniProt P02879). In preferred embodiments the cytotoxin is selected from the group consisting of ADP-ribosyltransferase (ART) toxin ExoA (UniProt A0A0H2Z8B0), Actin cross-linking toxin VgrG1 (UniProt Q9KS45); RTX Toxin (Uniprot Q9KS12); ADP-ribosyltransferase (ART) toxin TccC3 (UniProt Q8GF97); and variants or fragments thereof.

The term "effector proteins of T3SS" as used herein refers to proteins which are naturally injected by T3SS systems into the cytosol of eukaryotic cells and to proteins which are naturally secreted by T3S systems or that might form the translocation pore into the eukaryotic membrane (including pore-forming translocators, such as enteropathogenic or enterohemorrhagic *E. coli* EspB and EspD proteins. Proteins which are naturally injected by T3SS into the cytosol of eukaryotic cells are preferably used (for a non-exhaustive list see for example http://effectors.bic.nus.edu.sg/index.php)

In some embodiments of the present invention the effector proteins of T3SS are selected from a group consisting of any effector proteins of enteropathogenic or enterohemorrhagic *E. coli* strains and fragments or variants thereof.

In preferred embodiments of the present invention the effector protein of T3SS is selected from the group consisting of: EspH (UniProt A0A04515T0; Tir (UniProt B7UM99); NleC (UniProt Q8X834); Map (UniProt B7UMA0); and fragments or variants thereof.

The expression "proteins inducing cell death" as used herein refer to proteins that induce cell death or demise to cells, wherein cell death includes apoptosis and necrosis, including but not limited to fragments and/or variants thereof. In some embodiments of the present invention the protein inducing cell death is selected from the group consisting of: BID (UniProt P55957); BIM (UniProt 043521); Granzyme B (UniProt P10144); and any fragments or variant thereof.

The term "prodrug converting enzyme" as used herein refers to a protein that has a function of converting an inactive drug into an active drug through an enzymatic reaction. When using such a prodrug converting enzyme, the inactive drug is metabolized and converted into the active form in the target cells.

In some embodiments of the invention the prodrug converting enzyme is an anti-cancer prodrug converting enzyme. In a preferred embodiment of the invention, the prodrug converting enzyme is selected from the group consisting of: human herpesvirus-1 thymidine kinase (Uniprot Q9QNF7); *E. coli* cytosine deaminase (UniProt P25524); Carboxypeptidase G2 (Uniprot P06621); β-glucuronidase (Uniprot P08236); and any variants or fragments thereof.

The term "immunogenic antigens" as used herein refers to a substance that is recognized and bound specifically by an antibody or by a T cell antigen receptor, eliciting a cellular and/or humoral immune response.

In some embodiments of the invention the immunogenic antigen is a tumor-associated peptide or protein antigen selected from, but not limited to, the group consisting of: Survivin/Birc5 (Uniprot 015392); Tyrosinase (Uniprot P14679); TRP-1 (Uniprot P17643); TRP-2 (Uniprot P40126); Melan-A (Uniprot Q16655); gp100/Pmel17 (Uniprot P40967); Cancer-testis antigens (CTAs) (Uniprot O75638); NY-ESO-1 (Uniprot P78358); Melanoma associated antigens (MAGEs) (Uniprot P43355; P43356; P43357; P43358; P43364; P43365); B melanoma antigen-1 (BAGE) (Uniprot Q13072); and any variants or fragment thereof. In a preferred embodiment the immunogenic antigen is selected from the group consisting of: Survivin (Uniprot 015392); Ovolabumin (OVA) (UniProt P01012); and fragments or variants thereof.

The term "genetic reprogramming factors" as used herein refers to a compound/protein that is capable of inducing directly or indirectly the expression of morphological and/or functional characteristics of a desired cell type different from the cell type of the target cell. Preferred compounds include those capable of driving directly or indirectly transformation of the diseased target cell into a healthy or apoptotic/necrotic cell type. In preferred embodiment, the reprogramming agent is selected for inducing a direct or indirect endogenous expression of at least one gene regulator as defined herein. There are many compounds that may be helpful in reprogramming a cell according to the invention and these compounds can be used alone or in combinations. For example, the compound may be a molecule that induces epigenetic changes (chromatin remodeling, ex. histone acetylation and/or DNA demethylation) or a cytoskeleton disruptor that is helpful in reprogramming a cell according to the invention (or alternatively the culture conditions can include one or more compounds, materials, environmental (physical or chemical) effects or conditions that induce epigenetic changes and/or cytoskeleton disruptors that support the transformation to the desired cell). The genetic reprogramming factor is preferably Sox2 (UniProt P48431); Oct4 (Uniprot Q01860); Cas9 (Uniprot G3ECR1); or a fragment or variant thereof.

The term "gene regulator" as used herein refers to a polynucleotide or polypeptide whose expression is associated with a series of intracellular events leading to the transformation of a given cell of a first type into a different type from the first cell type, e.g., a pluripotent, multipotent and/or unipotent cell.

The bacterial strain of the present invention also comprises an inducible genetic regulatory circuit, the regulatory circuit of the invention, to regulate the transcription of the T3SS. The expression "genetic regulatory circuit" as used herein refers to DNA sequences and/or protein sequences which can interact with a substance or signaling molecules or act in an autologous fashion to either increase, decrease, start, stop or delay transcription of other functionally linked nucleic acid sequences or even to adjust mRNA and/or protein half-life after transcription and/or translation. Examples of such nucleic acid sequences are, without limitation, promoters, enhancers, ribosome binding sequences (RBS), terminators, internal ribosome entries (IRES), degradation tags, among others.

The term "functionally linked" as used herein refers to at least two separate nucleic acid sequences functionally associated such that an event at one can precipitate a response from the other. Two or more functionally linked nucleic acid sequences can, in combination, comprise an independent genetic element.

The term "promoter" has already been defined. The term "repressor" as used herein refers to a DNA or RNA binding protein that inhibits the expression of one or more DNA/RNA sequences by binding to the operator or promoter that drives the transcription/translation of said DNA/RNA sequences.

In some embodiments of the invention the nucleotide sequences encoding the regulatory circuit of the invention comprise a DNA sequence encoding the tac promoter (Ptac) (IGEM: BBa_K864400) (SEQ ID NO: 6). Said promoter is repressed by the protein Lacl (UniProt: P03023)(SEQ ID NO: 11) Thus, in some embodiments of the invention the nucleotide sequences encoding the regulatory circuit of the invention further comprise a nucleotide sequence encoding the *lacl* gene. Preferably, the nucleotide sequence encodes the variant protein Lacl^{W220F}, wherein the original amino acid tryptophan in the homologous position 220 is substituted by the amino acid phenylalanine (W220F) according to SEQ ID NO: 12

In some embodiments of the invention the nucleotide sequences encoding the regulatory circuit of the invention comprise a nucleotide sequence encoding the bacteriophage λ major lytic promoter (PR) of the bacteriophage lambda according to SEQ ID NO: 13.

PR is controlled by the repressor cl of the bacteriophage A (SEQ ID NO:16). Thus, the nucleotide sequences encoding the regulatory circuit of the invention may also comprise a nucleotide sequence encoding cl. Preferably, the nucleotide sequence encodes the mutant variant cl ind, wherein the glutamate residue (E) at the homologous position 118 is substituted by a lysine residue (K) (SEQ ID NO: 17).

In some embodiments the nucleotide sequences encoding the regulatory circuit of the invention further comprise a nucleotide sequence encoding the tetracycline-controlled promoter (PtetA) (SEQ ID. NO: 14). Said promoter is repressed by the tetracycline repressor (TetR). Thus, the nucleotide sequences encoding the regulatory circuit of the invention may further comprise a nucleotide sequence encoding the TetR protein (SEQ ID NO: 15).

In preferred embodiments of the regulatory circuit of the invention the nucleotide sequence encoding PR is functionally linked to the nucleotide sequence encoding the mutant protein Lacl^{W220F}. In some preferred embodiments of the regulatory circuit of the invention the nucleotide sequence encoding PtetA is functionally linked to the nucleotide sequence encoding cl ind-.

The nucleotide sequences encoding the regulatory genetic circuit of the invention may also comprise a nucleotide sequence encoding a terminator. The term "terminator" as used herein refers to a transcription terminator of a DNA sequence that marks the end of a gene or operon or transcribed unit. The terminator in the regulatory genetic circuit of the invention may be any terminator described in the state of the art (for a non-exhaustive list, see http://parts.igem.org/Terminators). In some embodiments of the invention the inducible genetic regulatory circuit comprises at least one nucleotide sequence encoding a terminator selected from the group consisting of: T0 (SEQ ID NO: 18), T1 according (SEQ ID NO: 19), or any combination thereof.

The nucleotide sequences encoding the regulatory genetic circuit of the invention may also comprise ribosome binding sequences. The term "ribosome binding sequence" or "RBS" as used herein refers to a sequence of nucleotides upstream of the start codon of an mRNA transcript that is responsible for the recruitment of a ribosome during the initiation of protein translation. In some embodiments of the invention the nucleotide sequences encoding the regulatory circuit comprises an RBS according to SEQ ID NO: 20.

The nucleotide sequences encoding the regulatory genetic circuit of the invention may also comprise at least one DNA sequence encoding at least one degradation tag. The term "degradation tag" as used herein refers to short peptides that label a protein for degradation by the cell's protein recycling machinery. In some embodiments of the invention the degradation tag may be functionally linked to the nucleotide sequence encoding Lacl. In some preferred embodiments, the degradation tag is selected from the group consisting of: AAV-ssrA according to SEQ ID NO: 21, ASV according to SEQ ID no: 22, LVA-ssrA according to SEQ ID NO: 23, LAA-ssrA according to SEQ ID NO: 24 and any combination thereof.

The genetic regulatory circuit of the invention is inducible. The term "inducible" as used herein means that the expression of the genes comprising the circuit can be activated by an inductor.

The inductor can act upon several nucleotide sequences or just upon one global nucleotide sequence, which directly or indirectly controls all of the gene components of the inducible genetic regulatory circuit.

In some embodiments of the present invention the inducible genetic regulatory circuit is controlled by anhydrotetracycline (aTc).

In some embodiments of the invention the nucleotide sequences encoding the regulatory circuit preferably comprise nucleotide sequences encoding: the lactose operon repressor Lacl W220F according to SEQ ID NO: 12 and a protein degradation tag fused to the C-terminus according to SEQ ID NO: 21, SEQ ID NO: 22, SEQ ID NO: 23 or SEQ ID NO: 24; the bacteriophage A major lytic promoter (PR) according to SEQ ID NO: 13; the repressor protein cl comprising a E118K mutation (cl ind-) according to SEQ ID NO 17; and the tetracycline-controlled promoter PtetA according to SEQ ID NO: 14, and the expression of the T3SS and the genetic circuit is induced in the presence of aTc. In some embodiments, the nucleotide sequence encoding the regulatory circuit of the invention consists of SEQ ID NO: 26 or variants thereof.

The recombinant bacterial strain of the invention comprises at least one synthetic adhesin (SA) driving the specific adhesion of the strain to a target cell, from here onwards the SA of the invention. The term "synthetic adhesin (SA)" as used herein refers to a fusion protein which enables to precisely program the adhesion properties of bacteria to different target surfaces. SAs structure is organized in two domains. The first domain anchors the SA to the bacterial outer membrane, for example, and without limitation, to the N-terminal fragment of Intimin (UniProt P43261), comprising amino acid residues 1-654; or similar fragments of other Intimin (UniProt P19809) or Invasin (UniProt P19196; P11922) proteins. The second domain determines the adhesion properties of the SA, based on antibodies fragments, namely a VHH fragment. Conveniently, the at least one SA of the invention may comprise one or more different VHH fragments. This modular organization allows the adhesion domain of the SA to quickly and easily change the adhesion properties of the SA of the invention, and consequently of the bacterial strain of the invention.

In some embodiments, the recombinant bacterial strain of the invention comprises at least one SA wherein the at least one adhesion domain VHH recognizes and binds a tumor-associated cell surface antigens selected from the group consisting of: human Epidermal Growth Factor Receptor (EGFR) (UniProt P00533); Human Epidermal growth factor Receptor 2 (HER2) (UniProt P04626); Epithelial Cell Adhesion Molecule EpCAM (UniProt P16422); Fibroblast Growth Factor Receptor 3 FGFR3 (UniProt P22607); Programmed Death 1 Ligand 1 PD-L1 (UniProt Q9NZQ7); Cytotoxic T-lymphocyte protein 4 CTLA-4 (UniProt P16410); Vascular endothelian growth factor receptors VEGFRs (UniProt P17948, P35968, P35916); CD19 (UniProt P15391); CD20 (UniProt P11836); CD33 (UniProt P20138); CD47 (UniProt Q08722); CD52 (UniProt P08174); Carcino-embryogenic antigens CEA (including but not limited to UniProt P13688, P06731, P40198); Mucins MUC (including but not limited to UniProt P15941 Q02817, Q9H3R2); C-X-C chemokine receptor type 4 CXCR4 (UniProt P61073); Granulocyte colony-stimulating factor receptor G-CSFR, (UniProt Q99062); Leu-rich repeat containing G protein-coupled receptor 5 LGR5 (UniProt O75473); Syndecan proteins (UniProt P18827, P34741, O75056, P31431); or any variants or fragments thereof, in any combination.

In some embodiments of the invention, the number of different SA is 1, 2, 3, 4, 5, 6, 7, 8, 9 or 10. Each of the different SA may in turn comprise 1, 2, 3, 4, 5, or 6 different VHH domains i.e. VHH domains recognizing different antigens.

In some embodiments, the recombinant bacterial strain of the invention comprises at least one SA wherein the adhesion domain VHH recognizes and binds human Epidermal Growth Factor Receptor (EGFR) (UniProt P00533). In some embodiments, the recombinant bacterial strain of the invention comprises a first SA wherein the adhesion domain VHH recognizes and binds human Epidermal Growth Factor Receptor (EGFR) (UniProt P00533); and a second SA wherein the adhesion domain VHH recognizes and binds HER2 (UniProt P04626) or PD-L1 (UniProt Q9NZQ7). In some other embodiments, the recombinant bacterial strain of the invention comprises a first SA wherein the adhesion domain VHH recognizes and binds human Epidermal Growth Factor Receptor (EGFR) (UniProt P00533); a second SA wherein the adhesion domain VHH recognizes and binds HER2 (UniProt P04626); and a third SA wherein the adhesion domain VHH recognizes and binds PD-L1 (UniProt Q9NZQ7).

In some embodiments, the recombinant bacterial strain of the invention comprises at least one SA wherein a first adhesion domain VHH recognizes and binds human Epidermal Growth Factor Receptor (EGFR) (UniProt P00533); and a second adhesion domain VHH recognizes and binds HER2 (UniProt P04626) or PD-L1 (UniProt Q9NZQ7). Similarly, in other embodiments, the recombinant bacterial strain of the invention comprises at least one SA wherein a first adhesion domain VHH recognizes and binds human Epidermal Growth Factor Receptor (EGFR) (UniProt P00533); a second adhesion domain VHH recognizes and binds HER2 (UniProt P04626); and a third adhesion domain VHH recognizes and binds PD-L1 (UniProt Q9NZQ7).

In some specific embodiments the nucleotide sequence encoding the at least one SA of the present invention consists of SEQ ID NO: 25 or variants thereof.

In order to engineer the recombinant bacterial strain of the invention, the host gram-negative bacterial strain needs to be transformed with the genes encoding the different components of the recombinant strain (genes of interest) There are numerous techniques that allow the insertion of foreign DNA into bacteria, which the expert in the art will be well aware off (Froger and Hall, J Vis Exp. 2007;(6):253. doi: 10.3791/253. Epub 2007 Aug 1.; Wilharm et al, Journal of Microbiological Methods February 2010, Vol. 80, 2, 215-216; Green and Rogers, Methods Enzymol. 2013 529:329-36).

Once the bacterial strain is transformed with the genes of interest, these can be maintained in the cell in stable vectors or they can be integrated into the bacterial genome by several techniques well known to the skilled person, including but not limited to homologous recombination, site-specific recombination and transposon-mediated gene transposition.

The term "vector" as used herein refers to a nucleic acid molecule capable of transporting one or more other nucleic acid sequence(s) to which it has been linked or which was introduced into said vector. One type of vector is a "plasmid", which refers to a circular double stranded DNA into which additional DNA segments may be cloned. Another type of vector is a viral vector, wherein additional DNA segments may be cloned into the viral genome. Certain vectors are capable of autonomous replication in a host cell into which they are introduced (e.g., bacterial vectors having a bacterial origin of replication). Other vectors (e.g., bacterial suicide vectors) or parts thereof, are integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" or simply "expression vectors". In general, expression vectors of utility in recombinant DNA techniques are often in the form of plasmids. In the present specification, "plasmid" and "vector" may be used interchangeably, as the plasmid is the most commonly used form of vector. However, the disclosure is intended to include other forms of expression vectors, such as viral vectors (e.g., replication defective retroviruses, adenoviruses and adeno-associated viruses), which serve equivalent functions. The vector may also contain additional sequences, such as a "polylinker" or "multiple cloning site" for subcloning of additional nucleic acid sequences. A "vector backbone" or "plasmid backbone" refers to a piece of DNA containing at least a plasmid origin of replication and a selectable marker which allows for selection of hosts cells containing the plasmid or vector. When a particular plasmid or vector is modified to contain non-plasmid elements (e.g., insertion of Ad sequences and/or a eukaryotic gene of interest linked to a ribosomal promoter), the plasmid sequences are referred to as the plasmid backbone. The term "selectable marker" has used herein means an identifying factor, usually an antibiotic or chemical resistance gene, that is able to be selected for based upon the marker gene's effect, i.e., resistance to an antibiotic, resistance to an herbicide, colorimetric markers, enzymes, fluorescent markers, and the like, wherein the effect is used to track the inheritance of a nucleic acid of interest and/or to identify a cell or organism that has inherited the nucleic acid of interest. Examples of selectable marker genes known and used in the art include: genes providing resistance to ampicillin, streptomycin, gentamycin, kanamycin, hygromycin, bialaphos herbicide, sulfonamide, and the like; and genes that are used as phenotypic markers, i.e., anthocyanin regulatory genes, isopentanyl transferase gene, and the like. Vectors may be introduced into the desired host cells by methods known in the art, e.g., transfection, electroporation, microinjection, transduction, cell fusion, DEAE dextran, calcium phosphate precipitation, lipofection (lysosome fusion), use of a gene gun, or a DNA vector transporter (see, e.g., Wu et al., J. Biol. Chem. 1992. 267:963-967; Wu and Wu, J. Biol. Chem. 1988, 263:14621-14624; and Hartmut et al., Canadian Patent Application No. 2,012,311). The term "transfection" means the uptake of exogenous or heterologous RNA or DNA by a host cell. A cell has been "transfected" by exogenous or heterologous RNA or DNA when such RNA or DNA has been introduced inside the host cell. A cell has been "transformed" by exogenous or heterologous RNA or DNA when the transfected RNA or DNA effects a phenotypic change. The transforming RNA or DNA can be integrated (covalently linked) into chromosomal DNA making up the genome of the cell. "Transformation" refers to the transfer of a nucleic acid fragment into the genome of a host cell or organism, resulting in genetically stable inheritance. Host cells or organisms containing the transformed nucleic acid fragments are referred to as "transgenic" or "recombinant" or "transformed" organisms. In addition, the vector of the invention comprising the cassette of the invention may include one or more origins for replication in the cellular hosts in which their amplification or their expression is sought, reporter genes or selectable markers. In a particular embodiment, the vectors of the present invention may be episomal or integrating.

In some embodiments of the invention, the nucleotide sequences encoding the polypeptide components of the T3SS, the inducible genetic regulatory circuit and the SA are present in the bacterial strain of the invention in at least one self-replicating plasmid comprising one or more nucleotide sequences selected from the list consisting of: SEQ ID NO: 1 to SEQ ID NO: 26.

In other embodiments of the invention, the nucleotide sequences encoding the polypeptide components of the T3SS, the inducible genetic regulatory circuit and the SA are integrated into the chromosome of the bacterial strain. In some embodiments the nucleotide sequences encoding the polypeptide components of the T3SS are integrated into the chromosome of the bacterial strain of the invention in multicopy; in other embodiments they are integrated into the chromosome of the bacterial strain of the invention in monocopy.

The term "monocopy" as used herein refers to the presence of a nucleotide sequence in the bacterial strain with a copy number of one. The term "multicopy" as used herein refers to the presence of a nucleotide sequence in the bacterial strain with a copy number of more than one.

The bacterial strain of the present invention may be used in the treatment of several diseases due the possibility of modifying the not only the cargo proteins but also the SA molecules, allowing the strain to be target different types of cells and inject different compounds with different therapeutic properties into the cytosol of the cells. Therefore, another aspect of the present invention refers to the bacterial strain for use as a medicament, from here onwards the medical use of the invention. Another aspect of the invention relates to the use of the bacterial strain of the invention for the treatment of cancer is a subject, from here onwards the treatment use of the invention.

As used herein, the term "treatment" (or "treat" or "treating" or "therapy") refers to processes involving a slowing, interrupting, arresting, controlling, stopping, reducing, or reversing the progression or severity of an existing symptom, disorder, condition, or disease, but does not necessarily involve a total elimination of all disease-related symptoms, conditions, or disorders. Furthermore, the term "treatment" also refers to a process involving the avoiding of the appearance of symptoms which have not manifested themselves yet, but which will manifest themselves due to an untreated progression of the disorder, condition or disease. The treatment of a disorder or disease may, for example, lead to a halt in the progression of the disorder or disease (e.g., no deterioration of symptoms) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. Accordingly, the "treatment" of a disorder or disease may also refer to an amelioration of the disorder or disease, which may, e.g., lead to a halt in the progression of the disorder or disease or a delay in the progression of the disorder or disease. Such a partial or complete response may be followed by a relapse. It is to be understood that a subject/patient may experience a broad range of responses to a treatment (such as the exemplary responses as described herein above). In the present invention, the condition or disorder to be treated is cancer.

As used herein, the term "cancer" refers to a cellular disorder characterized by uncontrolled or disregulated cell proliferation, decreased cellular differentiation, inappropriate ability to invade surrounding tissue, and/or ability to establish new growth at ectopic sites. The term "cancer" includes, but is not limited to, solid tumors and hematologic malignancies; diseases of skin, tissues, organs, bone, cartilage, blood, and vessels; and primary and metastatic cancers

Non-limiting examples of solid tumors include pancreatic cancer; bladder cancer; colorectal cancer; biliary tract cancer, breast cancer, including metastatic breast cancer; prostate cancer, including androgen-dependent and androgen-independent prostate cancer; renal cancer, including, e.g., metastatic renal cell carcinoma; hepatocellular cancer; lung cancer, including, e.g., non-small cell lung cancer (NSCLC), bronchioloalveolar carcinoma (BAC), and adenocarcinoma of the lung; ovarian cancer, including, e.g., progressive epithelial or primary peritoneal cancer; cervical cancer; gastric cancer; esophageal cancer; head and neck cancer, thymus carcinoma, including, e.g., squamous cell carcinoma of the head and neck; skin cancer, including e.g., malignant melanoma; neuroendocrine cancer, including metastatic neuroendocrine tumors; brain tumors, including, e.g., glioma, anaplastic oligodendroglioma, adult glio-blastoma multiforme, and adult anaplastic astrocytoma; neuroblastoma, bone cancer; soft tissue sarcoma; and thyroid carcinoma.

Non-limiting examples of hematologic malignancies include acute myeloid leukemia (AML); chronic myelogenous leukemia (CML), including accelerated CML and CML blast phase (CML-BP); acute lymphoblastic leukemia (ALL); chronic lymphocytic leukemia (CLL); Hodgkin's disease (HD); non-Hodgkin's lymphoma (NHL), including follicular lymphoma and mantle cell lymphoma; B-cell lymphoma; T-cell lymphoma; multiple myeloma (MM); Waldenstrom's macroglobulinemia; myelodysplastic syndromes (MDS), including refractory anemia (RA), refractory anemia with ringed siderblasts (RARS), (refractory anemia with excess blasts (RAEB), and RAEB in transformation (RAEB-T); and myeloproliferative syndromes.

In some embodiments of the treatment use of the invention the cancer is an epithelial tumor. In some preferred embodiments of the invention the cancer is an epidermal growth factor receptor (EGFR) overproducing cancer.

The term "epidermal growth factor receptor" or "EGFR" as used herein refers to a gene that encodes a transmembrane glycoprotein member of the protein kinase superfamily. This protein is a receptor for members of the epidermal growth factor family. Binding of the protein to a ligand induces receptor dimerization and tyrosine autophosphorylation and leads to cell proliferation. Overexpression of this gene is associated with cancers like, without limitation, epithelial cancer, lung cancer, non-small cell lung cancer, colorectal cancer, astrocytoma, esophageal cancer, cervical cancer, synovial sarcoma, amongst other. Furthermore, overexpression of EGFR is associated with adverse prognosis.

In some embodiments of the treatment use of the invention the bacterial strain comprises at least one SA which binds EGFR and the cancer disease is an epithelial tumor.

In some embodiments, the treatment use of the invention comprises the use of at least two of the bacterial strains, wherein each of the bacterial strains expresses a different cargo protein. In some specific embodiments, the treatment of the invention comprises the use of (a) a bacterial strain expressing ExoA or a fragment thereof and (b) a bacterial strain expressing TccC3 or a fragment thereof.

As used herein, the term "subject" refers to human and non-human animals, such as veterinary subjects. The term "non-human animal" includes all vertebrates, e.g., mammals and non-mammals, such as non-human primates, mice, rabbits, sheep, dog, cat, horse, cow, chickens, amphibians, and reptiles. In some particular embodiments of the invention, the subject is a human, of any gender, age or race.

The bacterial strain of the present invention may be used in combination with one or more other drugs in the treatment, control, amelioration, prevention of symptoms of cancer, including but not limited to epithelial cancers, and more specifically EGFR overexpressing cancers, where the combination of the drugs together is safer or more effective than either drug alone. Thus, in particular embodiments, the bacterial strain of the invention is administered in combination with at least one other compound useful for treating cancer. Such other compound(s)/drug(s) may be administered by any route and in any amount commonly used, concomitantly or sequentially with the bacterial strain of the invention. When the bacterial strain of the invention is used concomitantly with one or more other drugs, a pharmaceutical composition in unit dosage form containing such other drugs and the bacterial strain of the invention is preferred. However, the combination therapy may also include therapies in which the bacterial strain of the invention and one or more other drugs are administered on different overlapping schedules. It is also contemplated that when used in combination with one or more other active ingredients, the bacterial strain and the other active ingredients may be used in lower doses than when each is used singly. Accordingly, the pharmaceutical compositions of the present invention include those that contain one or more other active ingredients, in addition to the bacterial strain of the invention.

The weight ratio of the bacterial strain of the present invention to the other active ingredient(s) may be varied and will depend upon the effective amount of each ingredient. Generally, a therapeutically effective amount of each will be used. Thus, for example, when the bacterial strain of the present invention is combined with another agent, the weight ratio of the bacterial strain to the other agent will generally range from about 1000:1 to about 1:1000, or from about 200:1 to about 1:200. Combinations of bacterial strain and other active ingredients will generally also be within the aforementioned range, but in each case, a therapeutically effective amount of each active ingredient should be used.

The bacterial strain of the present invention may be administered by oral, parenteral (e.g., intramuscular, intraperitoneal, intravenous, ICV, intracisternal injection or infusion, subcutaneous injection, or implant), by inhalation spray, nasal, intravesical in the urinary bladder, vaginal, rectal, sublingual, buccal or topical routes of administration and may be formulated, alone or together, in suitable dosage unit formulations containing conventional non-toxic pharmaceutically acceptable carriers, adjuvants and vehicles appropriate for each route of administration. In addition to the treatment of warm-blooded animals the bacterial strain of the invention is effective for use in humans.

The bacterial strain of the present invention can be incorporated into pharmaceutical compositions for its administration to the subject. Thus, in a particular embodiment, the bacterial strain of the invention is comprised within a pharmaceutical composition in a therapeutically effective amount. The bacterial strain of the present invention may conveniently be presented in dosage unit form and may be prepared by any of the methods well known in the art of pharmacy. All methods include the step of bringing the active ingredient into association with the carrier which constitutes one or more accessory ingredients. In general, the pharmaceutical compositions are prepared by uniformly and intimately bringing the active ingredient into association with a liquid carrier or a finely divided solid carrier or both, and then, if necessary, shaping the product into the desired formulation. In the pharmaceutical composition the active compound is included in an amount sufficient to produce the desired effect upon the process or condition of diseases. As used herein, the term "composition" is intended to encompass a product comprising the specified ingredients in the specified amounts, as well as any product which results, directly or indirectly, from combination of the specified ingredients in the specified amounts.

The pharmaceutical compositions containing the active ingredient may be in a form suitable for oral use, for example, as tablets, troches, lozenges, aqueous or oily suspensions, dispersible powders or granules, emulsions, solutions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. Oral tablets may also be formulated for immediate release, such as fast melt tablets or wafers, rapid dissolve tablets or fast dissolve films.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. In addition, fatty acids such as oleic acid find use in the preparation of injectables. The compounds of the present invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions and the like, containing the compounds of the present invention are employed. Similarly, transdermal patches may also be used for topical administration.

In the treatment, control, amelioration, prevention of symptoms or reduction of risk of cancer, an appropriate dosage level of the bacterial strain of the invention will generally be about 0.01 to 500 mg per kg patient body weight per day which can be administered in single or multiple doses. A suitable dosage level may be about 0.01 to 250 mg/kg per day, about 0.05 to 100 mg/kg per day, or about 0.1 to 50 mg/kg per day. Within this range the dosage may be 0.05 to 0.5, 0.5 to 5 or 5 to 50 mg/kg per day. For oral administration, the compositions may be provided in the form of tablets containing 1.0 to 1000 milligrams of the active ingredient, particularly 1.0, 5.0, 10.0, 15.0. 20.0, 25.0, 50.0, 75.0, 100.0, 150.0, 200.0, 250.0, 300.0, 400.0, 500.0, 600.0, 750.0, 800.0, 900.0, and 1,000.0 milligrams of the strain for the symptomatic adjustment of the dosage to the patient to be treated. The compounds may be administered on a regimen of 1 to 4 times per day, or may be administered once or twice per day.

It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### DESCRIPTION OF THE DRAWINGS

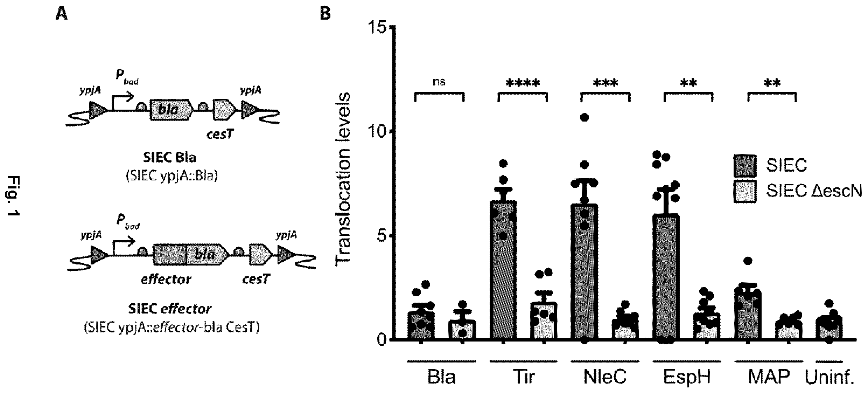

**Fig. 1****. Translocation of T3SS effectors fused to the β-lactamase reporter to HeLa cells using the strain. (A) Schematic representation of the constructs integrated in the ypjA genomic locus for the co-expression of the effectors fused to bla and the chaperone CesT. (B). Translocation of the effectors Tir, NleC, EspH and MAP.**

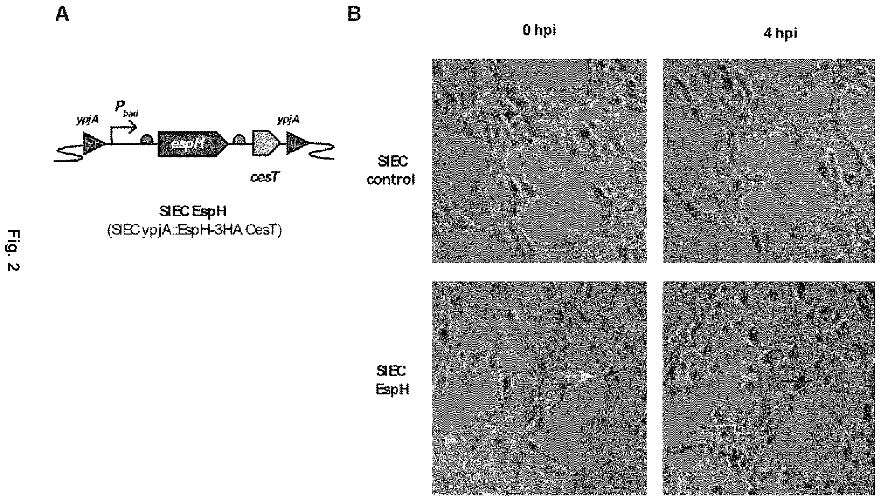

**Fig. 2****. Effect of the T3SS effector EspH on Her14 mouse fibroblasts after its delivery by SIEC.** Time-lapse brightfield microscopy images of the cell culture after the infection with SIEC control and SIEC EspH, at 0 and 4 hours post infection (hpi). Arrows (yellow at 0 hpi and red at 4 hpi) indicate location of the same individual cells at each timepoint.

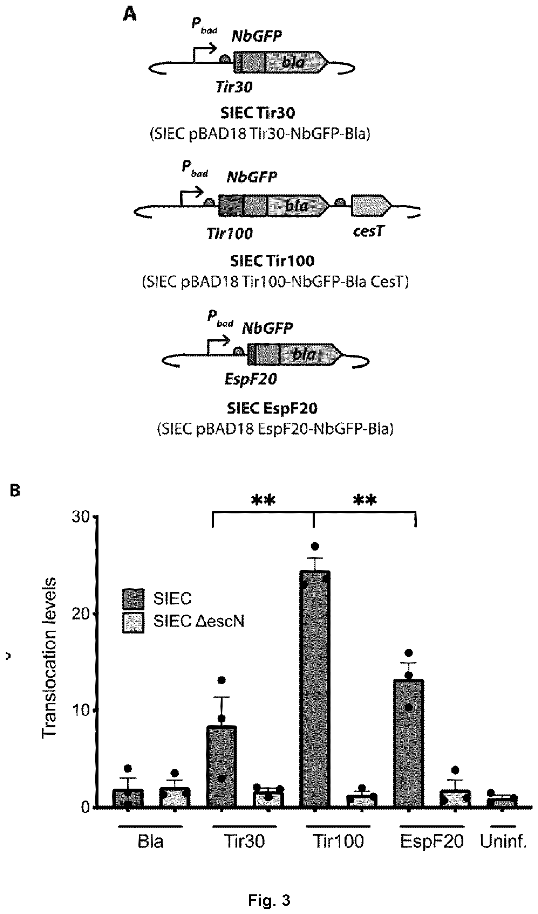

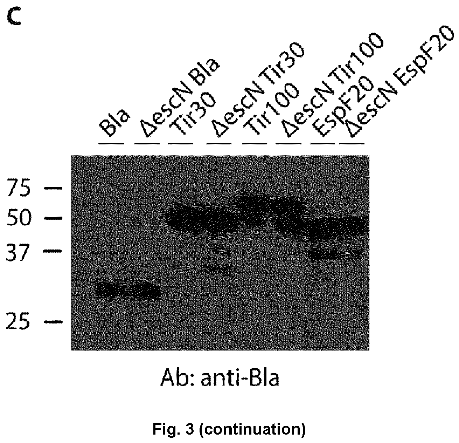

**Fig. 3****. Expression cassettes with different secretion signals for translocation of heterologous, non-T3SS proteins.** (A) Schematic representation of the constructs derived from pBAD18 for the co-expression of the different secretion signals fused to the NbGFP and bla, with the presence of the chaperone CesT if indicated. (B) Translocation triggered by the different secretion signals. (C) Western blot expression of the different constructs expressed in SIEC and SIECΔescN.

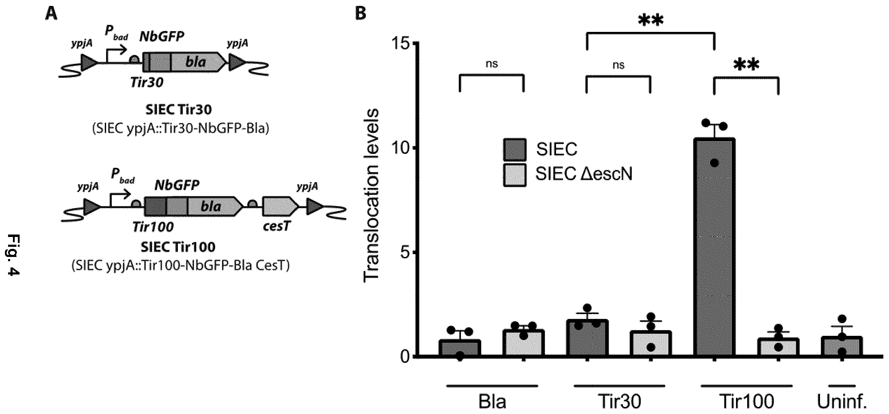

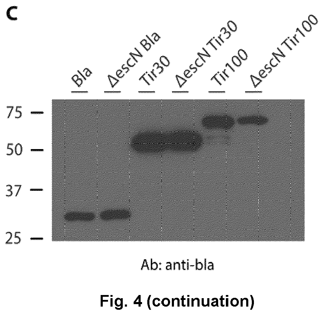

**Fig. 4****. Construct design for the translocation of cargo proteins expressed from genes integrated in the chromosome.** (A) Schematic representation of the constructs integrated in the ypjA genomic locus for the co-expression of the different secretion signals fused to the NbGFP and bla, with the presence of the chaperone CesT if indicated. (B) Translocation triggered by the different secretion signals. (C) Western blot expression of the different constructs expressed in SIEC and SIECΔescN. Statistical significance inferred by unpaired t-test analysis.

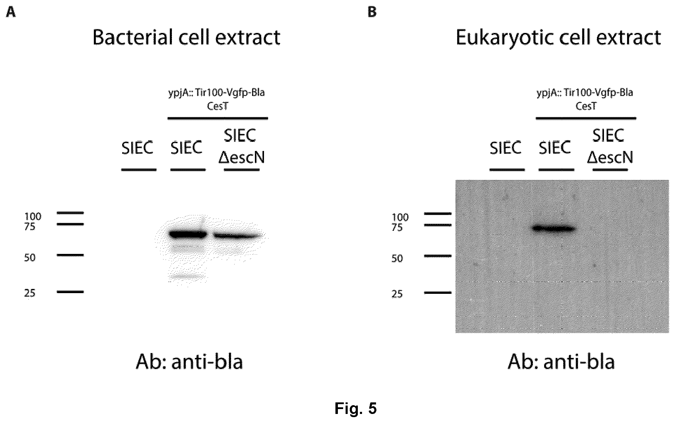

**Fig. 5****. Detection of translocated cargo proteins by western blot.** Images show (A) presence of the heterologous fusion protein in the bacteria and (B) injected into the eukaryotic cytoplasm. Induction and infection conditions were the same used in Figure 1.

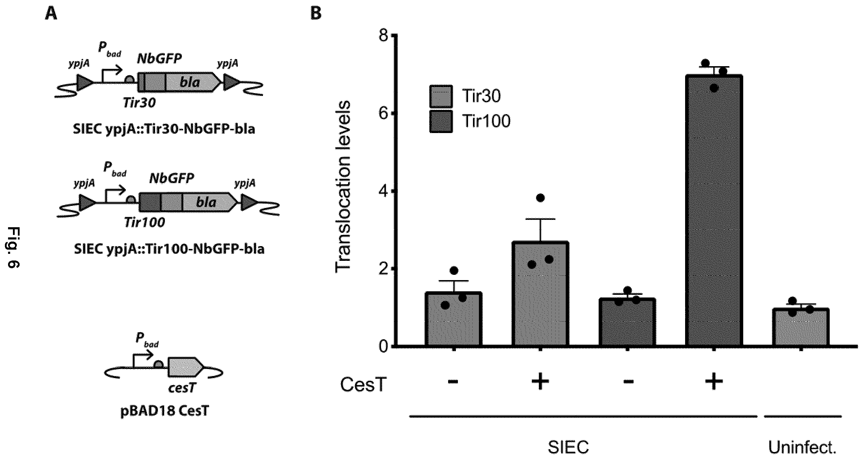

**Fig. 6****. Role of the T3SS chaperone CesT on the delivery of a cargo protein fused to different secretion signals to the cytoplasm of eukaryotic cells.** (A) Schematic representation of the generated strains integrated in the ypjA genomic locus for and the plasmid pBAD18 CesT for expressing the chaperone CesT. (B) Translocation triggered by the different strains expressing or not the CesT chaperone.

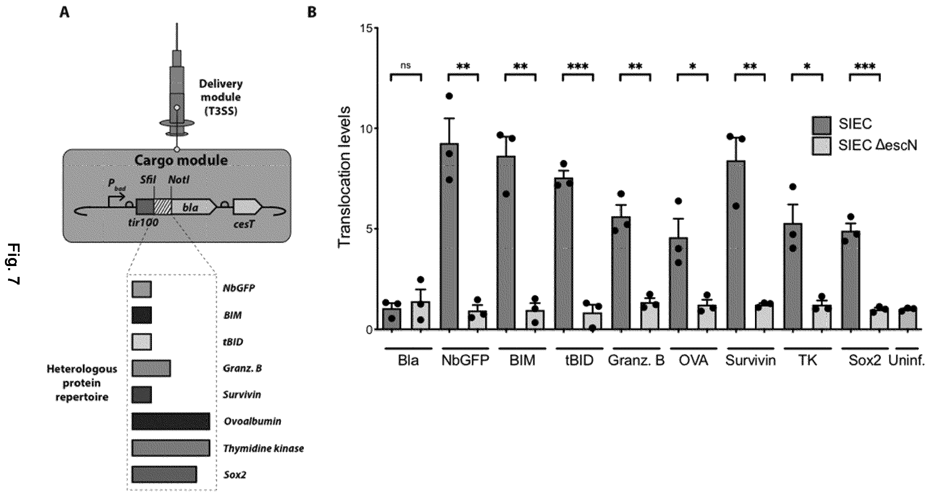

**Fig. 7****. "Cargo module" and the translocation of a repertoire of cargo proteins using the SIEC strain.** (A) Scheme represents the most efficient cassette engineered for delivery of cargo proteins, which would constitute the cargo module of SIEC, and the different cargo proteins inserted into it to generate the protein repertoire. (B) Translocation of cargo proteins from the repertoire and (C) further proteins engineered in the cargo module which did not show translocation signal, including the NbGFP positive control.

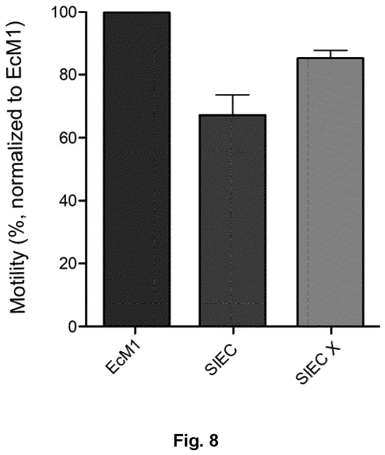

**Fig. 8****. Motility of SIEC vs SIEC X.**

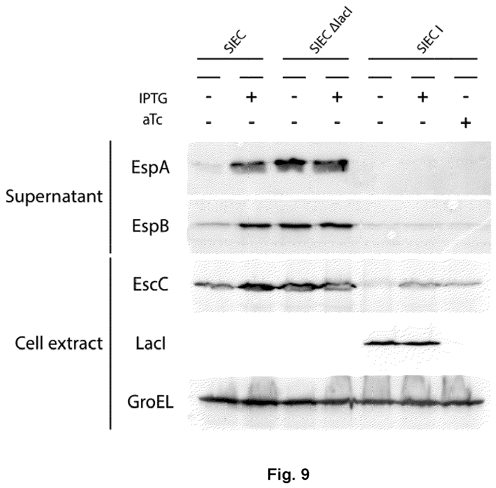

**Fig. 9****. Functionality of the first circuit design: derrepression problem.** Western blot expression of the Lacl repressor, T3SS proteins and GroEL control in the indicated strains after induction with IPTG or aTc.

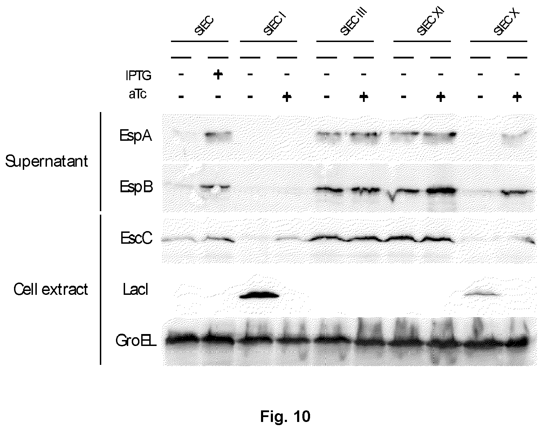

**Fig. 10****. Functionality of the circuit using the different ssrA tags fused to the Lacl W220F repressor.** (A) Representation of the different ssrA degradation tags fused to the repressor Lacl W220F and its strength. (B) Western blot expression of the Lacl/ LaclW220F repressor, T3SS proteins and GroEL control in the indicated strains.

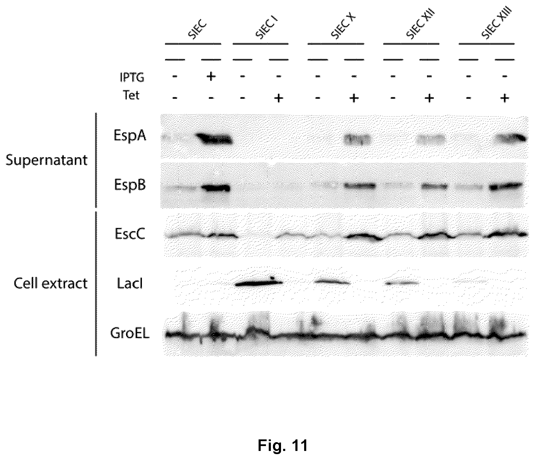

**Fig. 11****. Attenuating Lacl-ASV expression for full activation of the system.** (A) Representation of the engineered circuits versions containing the gene lacl with weaker RBS and alternative transcription start. (B) Western blot expression of the Lacl repressor, T3SS proteins and GroEL control in the indicated strains.

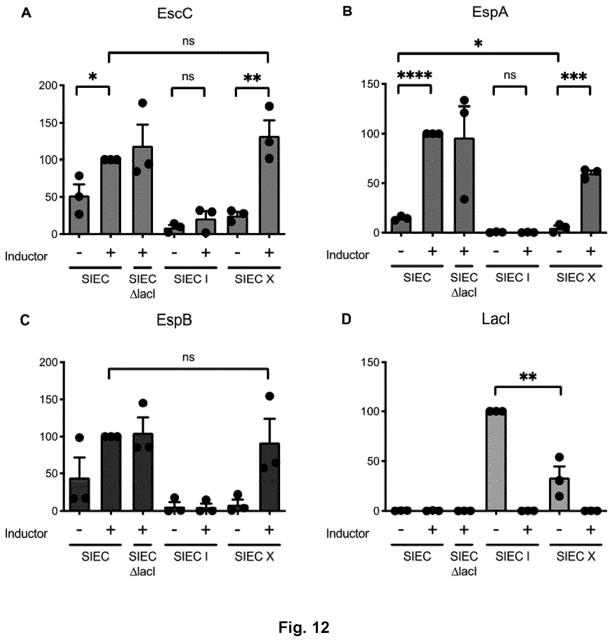

**Fig. 12****. Expression quantification of T3SS proteins and Lacl repressor.** Western blot expression levels (A, B, C) relative to induced SIEC of the T3SS components (A) EscC, (B) EspA and (C) EspB, or (D) relative to non-induced SIEC I in the case of the repressor Lacl.

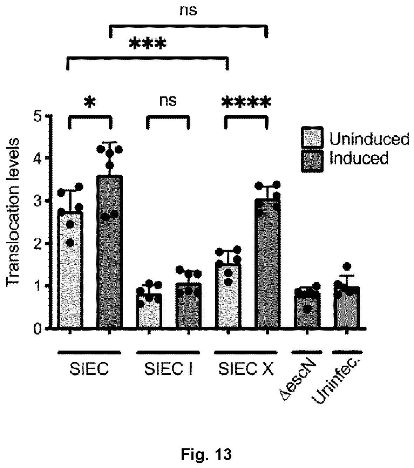

**Fig. 13****. Comparison of protein translocation levels of different SIEC strains containing different genetic circuit versions.**

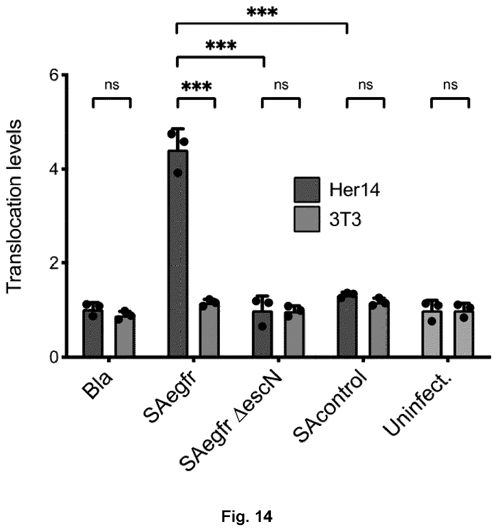

**Fig. 14****. Specific injection to cells expressing EGFR by specific attachment of SIEC X containing SAegfr** (Translocation levels). Bla corresponds to SIEC pBAD18 Bla, SAegfr to SIEC SAegfr pBAD18 Tir100-Vgfp-Bla, SAegfr ΔescN to SIECΔescN SAegfr pBAD18 Tir100-Vgfp-Bla and SAcontrol to SIEC SAcontrol pBAD18 Tir100-Vgfp-Bla.

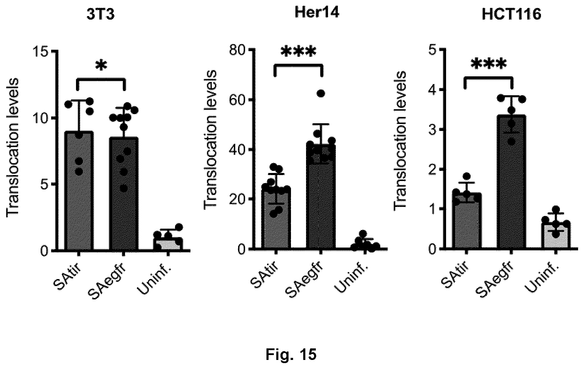

**Fig. 15****. Injection enhancement for target cells by using specific Synthetic Adhesins (translocation levels).** SAtir corresponds to SIEC X SAtir ypjA::Ptet-Tir100-NbGFP-Bla and SAegfr corresponds to SIEC X SAegfr ypjA::Ptet-Tir100-NbGFP-Bla.

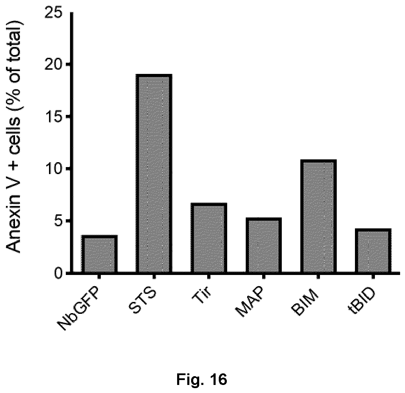

**Fig. 16****. Apoptosis induction upon injection of T3SS natural effectors and cargo proteins.** Percentage of apoptotic (annexin V positive) cells 24 hours post injection.

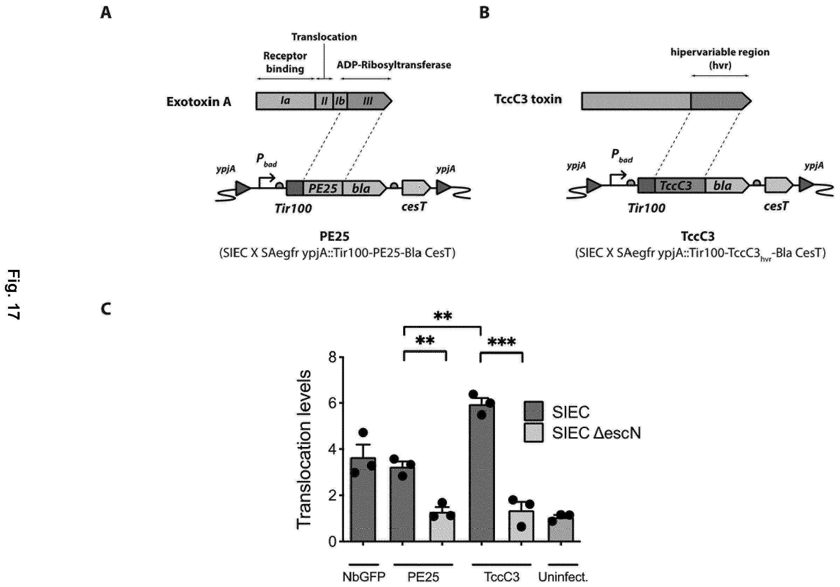

**Fig. 17****. Injection of ADP-ribosilating toxins: Exotoxin A and TccC3.** A) and (B) Toxin regions inserted in the cargo module for generating the SIEC X strains for testing translocation of (A) PE25 and (B) TccC3hvr. (C) Translocation calculated as the ratio of OD450/OD520 and normalized by the non-treated cells signal.

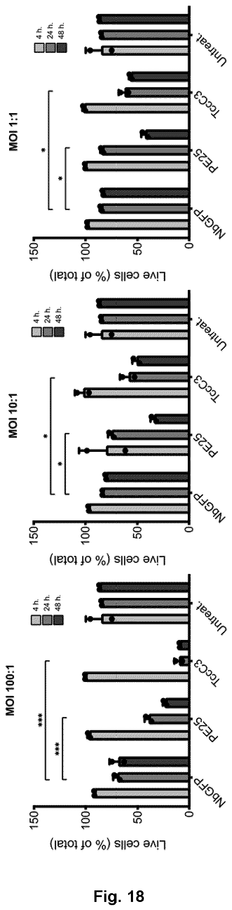

**Fig. 18****. Analysis of tumor cell killing using different bacterial doses.** Percentage of living cells in culture at 4-, 24- and 48-hour post infection of SIEC at 3 different MOls and injecting NbGFP, ExoA or TccC3.

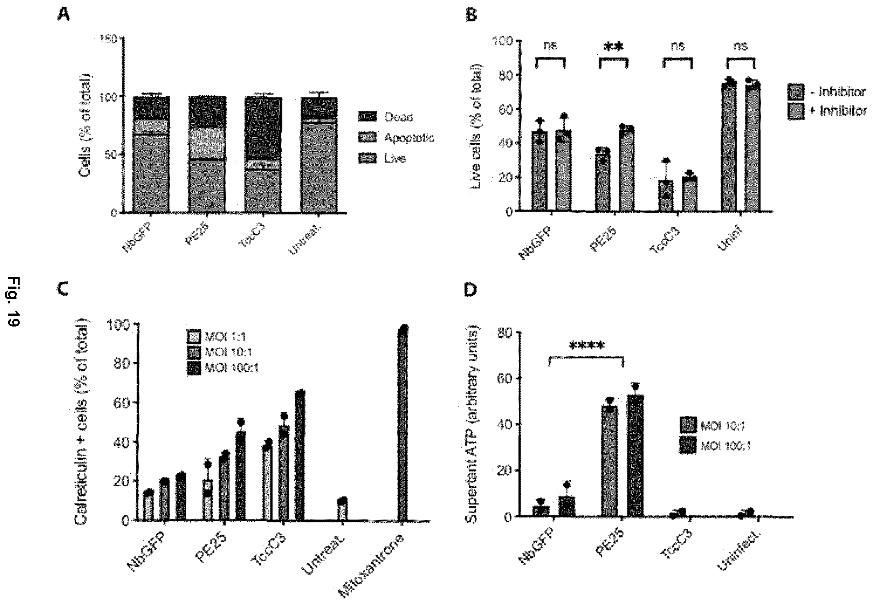

**Fig. 19****. Toxins mechanism of cell killing after injection and immunogenic cell death.** (A) percentage of live, apoptotic, and dead cells by FLICA staining and flow cytometry analysis 24 hours after translocation of the indicated cargos, (B) percentage of live cells by LIVE/DEAD staining and flow cytometry analysis 24 hours after translocation of the indicated cargos with presence or not of the pan-caspases inhibitor Z-VAD-FMK, (C) percentage of calreticulin positive cells by flow cytometry analysis 72 hours after translocation of the indicated cargos and (D) ATP release to the extracellular media by biochemical luminescence analysis 72 hours after translocation of the indicated cargos.

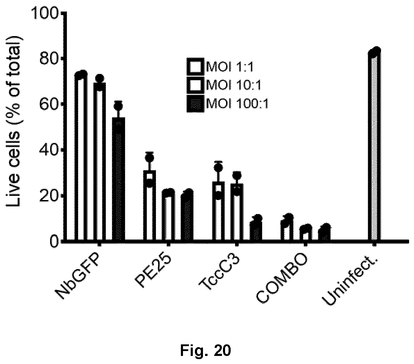

**Fig. 20****. Combination of anti-tumor toxins for effective cell killing at low bacterial doses.** Percentage of live cells after injection of individual toxins and combination at 48 hpi. MOls of 100:1, 10:1 and 1:1.

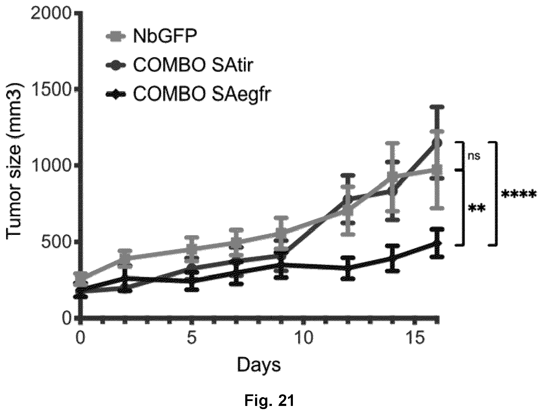

**Fig. 21****. HCT116 tumor growth evolution in Nude mice upon i.t. administration of 10⁸ bacteria/mouse.** Graph shows 16-days evolution of tumor volume after the bacterial administration, with lower arrows indicating administration days. Each point represents tumor volume mean of 6 mice.

### Examples

### Materials and methods

### 1 - Bacterial culture general specifications.

Bacteria used in this work were grown in Liquid Broth (LB) medium unless specified. For solid media formulations, LB-agar plates (1.5% w/v) were employed. For growing purposes, bacteria were incubated at 37°C. When selection was needed, antibiotics were added at the following concentrations: kanamycin (Km) at 50 µg/ml, spectinomycin (Sp) at 50 µg/ml and ampicillin (Ap) at 150 µg/ml.

### 2 - Plasmid constructs generation and strain engineering.

Plasmids were constructed following standard restriction enzyme based genetic engineering. Designed primers were ordered to Sigma-Aldrich Oligos Service, and are listed in Table 1.

**Table 1: List of primers used in the examples**

| **SEQ ID NO:** | **Name** | **SEQ ID NO:** | **Name** |
|---|---|---|---|
| 27 | F_Xbal_RBS_MAP | 50 | F_Sfil_Oct4 |
| 28 | R_Notl_MAP | 51 | R_Notl_Oct4 |
| 29 | ypjA_5' | 52 | R_cargo_seq |
| 30 | ypjA_3' | 53 | 3' del Lacl confirm |
| 31 | R_AraC_test | 54 | R_Notl_tBID |
| 32 | F_CesT_test | 55 | 5prima_SReginteg |
| 33 | F_Sacl_RBS_CesT | 56 | F_Lacl_seq |
| 34 | F_Sfil_Gly_ZF6x | 57 | R_3prima de Lacl |
| 35 | R_Notl_ZF6x | 58 | F_EcoRI_RBS0034_Lacl |
| 36 | F_Sfil_GranzymeB | 59 | R_Kpnl_ssrAtag(LAA)_Lacl |
| 37 | R_Notl_GranzymeB | 60 | F_EcoRI_RBS0034_Lacl (NEW) |
| 38 | F_Sfil_OVA | 61 | F_EcoRI_RBS0034_(GTG)Lacl |
| 39 | R_Notl_OVA | 62 | F_EcoRI_RBSWT_(GTG)Lacl |
| 40 | F_Sfil_TK | 63 | R_Kpnl_Lacl |
| 41 | R_Notl_TK | 64 | R_Kpnl_ssrAtag(ASV)_Lacl |
| 42 | F_Sacl_RBS_EspF20 | 65 | R_Kpnl_ssrAtag(AAV)_Lacl |
| 43 | R_Spel_Bla | 66 | F_Sfil_PE25 |
| 44 | R_Notl_BIM | 67 | R_Notl_PE |
| 45 | F_Sfil_BIM | 68 | R_BgIII_STOP_Tir |
| 46 | F_Sfil_Cas9 | 69 | R_BgIII_STOP_PE25 |
| 47 | R_Notl_Cas9 | 70 | F_Sfil_TccC3 |
| 48 | F_Sfil_Sox2 | 71 | R_BgIII_STOP_TccC3 |
| 40 | R_Notl_Sox2 | | |

Restriction enzymes were obtained from New England Biolabs and Thermo Fisher Scientific. Insert amplifications were carried out by using the proofreading DNA polymerase Herculase II Fusion (Agilent Technologies), followed by an isolation step by size in agarose gel. Ligation of plasmid backbone and insert was catalyzed in an overnight reaction using the T4 DNA ligase (Roche). All generated constructs were first screened for the presence of the insert by PCR amplification using NZYProof 2x Green Master Mix (NZYtech) and afterwards sent for external sequencing (Macrogen, StabVida or Secugen) using specific checking primers. *E. coli* DH10B-T1R was used as cloning working strain for replicative plasmids, containing the pBR origin of replication, such as pBAD18 and derivatives. For cloning and propagation of suicide pGE-plasmid derivatives containing the conditional pir-dependent R6K origin of replication (Stalker, Kolter et al. 1982, J Mol Biol 161(1): 33-43), the *E. coli* strains BW25141 was employed. The main features of the plasmids used in this study are described below:
**pACBSR:** helper plasmid transformed for integration and deletion purposes. Contains the A Red recombinase and the I-Scel restriction enzyme under the control of the promoter BAD and the repressor AraC, inducible by L-ARA and repressed by glucose, and the Spectinomycin resistance gene.
**pGE:** suicide plasmid used for integration purposes. Contains the R6K origin of replication so it is dependent on the presence of the protein pir for its replication, and the Kanamycin resistance gene.
**pGE Δ(gene) family:** pGE derivatives, contain the homology regions needed for deletion of genes or operons.
**pGE ypjA PBAD-Bla:** pGE derivative, contains the homology regions needed for integration in ypjA locus of the reporter ß-lactamase controlled by the promoter BAD and the repressor AraC, inducible by L-ARA and repressed by glucose.
**pGE ypjA PBAD-(effector)-bla CesT-Etag family:** pGE derivatives, contain the homology regions needed for integration in ypjA locus and an EPEC's effector protein fused in the C-terminal part to the reporter ß-lactamase and controlled by the promoter BAD and the repressor AraC, inducible by L-ARA and repressed by glucose. Also contain downstream in a bicistronic configuration the chaperone CesT fused to the detection tag E-tag in the C-terminus.
**pBAD18:** multicopy plasmid containing the pBR322 origin of replication, the AraC repressor gene and the BAD promoter and the repressor AraC, inducible by L-ARA and repressed by glucose, and the Kanamycin resistance gene (Guzman, Belin et al. 1995).
**pBAD18 PBAD-Bla:** pBAD18 derivative containing the reporter ß-lactamase.
**pBAD18 PBAD-(SecSignal)-NBgfp-Bla family:** pBAD18 derivatives containing different T3SS secretion signals fused to the nanobody against GFP and to the reporter ß-lactamase.Only in the case of Tir100 secretion signal this plasmid also contains downstream in a bicistronic configuration the chaperone CesT fused to the detection tag E-tag in the C-terminus.
**pGE PBAD-(SecSignal)-NBgfp-Bla family:** pGE derivatives, contain the homology regions needed for integration in ypjA locus of different T3SS secretion signals fused to the nanobody against GFP and to the reporter ß-lactamase and controlled by the promoter BAD and the repressor AraC, inducible by L-ARA and repressed by glucose. In the case of Tir100 secretion signal, when indicated, this plasmid can also contain downstream in a bicistronic configuration the chaperone CesT fused to the detection tag E-tag in the C-terminus.
**pBAD18 PBAD-CesT-Etag:** pBAD18 derivative, contains the chaperone CesT fused to the detection tag E-tag in the C-terminus.
**pBAD18 PBAD-Tir100-(cargo)-Bla CesT-Etag family:** pBAD18 derivatives containing the Tir100 T3SS secretion signal fused to different cargos (cargo proteins) and to the reporter ß-lactamase and. In the case of Tir100 secretion signal, when indicated, this plasmid can also contain downstream in a bicistronic configuration the chaperone CesT fused to the detection tag E-tag in the C-terminus. This family of plasmids constituted the "Cargo module" used in the Examples below.
**pGE curli RegCircuit (n) family:** pGE derivatives, contain the homology regions needed for integration in curli locus of the different versions of the Regulation Circuit.
**pBAD18 PBAD EspF20-Bla:** pBAD18 derivative, contains the EspF20 T3SS secretion signal fused to the reporter ß-lactamase.pGE recomb (SA) family: pGE derivatives, contain the homology regions needed for replacing the nanobody without changing the other regions in a Synthetic Adhesin previously integrated in the flu locus.
**pGE ypjA PBAD-(effector) CesT-Etag family:** pGE derivatives, contain the homology regions needed for integration in ypjA locus and an EPEC's effector protein and controlled by the promoter BAD and the repressor AraC, inducible by L-ARA and repressed by glucose. Also contain downstream in a bicistronic configuration the chaperone CesT fused to the detection tag E-tag in the C-terminus.
**pBAD18 PBAD-TirlOO-(cargo) CesT-Etag family:** pBAD18 derivatives containing the Tir100 T3SS secretion signal fused to different cargos. If indicated, three copies of the tag HA are fused to the C-terminus. Also contain downstream in a bicistronic configuration the chaperone CesT fused to the detection tag E-tag in the C-terminus.
**pGE ypjA PBAD-Tir100-(cargo) CesT-Etag family:** pGE derivatives, contain the homology regions needed for integration in ypjA locus and the T3SS secretion signal Tir100 fused to various cargo proteins. Also contain downstream in a bicistronic configuration the chaperone CesT fused to the detection tag E-tag in the C-terminus.
**pGE ypjA Ptet-Tir100-(cargo) CesT-Etag family:** pGE derivatives, contain the homology regions needed for integration in ypjA locus and the T3SS secretion signal Tir100 fused to various cargo proteins. The AraC and BAD promoter were replaced by the tet promoter inducible by aTc. Also contain downstream in a bicistronic configuration the chaperone CesT fused to the detection tag E-tag in the C-terminus.

Transformation of non-suicide plasmids was carried out using the TSS method (Chung, Niemela et al. 1989, Proc Natl Acad Sci U S A 86(7): 2172-2175) or by electroporation of electrocompetent cells. Transformation of suicide plasmids was always performed by electroporation. Site-specific deletions and insertions in the chromosome of E. coli were performed based on expression of I-Scel endonuclease leaving no scars, with a marker-less strategy of genome edition. Briefly, the *E. coli* strain to be modified was initially transformed with plasmid pACBSR (SpR variant), expressing I-Scel and A Red proteins under the control of PBAD promoter (inducible by L-arabinose), and subsequently electroporated with the corresponding pGE-based suicide vector (KmR). Cointegrants were selected on LB-Sp-Km plates incubated at 37°C. Individual colonies were isolated and grown for 6 h in LB-Sp liquid medium containing ARA, (L-arabinose at 0.4% w/v) with agitation (160 rpm). After this period, the culture was streaked on LB-Sp plates using an inoculating loop and incubated overnight. Individual colonies were replicated in LB-Sp along with LB-Sp-Km to screen for Km-sensitive colonies that have performed resolution of the cointegrant vector after I-Scel induction. Individual Km-sensitive colonies were screened by PCR with specific oligonucleotides to identify those with the desired modification in their chromosome (i.e., deletion, insertion, substitution). If necessary, bacterial chromosome was isolated and the integrated region amplified with the proofreading DNA polymerase Herculase II Fusion, followed by agarose gel purification of the corresponding amplicon band and sent for verification by sequencing with specific primers.

### 3 - SDS-PAGE and Western Blot

Sodium Dodecyl Sulfate-Polyacrylamide gel electrophoresis (SDS-PAGE) was the technique used for denaturalizing separation of proteins in polyacrylamide gels. Electrophoresis was carried out following standard methods using the Miniprotean III system (Bio-Rad). Once separated by SDS-PAGE, proteins were either stained with a protein specific dye (Coomasie Blue R-250, Bio-Rad) or transferred to a polyvinylidene difluoride membrane (PVDF, Immobilon-Pm Milipore) for western blot analysis. This transference was performed by semi-dry electrophoresis. Membranes were developed by using the Western ECL Substrate kit (Bio-Rad). The developed membranes were placed into a dark cassette and solved by film exposition or Chemidoc Touch Imaging System (Bio-Rad) image acquisition.

### 4 - In vitro eukaryotic cell culture

Immortalized eukaryotic cell lines were used for this work. Cell lines were grown in monolayer in culture flasks using appropriate cell culture media: HeLa epithelial tumor cells and 3T3 and Her14 mouse embryo fibroblasts were cultivated in Dulbecco's modified Eagle's medium (DMEM), supplemented with 10% fetal bovine serum (FBS) (complete DMEM). HCT116 colorrectal carcinoma epithelial cells (ATCC^{®} CCL-247^{™}) were grown in McCoys 5A medium supplemented with 10% fetal bovine serum (FBS) (complete McCoys). All cell lines were cultivated at 37°C with 5% CO2 in static conditions. For in vitro cell assays performed in this work, cells were harvested from flasks at 100% confluency and placed on 6, 24 or 96-well plates, sterile and TC treated, at desired cell concentration.

### 5 - Synthetic Injector E. coli (SIEC) induction and infection

For induction of SIEC strains for analysis of the T3SS components expression, bacteria were grown o/n from a single colony with agitation (160 rpm) at 37 °C. Next day, cultures were diluted 1:100 in 5 ml of LB with the appropriate T3SS inductor (IPTG at 0.1 mM or aTc at 200 ng/ml), in capped Falcon tubes (BD Biosciences). Bacteria were grown for 6 hours with agitation (160 rpm) at 37 °C, when the culture was centrifuged at 4000 rpm for 15 minutes. After the centrifugation, pellet and supernatant fractions were split. Pellet samples were washed in PBS and concentrated 10 times before boiling with loading buffer containing ß-mercaptoethanol for 10 minutes. Supernatant fraction was centrifuged 3 times for eliminating bacterial remnants and directly boiled with loading buffer containing ß-mercaptoethanol for 10 minutes. If concentration of supernatant proteins was also performed, correspondent samples were chilled on ice and incubated 60 min with trichloroacetic acid (TCA, Merck) at 20% weight/volume for precipitation of proteins. After cold centrifugation (20000g, 15 min), TCA-precipitated protein pellets were rinsed with cold acetone (-20°C) and resuspended in PBS before boiling with loading buffer containing ß-mercaptoethanol for 10 minutes. When preparing SIEC for infecting eukaryotic cells, two experimental configurations were designed for this work: preinduction of SIEC prior to infection and induction over-cells. If preinduction was used, strains were grown o/n from a single colony in 5 ml of LB in capped Falcon tubes at 160 rpm and 37°C. Next day, bacterial cultures were diluted 1:100 in 5 ml of LB with the appropriate T3SS inductor (IPTG at 0.1mM or aTc at 200 ng/ml). Bacteria were induced for 2 hours with agitation (160 rpm) at 37 °C, when the cargo inductor ARA (L-Arabinose) or aTc was added. After one more hour of induction, bacteria were diluted in serum free DMEM (containing IPTG or aTc and ARA or aTc) to the desired concentration and added over the cells. If the induction was developed over-cells, bacteria were grown o/n from a single colony in glass flasks in 10 ml of LB, in static conditions at 37°C. O/N cultures were then directly diluted in serum free DMEM containing IPTG or aTc and ARA or aTc except in attach-and-inject experiments (see "Attach-and-inject assays"), and finally added over the cells.

### 6 - Eukaryotic cell content fractionation

In order to selectively separate the cytoplasmic content of the cell culture infected with bacteria after the injection process in 6-well plates, the following protocol was performed: first, cells were washed extensively (10 times) with PBS to eliminate as many bacteria as possible. Afterwards, the dry plate containing the cells was frozen at -80°C. Next day 0.5 ml of 1x lysis buffer was added to each well. Then, cells were scrapped and transferred to 1.5 ml microtubes. After an incubation of 30 minutes at 4°C, tubes were centrifuged at 15000 rpm for 15 minutes at 4°C. Supernatant of each tube was collected and boiled with loading buffer containing ß-mercaptoethanol for 10 minutes for analysis by western blot.

### 7 - ß-lactamase translocation reporter assay

After the injection process was carried out, protein translocation quantification or visualization was assessed by using the LiveBLAzer^{™} FRET B/G Loading Kit with CCF2-AM (ThermoFisher Scientific). Shortly, eukaryotic cells were seeded two days prior to the experiment in a TC-treated 96-well black plate with flat clear bottom (Corning) at a concentration of 104 cells per well. A minimum of 3 wells per experimental condition, including non-infected cells, were prepared. Wells with no cells were also set as blanks. The plate was incubated at 37°C with 5% CO2 in static conditions until the day of the experiment (aprox. 48h). The day before the experiment, bacterial strains were grown o/n, and the day of the experiment preinduced if necessary and diluted in serum-free DMEM. Prior to the addition of the bacteria, cell culture was washed 3 times with serum-free DMEM in order to completely eliminate FBS from the culture medium. After the infection and translocation process occurred, wells were washed 3 times with preheated phenol red free HBSS (Gibco). Then, each well, including blanck wells, were covered with the ß-lactamase substrate, composed by a 100 µl of HBSS + 20 µl of 6x CCF2/AM solution freshly prepared with the CCF2/AM loading kit (CCF2/AM final concentration: 1 µM). Following the supplier guidelines to load cells with substrate, the plate was incubated at room temperature in dark for 90 minutes before introduced in the microplate reader for quantification or visualized in fluoresce microscopy. For quantification of translocation levels, a SpectraMax iD5 Multi-mode Microplate Reader (Molecular Devices) was employed. The reading configuration used, following the supplier indications, was a double fluorescence lecture: excitation at 409 nm wavelength, emission at 450 nm (blue fluorescence) firstly, and excitation at 409 nm, emission at 520 nm (green fluorescence) secondly. Blank wells emissions values at correspondent wavelength were subtracted from each well. For representation of results, relative translocation levels were obtained by calculating the fluorescence emission ratio between 450nm and 520nm (blue emission/green emission) for each well. Each experimental condition was normalized by the emission ratio of the non-infected cells, and therefore represented in relative units. For fluorescence microscopy endpoint visualization of translocation, a confocal multispectral Leica TCS SP8 was used (Advanced Light Microscopy Service of CNB). For this purpose, IBIDI 4 or 8-wells chambers were employed instead of 96 well plates. The infection process was developed as previously described, and each well was eventually covered with ß-lactamase reagent prepared at the same concentration that for quantification in microplate, but more volume was used to cover the whole surface of the IBIDI chamber (around 0.5 ml per well). After incubating 90 minutes in dark, images were taken in the confocal microscope using appropriate filters for green and blue fluorescence emission. Both colors images were taken simultaneously for each well and superposed for visualizing the final composite. Acquisition conditions were maintained for all wells. ImageJ software was used for image composition. If time-lapse images were captured, ß-lactamase substrate preloading into eukaryotic cells was necessary prior to infection. Preloading process was adapted from (Mills, Baruch et al. 2008). In short, cells were cultivated in IBIDI chambers for imaging as described, but prior to the addition of bacteria, cells were covered with ß-lactamase reagent solution prepared following the supplier indications, but this time containing probenecid (Sigma) at 2.5 mM final concentration. Cells were then incubated for 60 minutes in the dark at room temperature and then for 15 minutes at 37°C. Then, bacteria were finally added in serum-free DMEM also containing probenecid at 2.5 mM. Images were captured in multispectral Leica TCS SP8 at different timepoints, now incubating at 37°C for maintaining eukaryotic cell and bacteria viability. If washes were performed, fresh media added contained again probenecid at 2.5 mM. If necessary, culture was reloaded with more ß-lactamase substrate solution containing probenecid and incubated. DMEM media used in this experiment also contained a pH buffer for maintaining culture pH stability while the time lapse process occurred inside the confocal microscope. Acquisition conditions were maintained in all wells. ImageJ software was used for time lapse composition.

### 8 - Synthetic Adhesins bacterial exposure analysis

Bacteria culture correspondent to 1 OD (600nm wavelength read) was centrifuged at 4000g for 3 minutes, washed twice in prefiltered PBS and finally resuspended in 1 ml of PBS containing 10% (volume/volume) of goat serum (Sigma). 200 µl of each sample were transferred to a new tube and incubated with anti-myc (Cell Signaling Technology, diluted 1:200) for 1 hour. Afterwards, the samples were washed three times in prefiltered PBS twice, resuspended in 500 µl of PBS with 10% (volume/volume) of goat serum and anti-mouse Alexa 488 secondary antibody (Life Technologies, 1:500 dilution) and incubated for 45 minutes in dark. Finally, samples were washed three times, resuspended in 1 ml of PBS and transferred to cytometry tubes for analysis in the Flow Cytometry Service of the CNB in a Gallios Cytometer (Beckman Coulter). Results were analyzed using FloJo software.

### 9 - Bacterial adhesion visualization

Strains to test adhesion were cultivated O/N at 37°C in 10 ml of LB in static conditions in glass flasks. Next day, culture was pelleted at 4000g for 3 minutes, washed with PBS and diluted in serum-free DMEM to a final concentration of 0.1 OD/ml (determined to be 3x107 CFU/ml). This sample was further diluted if necessary, or directly used to infect cells. Cells were grown the day before the experiment on sterile coverslips (13 mm diameter, VWR international) placed at the bottom of the well of a 24-well plate. The infection was incubated for 1 hour at 37°C, 5% CO2 for attachment, followed by 5-10 PBS washes (1 ml per well) until no unattached bacterial is observed in the media. Coverslips were then fixed with paraformaldehyde 4% (w/v) diluted in PBS (0.5 ml per well) for 20 minutes at room temperature. After that, paraformaldehyde was removed and coverslips washed 3 times with PBS. Next, coverslips were blocked and stained for 1 h at room temperature in a wet chamber with 50 µl of PBS-10% goat serum solution containing the primary antibody for desired staining. The coverslips were washed by immersion 15 times in a large volume of PBS (100 ml), placed again the wet chamber and incubated for 45 min at room temperature with 50 µl of PBS-10% goat serum solution, having the correspondent conjugated secondary antibody. Finally, the coverslips were washed with PBS as above, the excess of liquid was removed and the preparation was mounted with 2 µl of Prolong (Invitrogen) on glass slides. The samples were examined by confocal microscopy in a Leica TCS SP8 multispectral confocal system. ImageJ software was used for image composition.

### 10 - Attach-and-inject assays

In the experiments bacterial adhesion process was performed prior to injection, infection was carried out in two steps: bacterial adhesion to target cells and injection. Bacteria were grown o/n as previously, but next day cultures were directly diluted in serum-free DMEM to the desired concentration and added over the eukaryotic cells for attachment. After an incubation of 30min/1 hour at 37°C with 5% CO2 in static conditions, unbound bacteria were washed away (if necessary) by washing 5-10 times with serum-free DMEM. Finally, bacteria were induced in serum-free DMEM (containing IPTG or aTc and ARA or aTc) until the injection process occurred and experiment was finished. Injection was assessed by ß-lactamase activity measurement (see "ß-lactamase translocation reporter assay") or culture was long-term monitored (see Examples below).

### 11 - Bacterial infection stop for long-term culture experiments

For assessing in vitro cell killing, apoptosis, or immunogenic cell death after injection of toxins by SIEC, cell culture was monitored at various timepoints until 48 hours post infection. To stop bacterial growth during the long-term following, bacterial infection was stopped 4 hours after induction with DMEM-diluted gentamycin (Corning). For that purpose, culture was washed three times with DMEM, and gentamycin added first at high concentration (100 µg/ml). Plate was incubated for 1 hour at 37°C with 5% CO2 in static conditions and washed with DMEM. Finally, gentamycin was added at a lower concentration (10 µg/ml) diluted in complete DMEM (containing FBS 10%) and incubated at 37°C with 5% CO2 in static conditions until the plate was processed. The presence of FBS in the final media was critical, as the cells should continue growing for several hours. The endpoints assessed in these long-term cultures were cell viability, apoptosis induction and immunogenic cell death, detailed below.

### 12 - Eukaryotic cell viability determination

After injection of different cargo proteins in various experimental conditions, cell culture viability was assessed at various timepoints until 48 hours. For that purpose, the LIVE/DEAD^{™} Viability/Cytotoxicity Kit for mammalian cells (ThermoFisher Scientific) was employed. Shortly, this kit uses calcein and ethidium homodimer (EthD-1). Calcein is well retained within live cells, producing an intense uniform green fluorescence in live cells (ex/em -495 nm/~515 nm). EthD-1 enters cells with damaged membranes and undergoes a 40-fold enhancement of fluorescence upon binding to nucleic acids, thereby producing a bright red fluorescence in dead cells (ex/em -495 nm/~635 nm). EthD-1 is excluded by the intact plasma membrane of live cells. Background fluorescence levels are inherently low with this assay technique because the dyes are virtually non-fluorescent before interacting with cells. If cell viability is visualized on fluorescence microscopy, reagents were added directly over the culture following the supplier indications, incubated 10 minutes in dark at room temperature and observed under a Yoda WF-TIRFM fluorescence microscope using GFP (green) and propidium iodide (red) standard filters. ImageJ software was used for image composition. If viability was assessed by flow cytometry, a minimum of 2 replicates were used per experimental condition. First, culture supernatants were saved apart and cells were harvested from the plate incubating with 0.5 ml of trypsin for 15 minutes. After that, neutralization with its own supernatant was performed for each sample, centrifuged for 1000 rpm 5 min and stained with LIVE/DEAD reagents for 10 minutes in dark at room temperature. After that, samples were washed with PBS, finally resuspended in a minimum of 250 µl of PBS and transferred to cytometry tubes for analysis in the Flow Cytometry Service of the CNB in a Gallios Cytometer (Beckman Coulter) using 488 nm excitation and measuring green fluorescence emission for calcein (i.e., 530/30 bandpass) and red fluorescence emission for ethidium homodimer-1 (i.e., 610/20 bandpass). Results were analyzed using FloJo software.

### 13 - Apoptosis induction assessment

Apoptosis induction after injection of proteins was assessed by detecting active caspases in whole, living cells by using FAM FLICA^{™} Caspase-3/7 Kit (BioRad). This kit contains FLICA reagent, which binds covalently with active caspase enzyme. If there is an active caspase enzyme inside the cell and retain the green fluorescent signal within the cell and unbound FLICA will diff use out of the cell during the wash steps. Apoptotic and pyroptotic cells will retain a higher concentration of FLICA and fluoresce brighter than healthy cells. If the inhibitor of apoptosis Z-DEVD-FM K was employed, it was added after the infection into fresh media at 100 µM final concentration. For developing these assays, a minimum of 2 replicates were used per experimental condition. First, culture supernatants were saved apart and cells were harvested from the plate incubating with 0.5 ml of trypsin for 15 minutes. After that, neutralization with its own supernatant was performed for each sample, centrifuged for 1000 rpm 5 min and stained and washed following FAM-FLICA kit procedure. Propidium iodide (PI) included in the kit was used for staining dead cells at 0.5% v/v following kit procedure. Finally, samples were analyzed by flow cytometry, by excitation at 535 nm and emission at 617 nm for PI, and 490 nm excitation and 535 nm emission for FLICA. Results were analyzed using FloJo software.

### 14 - Immunogenic cell death assessment

For determination of immunogenic cell death, two molecular markers were used: calreticulin membrane exposure and Adenosine 5'-triphosphate (ATP) release to the media. For developing both assays, a minimum of 2 replicates were used per experimental condition. For calreticulin exposure analysis, Anti-Calreticulin-Alexa Fluor^{®} 647 conjugated antibody (Abcam) was employed. First, culture supernatants were saved apart and cells were harvested from the plate incubating with 0.5 ml of trypsin for 15 minutes. After that, neutralization with its own supernatant was performed for each sample, centrifuged for 1000 rpm 5 min and stained with Anti-Calreticulin-Alexa Fluor^{®} 647 conjugated antibody for 30 minutes at room temperature. After washing with PBS, samples were analyzed by flow cytometry using appropriate Alexa Fluor 647 channel (Excitation: 652nm, Emission: 668nm). For ATP release to the media determination, extracellular media ATP was quantified by employing the Adenosine 5'-triphosphate (ATP) Bioluminescent Assay Kit (Sigma-Aldrich). Culture supernatant was saved from each sample and directly tested for ATP presence following the Assay kit procedure. Right after reaction was initiated, the amount of light (luminescence) produced was measured using a microplate reader. Results were represented in arbitrary luminescence units.

### 15 - Implantation of tumors, administration of bacteria and induction of SIEC in mice

Experiments with mice were performed following the protocols stablished by the CNB Ethics Committee for Animal Experimentation, following the authorized project with reference PROEX 074/18, and the Synlogic/MisproBiotech's Institutional Animal Care and Use Committee (IACUC), in compliance with the national and European and American experimental animals' procedures legislation framework. Mice used in this work were athymic female Hsd:Athymic Nude-Foxn1 nu mice. Implantation of tumors was carried out by subcutaneous injection of 0.1 ml solution of cells (at indicated concentrations) in PBS containing 20% (v/v) Matrigel (BD Biosciences) in the abdominal right flank of each mouse. Tumor size was monitored at different timepoints using calipers, and tumor volume calculated using the formula: width2×Iength×0.52 until the end of the experiment. When bacteria were administered, a concrete tumor volume must have been reached. In this work, bacteria were administered when the number of mice necessary for the experiment presented a tumor size mean around 120 mm3, with 90% of the mice bearing tumors between 80 mm3 and 150 mm3. Bacteria were grown overnight in glass flasks at 37°C in static conditions, and next morning washed 3 times in PBS and diluted to the appropriate concentration in preheated PBS for preparing the injectable solution. For assuring the concentration of this injectable solution, bacteria were counted on a Cellometer and/or plated for next day colony counting. Finally, bacteria were dosed by intratumoral injection of 40 ul of the injectable solution per mouse. This process was repeated every three days, three doses in total. If necessary, mice food was supplemented with wet food during the process. The induction of the translocation was performed by intraperitoneal injection of aTc every day of the experiment since the moment the bacteria is delivered for the first time. 0.1 ml of aTc diluted in PBS, adjusted to administer a total of 10 µg of aTc per mice, were injected in each mouse. If bacteria were administered the same day, aTc injection was performed a minimum of 4 hours later.

### 16 - Statistics

Statistical studies showed in this work were performed using Prism software (GraphPad Software Inc). The specific statistical analysis used for each experiment is detailed in the correspondent figure letter. Data were considered significantly different when P <0.05.

### Example 1 - Translocation of natural T3SS effectors by the Synthetic Injector E. coli (SIEC) strain.

We assessed the ability of SIEC strain to deliver T3SS natural effectors from EPEC to the cytoplasm of eukaryotic cells. For a better characterization of the injection capacity of SIEC, we aimed to quantify the translocation levels of the effectors Tir, NleC, EspH and Map. For this purpose, we fused the ß-lactamase enzyme to the C-terminal part of each effector as a reporter (Figure 1A). If the cargo protein fused to ß-lactamase is successfully delivered to the cytoplasm of the eukaryotic cell, the ß-lactamase enzyme would transform the green-fluorescent reagent in a product that emits blue fluorescence. The prevalence of blue fluorescence compared to green fluorescence indicates delivery of the reporter to the cytoplasm of the eukaryotic cells has been accomplished (see Material and Methods). The gene encoding for the CesT chaperone was placed downstream of each fusion gene in a bicistronic design that was under the control of the promoter PBAD that is induced with L-arabinose (ARA). The four constructs were integrated into the SIEC and SIECΔescN genome in the ypjA chromosomic locus resulting the strains SIEC (ΔescN) ypjA::Bla, SIEC (ΔescN) ypjA::tir-bla, SIEC (ΔescN) ypjA::nleC-bla, SIEC (ΔescN) ypjA::espH-bla and SIEC (ΔescN) ypjA::map-bla (Figure 1A). The SIECΔescN mutant is defective in the ATPase EscN that is required for the functionality of the injectisome, thus its derived strains were used as negative controls of translocation. For the translocation assays, induced cultures of the generated strains with IPTG (Isopropyl β-D-1-thiogalactopyranoside, for expression of the injectisomes) and ARA (L-arabinose, for expression of the corresponding fusion effector-bla) were used to infect HeLa cells. After the infection occurred for 3 hours, we measured ß-lactamase activity inside the cell as an indicator of the translocation levels of the effectors fused to the reporter. Differences observed between each SIEC strain and its correspondent SIECΔescN strain control represent T3SS-dependent translocation. SIEC was able to translocate the 4 effectors (Figure 1 B). As expected, the SIECΔescN derived strains that lack functional injectisomes were not able to translocate any of the effectors showing background translocation levels similar to uninfected cells.

These results indicate that SIEC derived strains were able to translocate, besides Tir, other T3SS effectors. Likewise, we intended to test whether the translocated effector EspH by the strain SIEC ypjA::espH-3HA (SIEC EspH) was active in the cytoplasm of Her14 cells (mouse fibroblasts). EspH has been reported to trigger cytoskeletal modifications, such as destruction of tight junctions, that eventually causes cell rounding in the cells infected (Wong, ARCet al. 2012 mBio 3(1)). This phenotype contrasts to the elongated shape cells, and specially fibroblasts, exhibit in regular conditions. Thus, this constitutes an easily detectable characteristic that could be observed by brightfield microscopy. For that aim, we built another construct to co-express EspH alone without the bla reporter and the chaperone CesT (Figure 2A) to be integrated in the ypjA locus of SIEC resulting the strain SIEC ypjA::espH-3HA. A SIEC-derived strain expressing a control cargo (SIEC pBAD18 Tir100-NbGFP-Bla) was used as control strain. Induced cultures of both strains were used to infect Her14 cells, and morphological cell changes were monitored by acquiring microscopy images in a time-lapse sequence. We observed a clear cell-rounding phenotype of the Her14 cells at 4 hours post infection with (strain SIEC ypjA::espH-3HA), while no changes in morphology were observed in the infected cells with SIEC (Figure 2B).This result demonstrates that the effector EspH was functional after being translocated into eukaryotic cells through the injectisome of SIEC.

### Example 2 - Designing expression cassettes for efficient delivery of cargo proteins through the injectisome of SIEC

One of the main objectives of this work was to engineer SIEC to translocate any desired cargo proteins. We tested different secretion signals based on the N-terminal part of EPEC effectors. Specifically, we designed Tir30 and Tir100 secretion signals, which correspond to the first 30 and 100 amino acids (N-terminal part) of the effector Tir, respectively. We also used the EspF20 secretion signal that consists in the 20 first amino acids of the effector EspF. The secretion signal Tir100 also includes the binding site for the chaperone CesT, but not in the case of Tir30 nor EspF20. The three N-terminal secretion signals were fused to a GFP- binding nanobody (NbGFP) (Blanco-Toribio A et al. 2010 PLoS ONE 5(12)) that would act as heterologous cargo protein. This cargo was fused to the reporter ß-lactamase at the C-terminal part for quantification of the translocation levels. To also assess the contribution of CesT in translocation efficiency, we included downstream the gene encoding the chaperone in the construct with the Tir100 secretion signal, in a bicistronic configuration (Figure 3A).

The different constructs were cloned in the plasmid pBAD18 under the control of the PBAD promoter, and therefore inducible by ARA. The resulting plasmids pBAD18 Tir30-NbGFP-Bla CesT, pBAD18 Tir100-NbGFP-Bla CesT and pBAD18 EspF20-NbGFP-Bla CesT (Figure 3A) and the negative control of ß-lac with no secretion signal pBAD18-Bla were used to transform SIEC and SIECΔescN strains. We set up the infection by using HeLa cells and the SIEC strains carrying the different constructs. Bacterial cultures were first preinduced to express the cargo proteins and the T3SS injectisome, and then added over the eukaryotic cells for 3 hours. All constructs allowed cargo proteins to be translocated (Figure 3B). The detected protein translocation into cells was dependent of an active injectisome since the control strain SIECΔescN showed no translocation. The expression of these constructs determined by western blot showed that they were produced at similar levels in all the strains (Figure 3C), demonstrating that any differences observed in translocation were not due to differences in expression.

These results indicate that SIEC was able to efficiently translocate cargo proteins into eukaryotic cells when these proteins presented a T3SS secretion signal.

### Example 3 - Translocation of cargo proteins expressed from genes integrated into the genome

The same constructs previously tested in plasmids were inserted in the ypjA genomic locus (Figure 4A) of SIEC and SIECΔescN, except for the fusion with the EspF20 secretion signal. The resulting strains were named SIEC ypjA::Tir30-NbGFP-bla and SIEC ypjA::Tir100-NbGFP-bla CesT. HeLa cells were infected with the different induced bacteria as before. In this case, we only detected translocation levels above the background in HeLa cells infected with the strain SIEC ypjA::Tir100-NbGFP-bla CesT that harbored the Tir100 fusion to the nanobody and the cesT gene (Figure 4B). We also checked the translocation differences were not due to differences in expression of the different constructs by western blot. (Figure 4C).

As an additional demonstration of cargo protein translocation, we tested the presence of Tir100-NbGFP-Bla fusion protein in the Her14 cells cytoplasm infected with SIEC ypjA::Tir100-NbGFP-bla CesT by western blot. The fusion protein was detected in the cell extracts of induced bacteria of SIEC ypjA::Tir100-NbGFP-bla CesT and SIECΔescN ypjA::Tir100-NbGFP-bla CesT (Figure 5A), but only the cell extract of eukaryotic cells infected with SIEC ypjA::Tir100-NbGFP-bla CesT presented the fusion (Figure 5B).

The construct design with Tir100 secretion signal and CesT to mediate translocation of cargo proteins was selected as scaffold for translocation of further cargo proteins.

### Example 4 - CesT chaperone contribution to protein delivery

For better understanding of the T3SS delivery process with SIEC, we wanted to study the contribution of the chaperone CesT to the translocation of cargo proteins. With this purpose, we integrated into the ypjA locus of SIEC the genes coding for heterologous fusion proteins composed by the NbGFP with the secretion signals Tir30 and Tir100, resulting in strains SIEC Tir30-NbGFP-bla and SIEC Tir100-NbGFP-bla, respectively (Figure 6A). Then, we transformed these strains with the plasmid pBAD18 CesT plasmid containing the gene cesT to compare the effect of the presence of CesT on translocation levels. It is important to remark that the secretion signal Tir100 contains a region where CesT is described to bind, but Tir30 does not contain this region. Nevertheless, when HeLa cells were infected with the different transformed and non-transformed strains, we observed that the expression of CesT increased the translocation levels in both strains, suggesting that CesT might improve translocation of the fusion protein by a different mechanism other than binding this protein.

### Example 5 - Translocation of a cargo protein repertoire of therapeutic cargos by SIEC.

We chose proteins from various organisms of different sizes and functions in order to test the capacity of the engineered SIEC strain to deliver diverse protein cargos. The cargo proteins that formed the injection repertoire in this work were the nanobody anti-GFP (NbGFP), the peptides BIM and tBID, granzyme B (Granz. B), ovoalbumin (OVA), survivin, thymidine kinase (TK) and Sox2. (Figure 7A). These proteins were selected for their therapeutic potential.

For the translocation of this set of cargo proteins, we devised a genetic modular backbone in which we would incorporate the corresponding nucleotide sequence coding region for each cargo protein. This backbone was formed by an ORF coding for a fusion protein comprising the Tir100 secretion signal, the cargo protein and the reporter ß-lactamase, followed downstream by the gene cesT in a bicistronic configuration. The coding nucleotide sequence for the heterologous cargo was flanked by the restriction sites of Sfil and Notl for allowing specific insertion in this location (Figure 7A), The recognition sequences of these restriction enzymes were unfrequently found in natural DNA sequences, favoring that they would be unique in the whole construct. Finally, the construct was cloned into the pBAD18 plasmid under the regulation of the PBAD promoter that is inducible by ARA. Overall, this engineered construct would represent a synthetic module implemented in the bacterial strains used in this work for cargo delivery, constituting the "cargo module" of SIEC (Figure 7A).

We performed the translocation assays to Her14 cells with the same induction conditions used in previous experiments, using the SIEC and SIECΔescN strains transformed with the different plasmids. As a control of the translocation, we used the plasmid expressing the fusion with NbGFP that was previously used for construct optimization. We were able to detect injection of 7 cargo proteins from the repertoire with different translocation levels: BIM and Survivin were translocated at the highest levels (around 9 units), followed by tBID (7.5 units). On the other hand, Granzyme B, Ovoalbumin (OVA), the herpes simplex virus thymidine kinase (HSV-TK) and Sox2 were translocated at lower levels, still showing a signal around 5 units (Figure 7B). Thus, the generation of the injection repertoire demonstrates that SIEC is able to translocate a wide range of proteins non-related to the T3SS and from different origins to the cytoplasm of eukaryotic cells.

### Example 6 - Flagela-T3SS interference and the relevance of controlling T3SS expression for achieving efficient attachment to target cells via the SA

Previous observations indicated the component FliC from the bacterial flagella was strongly reduced upon induction of SIEC, but not in the case of SIECΔp1 strain, which is not able to assemble the T3SS. This result suggested that expression of T3SS injectisomes in SIEC was interfering with the expression of the flagella. Reduction of FliC in SIEC was more intense in the presence of the inductor IPTG was added, but even its the absence the leakiness detected of T3SS expression induced a drop in the flagella levels compared to the parental strain EcM1. As a consequence, compared to the parental strain EcM1, the motility of SIEC strain was found to be reduced to about to 50% upon expression of the T3SS. Given the structural similarities between the flagellum and the T3SS, we speculated that this interference could be in the assembly of these nanomachines. Thus, we hypothesized that the reduction in the flagella levels found in SIEC would constitute a major drawback for the combination of SIEC T3SS protein delivery with the targeted cell-specificity provided by the synthetic adhesins. In fact, when a synthetic adhesin binding EGFR receptor was expressed in SIEC (SIEC SAegfr), we observed a very weak adhesion of bacteria to the EGFR+ colon carcinoma cells HCT116. In order to solve this severe limitation, we proposed that a genetic regulatory circuit that would tightly control the expression of the T3SS, reducing unwanted leakiness expression of the T3SS before bacterial adhesion to the tumor cell occurs, could provide a way to allow an effective combination of the synthetic adhesins targeted adhesion with the T3SS protein delivery. This regulatory circuit should not only repress expression before cell contact, but also be able to induce the T3SS injectisome and injected proteins once bacteria are attached to the cell. With this aim, we designed a synthetic regulatory circuit that tightly repress the T3SS components, leading to the generation of the SIEC X strain (see examples below). The efficient repression of the T3SS in this strain was translated in an improved motility of SIEC X, close to that of the parental unmodified strain EcM1 (Figure 8). The control provided by the regulation circuit in SIEC X SAegfr allowed this strain to specifically adhere to EGFR+ HCT116 cells in high numbers, as a first step in the attach-and-inject process. The genetic switch allowed T3SS injectisome to be silent avoiding its interference with the flagella and motility, thus enabling a correct adhesion process to a target cell via the Synthetic Adhesins.

### Example 7 - Designing a triple repressor genetic circuit for tight control of the T3SS machinery in SIEC

As previously presented, the expression of the T3SS injectisome in SIEC is driven by 5 engineered operons (eLEEs) that contain all the necessary genes for the assembly of this macromolecular complex. The expression of these 5 operons was controlled by the promoter Ptac, inducible by IPTG to the culture. Although the Ptac promoter was proven useful for the expression of the injectisome in SIEC, it has important limitations like a significant leakiness and the impossibility of using the inductor in vivo. To overcome these limitations, a genetic regulatory circuit that was inducible with anhydrotetracycline (aTc) and with the capacity of tightly regulate the transcription of the five synthetic operons was engineered. For that purpose, a triple repressor circuit which culminates with the expression control of the global repressor Lacl was conceived. Lacl protein would be, as in the original regulation, the agent that inhibits the expression of the eLEE operons. To optimize the system, the endogenous *lacl* gene was deleted and the gene lacl^{W220F} was included in the circuit. This version presents the mutation W220F that confers a 10-fold reduction in leakiness. The repressor Lacl^{W220F} would control the expression of the eLEE operons. The gene lacl^{W220F} was placed under the control of the strong inducible promoter PR. This promoter is controlled by the repressor cl from the lambda bacteriophage. This repressor is described to cleave itself during the SOS response to DNA damage in *E. coli.* To avoid this phenomenon, a special variant of cl ind- (cl mutant E118K) that was less susceptible to autodigestion (Gimble and Sauer 1986) was employed in the circuit. Finally, the expression of cl ind- was controlled with the Ptet promoter and its repressor TetR that is ultimately controlled by the inductor aTc. The location of the genetic elements in the circuit is depicted in Figure 10. A strong RBS (0030) was utilized for the translation of the genes cl ind-and IacIW220. The T0 terminator downstream thet tetR gene and the T1 terminator downstream cl ind- gene were also added. Every component of this genetic circuit was engineered in a modular way. Each genetic part including RBS and terminators are flanked by unique restriction sites so they can be replaced or eliminated if necessary.

The whole system would be derepressed with the inductor aTc that would bind the repressor TetR freeing the Ptet promoter driving the expression of cl ind-. This repressor would act over the promoter PR inhibiting the transcription of Lacl^{W220F}. Consequently, the Lacl^{W220F} levels in the cell would drop fully inducing the Ptac promoters of the eLEE operons.

The genetic circuit was integrated in the curli locus of SIEC generating the strain SIEC I.

### Example 8 - Generation of different versions for optimization of the genetic circuit: Original circuit design: the derrepression problem.

To test the functionality of the genetic circuit in SIEC I, the expression of the Lacl^{W220F} repressor and the T3SS proteins EspA, EspB and EscC was determined by western blot (Figure 9). In this assay there were included as controls: the SIEC strain, inducible by IPTG addition, and the mutant SIECΔ*lacl*. This mutant lacks the repressor Lacl, and consequently, T3SS components are constitutively induced in this control strain.

The expression of the global repressor Lacl/Lacl^{W220F} in the presence of the inductors IPTG and aTc was evaluated. Lacl production at endogenous levels was not detected in SIEC cell extract. In the absence of the inductor aTc, Lacl^{W220F} that was under the strong promoter P_{R} was detected in SIEC I cell extract. When the inductor aTc was added to the culture of SIEC I, the Lacl^{W220F} signal was no longer detected, as the cl ind- repressor produced inhibited the expression of Lacl^{W220F}, demonstrating the circuit interactions were working as predicted (Figure 9). As expected, no Lacl signal was observed in the case of the control strain SIECΔ*lacl*.

Besides testing the expression of the global repressor Lacl/Lacl^{W220F} in the different SIEC strains, the production of the T3SS proteins EspA, EspB and EscC was also evaluated as indication of the control of the expression of the injectisome that is the main output of the system. The genes coding for these threeT3SS components are located in 2 different eLEE operons: escC in eLEE2 and espA and espB in eLEE4. EscC is a structural component of the injectisome, thus it can be found in the cell extracts. The protein EspA forms the injectisome filament and EspB is part of the translocon in the tip of the injectisome. The detection of specific EspA and EspB signals in the culture supernatants implies that the injectisome is assembled and functional, since the active secretion of these components to the extracellular media is only possible when this macromolecular complex is completely formed and active. As previously described, in the absence of IPTG, these T3SS components were still detected in SIEC due to the high leakiness of the P_{tac} promoter. This was more evident in the case of EscC in the cell extract, but it is also observed for EspA and EspB proteins in the culture supernatant. When IPTG was added to the SIEC culture, intense signal levels of all the three T3SS proteins tested was detected (Figure 9). As expected, in the case of the unrepressed mutant SIECΔ*lacl*, T3SS proteins were continuously produced, regardless whether the inductor molecule was present or not. In the case of the strain carrying the engineered regulation circuit, SIEC I, high repression was observed when the inductors (IPTG to directly inhibit Lacl^{W220F}, or aTc to turn on the whole regulation circuit) were not added to the culture. Nevertheless, no efficient T3SS proteins production was accomplished upon addition with either of the inductors, indicating that the system was not efficiently activated (Figure 9). We hypothesized that, in the conditions tested, non-detected remnant Lacl ^{W220F} repressor was still present after turning off the *lacl ^{W220F}* gene, which would avoid complete derepression of the eLEE operons. This points out the necessity to improve the circuit so the amount of Lacl^{W220F} drops to levels that cannot any longer repress the Ptac promoters of the eLEE operons.

### Example 9 - Optimization of the genetic circuit by using ssrA degradation tags fused to Lacl^{W220F}

To decrease the levels of the remnant repressor Lacl ^{W220F} upon inductor addition and achieve complete derepression of the expression of the eLEE operons, the ssrA degradations tags -LAA, -AAV and -ASV were fused to the C-terminal part of the Lacl^{W220F} protein. Fusion to these three degradations tags were previously described to decrease GFP half-life to 40 min (-LAA tag), 60 min (-AAV tag) or 110 min (-ASV tag) in *E. coli,* where GFP is normally completely stable at least for 225 min without degradation tags. The genes encoding the Lacl ^{W220F} proteins with the degradation tags were included in the genetic circuit creating three new versions of the circuit that were integrated in SIEC genome generating SIEC III, SIEC XI and SIEC X strains (Figure 10). The capacity to control the expression of the eLEE operons of these new versions in comparison to the original construct was determined as before by western blot analysis of the production of Lacl^{W220F}, EscC, EspB and EspA in cultures of the different SIEC strains.

As observed before, high levels of Lacl^{W220F} were detected in non-induced SIEC I cultures (OFF state). Remarkably, no Lacl^{W220F} signal was detected in non-induced cultures of SIEC III and SIEC XI indicating that the tags -LAA and -AAV were leading to the rapid degradation of Lacl^{W220F}. In concordance to this result, strong leaky expression of the T3SS components was observed in these non-induced cultures, confirming that the amount of Lacl^{W220F} of these strains was not sufficient to maintain the repressed OFF state. On the contrary, Lacl^{W220F} was detected in non-induced SIEC X cultures that carried the version with the weakest degradation tag tested -ASV, and accordingly, the leakiness of the expression of the T3SS components was low. This result indicates that the levels of Lacl^{W220F} were enough for maintaining a good OFF state in this version. Consequently, the optimal tag for our purposes was -ASV, while the other two candidates were too strong to maintain detectable Lacl^{W220F} repressor levels, denoting in these circuit versions the repressed OFF state could be compromised.

When the inductor aTc was added to cultures of the strain SIEC X (ON-state), the expression levels of the T3SS components were higher than in induced cultures of SIEC I (Figure 10). Although this protein expression was still low compared to induced cultures of the parental SIEC strain, the reduction of the half-life of the remaining Lacl^{W220F} after inducing the system resulted in an improved-ON state, therefore approaching to the desired scenario.

In order to get full activation of the system, new versions of the regulation circuit were engineered. The rationale of the new versions design was to decrease the expression levels of Lacl fused to the ssrA tag -ASV, so that the ON state could be optimized maintaining a good OFF state in the absence of the inductor. For that purpose, a weaker RBS (RBS0034) upstream the gene *lacl* was tested in comparison to the RBS0030 used until now. The version with the RBS0034 was integrated in SIEC chromosome generating SIEC XII. Also, it is known that switching the transcription codon start from ATG to GTG makes the protein to be less efficiently expressed, and therefore the use of RBS0034 combined with the GTG codon start was also assessed (SIEC XIII). The Lacl expression levels in non-induced cultures of the different SIEC strains showed a gradual decrease following the order SIEC I > SIEC X > SIEC XII > SIEC XIII, indicating that the expression attenuation strategy was working as expected.

The functionality of the new circuit versions regulating the expression of T3SS components was also examined. The SIEC strains with the new versions that presented reduced Lacl expression in OFF state, showed higher expression of the T3SS components in comparison to SIEC I upon induction (ON state), especially in SIEC XIII that had the version with the lowest Lacl expression (Figure 11). However, as the induction increased, the leakiness of the system also increased. Consequently, genetic circuits with stronger ON states were generated, but with weakened expression control in OFF state.

Overall, these results indicate that the Lacl regulatory genetic circuit present in SIEC X shows an improved OFF state while maintaining efficient T3SS components expression by induction with aTc in ON state.

Western blot expression signals of the repressor Lacl and the T3SS components EspA, EspB and EscC from 3 independent induction experiments were quantified (Figure 10).

The T3SS structural component EscC detected in cell extracts of SIEC X was 33% more expressed compared to SIEC parental strain when induced, but maintaining EscC levels more than 50% lower when the inductors were not present (Figure 12A). Repressor Lacl levels of SIEC X compared to SIEC I were also lower in the cell extracts, probably due to the presence of the degradation tag compared to the original, but still able to efficiently repress the system.

Noticeably, in the supernatant fraction almost non-detectable EspA or EspB expression signal was observed in SIEC X prior to induction. However, when the system was induced, levels of these proteins were quantified in around 60% of the expression in parental SIEC strain in the case of EspA, and 92% in EspB (Figure12 B y C).

Finally, to evaluate the capacity of SIEC X to translocate proteins into eukaryotic cells, we performed a β-lactamase translocation assay with 3T3 cells (Figure 13). For that, we transformed SIEC, SIEC I, SIEC X and SIECΔ*escN* with the plasmid pBAD18 EspF20-Bla. This plasmid contained the secretion signal EspF20 fused to the reporter β-lactamase, and it is induced by ARA addition. We intended to evaluate the β-lac translocation capacity of the different strains carrying the different versions, and compare bacteria presenting functional T3SS injectisomes and preinduced β-lac (by using aTc and ARA) with bacteria with non-induced T3SS injectisomes, just preinduced β-lac (only inducing with ARA). This latter scenario would give us relevant information about the implications of T3SS leakiness expression in translocation with the different regulation levels of the different genetic circuits present in the different SIEC strains when the cargo is preinduced but T3SS operons are not. After performing this experiment, we could detect high translocation levels with the original SIEC when aTc was not present, calculated in 75% of the levels observed when the expression of T3SS injectisomes were induced (Figure 13). Importantly, the translocation leakiness of SIEC X was calculated in 50%, while reaching similar levels of translocation upon induction compared to original SIEC (around 90%). As expected, cells infected with induced SIEC I bacteria showed almost non-detectable levels of translocation.

The overall results of these characterization experiments of the different genetic circuit versions indicate that the Lacl regulatory genetic circuit present in SIEC X allows for an efficient translocation of proteins when expression of the T3SS injectisomes is achieved upon induction with aTc, while maintaining an improved repressed state when aTc is not added. Consequently, SIEC X was chosen as a particularly suitable chassis for therapeutic purposes.

### Example 10 - Specific injection to cells expressing EGFR by specific attachment of SIEC X SAegfr.

After confirming the specific adhesion of SIEC X SAegfr to EGFR expressing cells, we wanted to elucidate if this specific attachment was translated into specific injection to these cells in comparison to cells that do not express EGFR. For that, we designed an experiment in which we infected both 3T3 cells (without EGFR) and Her14 cells (with EGFR) with SIEC strains carrying the plasmid pBAD18 Tir100-Vgfp-Bla and expressing the adhesin against EGFR (SAegfr), and an adhesin control with no target specificity (SAcontrol). As negative translocation control we included SIEC pBAD18 Bla and SIECΔ*escN* SAegfr pBAD18 Tir100-Vgfp-Bla. After the infection, bacteria were let to adhere for 30 minutes, and then, we extensively washed the culture to get rid of the non-attached bacteria. After that, we induced the expression of the T3SS injectisomes and the cargo for 3 hours. At the end of the experiment, we could detect β-lac translocation signal only in the Her14 (express EGFR) infected with SIEC expressing the Synthetic Adhesin against EGFR and able to translocate the cargo (strain SIEC SAegfr pBAD18 Tir100-Vgfp-Bla), demonstrating specific injection upon specific attachment to these cells (Figure 14).

### Example 11 - Injection enhancement using specific and non-specific Synthetic Adhesins

We also aimed to further investigate the role of the Synthetic Adhesin for targeting cells by using the SIEC strain with the Synthetic Adhesin against the EGFR membrane receptor (SIEC X SAegfr ypjA::Ptet-Tir100-NbGFP-Bla) and SIEC containing the non-specific SAtir (SIEC X SAtir ypjA::Ptet-Tir100-NbGFP-Bla), to infect the EGFR-expressing cells Her14 and HCT116, and 3T3 cells without EGFR. As at this point, we wanted to assess whether the specific SAegfr enhances the injection to target cells by SIEC X SAegfr, we did not remove the unbound bacteria from the media after the adhesion. We could observe that in 3T3 cells that do not have the ligand EGFR, the presence or not of the specific adhesin SAegfr in the bacteria did not result in a variation in the translocation levels (Figure 15). On the contrary, when we used cells that express EGFR (Her14 and HCT116), we observed that the translocation levels increased around 100% when the cells were infected with SIEC containing SAegfr. These data indicate that the presence of the specific Synthetic Adhesin against EGFR (SAegfr) on the surface of SIEC X enhanced the injection capacity of this strain to cells expressing the cognate antigen.

### Example 12-Translocation of protein cargos through T3SS injectisome for in vitro killing of tumor cell lines: Translocation of pro-apoptotic cargos from the heterologous protein repertoire

After evaluating the capacity of SIEC to inject EPEC effectors and cargo proteins, we aimed to elucidate if some of these proteins described to cause apoptosis and expressed in the optimal strain SIEC X SAegfr could effectively be used to kill tumor cells *in vitro.* For that, we infected HCT116 colon carcinoma cells with SIEC X SAegfr strains expressing the natural effectors Tir and Map from the chromosome, and the pro-apoptotic peptides BIM and tBID fused to the Tir100 secretion signal, from plasmid. The different cargos were used this time without the reporter β-lactamase. In this assay, we used staurosporin (STS) as a positive control for cell killing, and SIEC X SAegfr injecting NbGFP from plasmid as a negative control. For monitoring cell death, the fosfatidilserine exposure on the external part of the eukaryotic cell membrane, which is a widely used apoptosis marker, was determined using the protein Anexin V that specifically binds this molecule. The Anexin V can be detected by flow cytometry and the percentage of apoptotic cells can be assessed.

After performing an attach-and-inject assay, we could observe that the injection of some of these proteins increased the percentage of apoptotic cells in culture 24 hours post infection compared to the negative control. Specifically, in the case of the protein BIM the translocation of this protein provoked 12% of the infected cells to become apoptotic, while translocation of the control cargo NbGFP caused less than 5% of cells to enter programmed cell death (Figure 16). However, this effect was not strong enough and the cell cultures infected with the different strains continue growing normally 24 hours post infection. On the contrary, the viability of STS treated cells was severely compromise (Figure 16). This experiment remarked the necessity of using proteins that efficiently cause cell death or irreversible apoptotic process upon translocation, as tumor cells are described to have internal mechanisms that could revert the apoptotic effects we observed.

### Example 13-Translocation of protein cargos through T3SS injectisome for in vitro killing of tumor cell lines: Translocation of ADP-ribosylating cytotoxins by SIEC

In order to generate effective cell death in the tumor cell culture upon delivery of the cargo by SIEC, we evaluated the translocation of the ADP-ribosyltransferase (ART) toxins ExoA and TccC3 that have been reported to present strong cytotoxic properties.

In the case of ExoA, we only used the ADP-ribosyltransferase domain that catalyzes the inactivation of the eEF-2 in the cytoplasm of the infected cell. This domain comprises part of the structural domain Ib and the whole domain III formed in total by 213 amino acids located in the C-terminal part of the protein. This region was named PE25 *(Pseudomonas* Exotoxin of 25 kDa molecular weight). As with other proteins, the coding DNA region for PE25 was cloned in the optimal construct (Tir100-cargo-bla cesT) for translocation between the coding region of Tir100 secretion signal and the β-lactamase reporter (Figure 17A). For the protein TccC3, we used the hypervariable region (hvr) that has the ribosyltransferase activity and it is also located in the C-terminal part. Thus, the TccC3 hvr coding region was inserted in the cargo module fused to β-lactamase for assessing translocation (Figure 17B). The resulting cassettes with PE25 and TccC3 hvr were integrated in the *ypjA* genome locus of SIEC X SAegfr generating the strains SIEC X SAegfr *ypjA*::Tir100-PE25-Bla and SIEC X SAegfr *ypjA*:Tir100-TccC3ₕᵥᵣ-Bla, respectively. The two constructs were also integrated in SIEC X SAegfr *ΔescN* to generate control strains for each case.

We performed the translocation assay using the tumor cell line HCT116 allowing the bacteria to attach to the cells for 1-hour, followed by the induction of the expression of the cargo and the T3SS injectisomes for 3 hours. After measuring the translocation levels, we detected delivery of both ART-toxin fusions with the SIEC strains (Figure 23C).

After verifying that PE25 and TccC3ₕᵥᵣ fusions could be delivered into HCT116 tumor cells by the SIEC X SAegfr derived strains, we wanted to test the cytotoxic effect of these translocated proteins *in vitro.* In order to do that, we removed the b-lactamase reporter from both fusions and the new cargo modules were cloned into pBAD18 generating the plasmids pBAD18 Tir100-PE25 and pBAD18 Tir100-TccC3. Both plasmids were used to transform SIEC X SAegfr driving the expression of the fusions Tir100-PE25 and Tir100-TccC3ₕᵥᵣ. With the transformed SIEC X SAegfr strains, we performed an attach-and inject experiment with HCT116 cells letting the injection occurred for 4 hours. At the end of the injection time, we could observe cell rounding morphology of the HCT116 infected with the bacteria expressing Tir100-TccC3ₕᵥᵣ. This was probably due to the actin-crosslinking effect that this protein is reported to cause when delivered into the cytosol of eukaryotic cells. We stopped the infection with gentamycin to avoid bacteria overgrowth, and we followed the cell culture 24 hours post infection to evaluate toxicity. By staining the cells with the LIVE/DEAD reagent and observing the cells cultures with fluorescence microscopy we could detect a dramatically decrease of the number of living cells (green cells) at 24 post infection in cell cultures infected with bacteria expressing any of the toxin-fusions. On the other hand, cells infected with the SIEC X SAegfr expressing the fusion control Tir100-NbGFP showed no apparently reduction in the number of living cells, but multiplication of the cells in the same time lapse.

Once the cytotoxicity effect of these the toxin fusions Tir100-PE25 and Tir100-TccC3ₕᵥᵣ expressed from plasmid was confirmed, we wanted to assess whether the capacity of triggering this cytotoxic effect is maintained when the toxin fusions are expressed from genes integrated in the genome of SIEC X SAegfr as the final strain to be used for therapeutic purposes should have all the engineered components integrated in the bacterial chromosome. The expression cassette for each fusion was integrated in the *ypjA* genomic locus of SIEC X SAegfr generating the strains SIEC X SAegfr *ypjA*:Tir100-PE25 and SIEC X SAegfr *ypjA*::Tir100-TccC3. An attach-and-inject infection assay was performed with the new generated strains as described above following the cell viability for 48 h. In this case we also observed cytotoxicity, but it was more evident at 48 hours post infection. The Tir100-TccC3ₕᵥᵣ toxicity effect appears to be slightly lower than in the case of Tir100-PE25.

### Example 14-Translocation of protein cargos through T3SS injectisome for in vitro killing of tumor cell lines: Bacterial doses needed for triggering efficient tumor cell killing in vitro

In the strains SIEC X SAegfr *ypjA*::Tir100-PE25 and SIEC X SAegfr *ypjA*::Tir100-TccC3 the expression of the injectisomes was induced with aTc and the expression of the toxin-fusions with ARA. To induce both components with aTc and this way simplify the induction protocol, the P_{BAD} promoter of the cargo module was replaced by the Pₜₑₜ and the new expression cassettes were integrated in the *ypjA* locus of SIEC X SAegfr. The resulting strains were named SIEC X SAegfr *ypjA*:Ptet-Tir100-PE25 and SIEC X SAegfr *ypjA*:: Ptet-Tir100-TccC3. For quantifying the cell-killing efficiency of induced cultures of both strains, an attach-and-inject assay was performed with HCT116 cells at different multiplicities of infection (MOI). As negative control, the strain SIEC X SAegfr *ypjA*::Tir100-NbGFP-bla was used. The tumor cells were stained with the LIVE/DEAD reagent at different times post infection and the percentage of living cells were determined by flow cytometry (Figure 19). As expected, for each translocated toxin fusion the lowest percentage of viable tumor cells was detected at 48 hpi in cell cultures infected at the highest bacterial dose (MOI 100:1). Remarkably, we detected cytotoxicity with both toxin fusions even in cell cultures infected at a MOI of 1:1 at 48 hpi. We observed a clear dose-response for both strains as the cell killing levels followed the tendency 100:1 > 10:1 > 1:1. Expectedly, the longer the incubation time the more cytotoxicity was detected. Interestingly, the strain SIEC X SAegfr *ypjA*::Tir100-PE25 seemed to require more time to kill cells, as the highest drop in culture viability (50%) occured from 24 to 48 hpi, while upon injection of Tir100-TccC3ₕᵥ the viability decrease was more evident from 4 to 24 hours. TccC3 injection seems to be more effective for tumor cell killing at high doses (100:1), though its effect is reduced drastically at 10:1 and 1:1 MOI, while ExoA cytotoxicity is higher at 100:1, but experience less reduction when lowering the bacterial dose (Figure 19).

### Example 15-Translocation of protein cargos through T3SS injectisome for in vitro killing of tumor cell lines: Mechanism of cell killing and immunogenic cell death upon injection of ADP-ribosylating cytotoxins

In order to study the mechanisms of toxicity of the translocated fusions, we carried out a set of experiments with different molecular probes. The FLICA staining detect the activation of caspases that occurs during apoptosis. Culture cells infected with different induced culture strains were treated with LIVE/DEAD reagent and FLICA, and analyzed by flow cytometry to determine percentages of live cells, dead cells and apoptotic cells. We could detect an important activation of caspases 24 hpi in cells infected with SIEC X SAegfr *ypjA*::Ptet-Tir100-PE25, while cells infected with SIEC X SAegfr *ypjA*::Ptet-Tir100-TccC3 did not show a significant activation of these proteases (Figure 19A). These results suggest that translocated Tir100-PE25 triggered apoptosis to kill HCT116 cells. On the other hand, translocated Tir100-TccC3 appeared to induce cell death by a different mechanism, that is faster and probably not related directly to apoptosis. This hypothesis was also tested by adding a pan-caspases inhibitor (Z-VAD-FMK) to the culture after the injection process. We observed that the viability of cells infected with SIEC X SAegfr *ypjA*:Ptet-Tir100-PE25 was affected in the presence of this inhibitor at 24 hpi, showing similar viability levels compared to cells infected with SIEC X SAegfr *ypjA*::Ptet-Tir100-NbGFP-bla negative control (Figure 19B). On the contrary, the inhibitor had no effect in protecting cells infected with SIEC X SAegfr *ypjA*::Ptet-Tir100-TccC3. All together, these results supports that the translocated Tir100-PE25 induced caspase-mediated apoptosis cell death but Tir100-TccC3 provoke cell death by a different mechanism.

We also tested some immunogenic cell death markers that would give us further information about the mechanism of action of these toxins, and also clues about the immune response that the injection of these toxins may elicit. For that we tested the calreticulin exposure on the tumor cell surface, which is a typically described eat-me signal. Addition of mitoxantrone to the culture was used as a positive control, provoking that almost all the cells in the culture exposed calreticulin (Figure 19C). As negative control SIEC X SAegfr *ypjA*::Ptet-Tir100-NbGFP-bla was used, showing slightly higher levels than infected cells. We observed a significant increase in calreticulin exposure in cell cultures infected with SIEC X SAegfr *ypjA*::Ptet-Tir100-PE25 and SIEC X SAegfr *ypjA*:: Ptet-Tir100-TccC3 in dose-dependent manner. The calreticulin exposure was approximately 20% higher in cells infected with SIEC X SAegfr *ypjA*:: Ptet-Tir100-TccC3 compared to cells infected with SIEC X SAegfr *ypjA*::Ptet-Tir100-PE25 (Figure 19C).

We also assessed the ATP release to the extracellular media, as another sign of immunogenic cell death (Figure 19D). In this case, we detected liberation of this molecule in cell cultures infected with SIEC X SAegfr *ypjA*::Ptet-Tir100-PE25 upon injection of ExoA, but no in TccC3 case (Figure 19D).

### Example 15-Translocation of protein cargos through T3SS injectisome for in vitro killing of tumor cell lines: Combination of ADP-ribosylating cytotoxins for effective tumor cell-killing at low bacterial doses

After determining the tumor cell killing capacity of the SIEC X SAegfr derived strains injecting the toxin fusions Tir100-PE25 and Tir100-TccC3 , we wanted to asses if the injection of both toxins combined could lead to a more powerful cytotoxic effect. To do that, we co-infected HCT116 cells with both toxin-injecting SIEC strains, maintaining the total bacterial MOI. We also included in the experiment cultures individually infected with each of the strains that inject toxins, and the previously used control injecting NbGFP. Remarkably, we observed that the co-infection with both strains SIEC X SAegfr *ypjA*:: Ptet-Tir100-PE25 and SIEC X SAegfr *ypjA*::Ptet-Tir100-TccC3 (COM BO condition) led to a significant stronger cytotoxicity effect compared to the infections with individual strains, so that at 48 hpi only few cells remain alive in the cell culture infected with the combination of strains, even at MOI 1:1 (Figure 20).

### Example 16 - HCT116 tumor growth evolution in Nude mice

For testing the anti-tumor capacities of the generated toxin-injecting SIEC strains (SIEC X SAegfr *ypjA*::Ptet-Tir100-PE25 and SIEC X SAegfr *ypjA*::Ptet-Tir100-TccC3) we first developed an *in vivo* tumor model based on the subcutaneous implantation of HCT116 cells on nude mice. We develop a model in which the mean tumor size of the subcutaneous tumors of the mice at 14 days post implantation was around 100 mm³, with all the mice bearing tumors between 75-150 mm³, conditions we estimated optimal for starting the bacterial treatment using SIEC derived strains.

We constructed isogenic strains replacing SAegfr adhesin by the control adhesin SAtir, generating SIEC-X SAtir ypjA::Ptet-Tir100-PE25 and SIEC-X SAtir ypjA::Ptet-Tir100-TccC3 strains . Both strains were used in a combination named COMBO SAtir at doses of 10⁸ bacteria/mouse. The strain SIEC-X SAegfr ypjA::Ptet-Tir100-NbGFP-bla was included in a control group of mice at a dose of 10⁹ bacteria/mouse . We observed a reduction in tumor growth in the COMBO SAegfr group at 16 days when 10⁸ bacteria/mouse were administered. The group treated with the COMBO SAtir administered at 10⁸ bacteria/mouse concentration did not trigger any reduction in tumor growth (Figure 21).

## Claims

1. A recombinant gram-negative bacterial strain comprising:
(i) a type III protein secretion system (T3SS);
(ii) at least one cargo component, wherein the cargo component comprises a secretion signal (SS) region recognized by the T3SS system and a cargo protein;
(iii) an inducible genetic regulatory circuit to regulate the transcription of (i) and (ii); and
(iv) at least one synthetic adhesin (SA) driving adhesion of the recombinant bacterial strain to a target cell,
wherein the strain is capable of adhering specifically the target cell and subsequently injecting the cargo protein into said cell.

2. The bacterial strain of claim 1, wherein first nucleotide sequence comprises a nucleotide sequence encoding a polypeptide selected from the group consisting of: Tir30; Tir100; EspF20; or variants thereof.

3. The bacterial strain of claim 2, wherein the T3SS further comprises a third nucleotide sequence encoding a Tir chaperone (CesT) or a variant or fragment thereof and the secretion signal region further comprises a nucleotide sequence encoding the binding site for CesT.

4. The bacterial strain of any one of claims 1 to 3, wherein the inducible genetic regulatory circuit comprises nucleotide sequences encoding: the lactose operon repressor (Lacl) comprising a W220F mutation (Lacl W220F) and a protein degradation tag fused to the C-terminus; the bacteriophage A major lytic promoter (PR); the repressor protein cl comprising a E118K mutation (cl ind-); and the tetracycline-controlled promoter PtetA, and wherein the expression of the T3SS and the genetic circuit is induced in the presence of aTc.

5. The bacterial strain of any one of claims 1 to 4, wherein the cargo protein is selected from the group consisting of:
a) Antibody fragment;
b) Cytotoxins
c) Effector proteins of T3SS
d) Proteins inducing cell death;
e) Prodrug converting enzymes;
f) Immunogenic antigens; and
g) Genetic reprogramming factors.

6. The bacterial strain of any one of claims 1 to 5, wherein the antibody fragment is a nanobody; the cytotoxin is selected from a group consisting of (a) ADP-ribosyltransferase (ART) toxin ExoA or a fragment or variant thereof and (b) ADP-ribosyltransferase (ART) toxin TccC3 or a fragment or variant thereof; the effector protein of the T3SS is selected from a group consisting of (c) EspH; (d) Tir; (e) NleC; (f) Map and (g) a fragment or variant of (c), (d), (e) or(f); the protein inducing cell death is selected from the group consisting of (g) BID;(h) BIM; (i) Granzyme B; and (j) a fragment or variant of (g), (h) or (i); the prodrug converting enzyme is Herpes Simplex Virus thymidine kinase or a fragment or variant thereof; the immunogenic antigen is selected from the group consisting of (k) Survivin; (I) ovolabumin (OVA); and (m) a fragment or variant of (k) or (I); and the genetic reprogramming factor is selected from the group consisting of Sox2 and a fragment or variant thereof.

7. The bacterial strain according to any one of claims 1 to 6, wherein the bacterial strain is a non-pathogenic *Escherichia coli* strain.

8. The bacterial strain according to any one of the preceding claims, wherein the bacterial strain is comprised within a pharmaceutical composition in a therapeutically effective amount.

9. The bacterial strain according to any one of claims 1 to 8, wherein the nucleotide sequences encoding components (i), (ii) and (iii) are integrated into the chromosome of the bacterial strain in monocopy.

10. The bacterial strain of any one of claims 1 to 9 for use as a medicament.

11. The bacterial strain according to any one of claims 1 to 9 for use in the treatment of a cancer disease in a subject.

12. The bacterial strain for use according to claim 11 wherein the strain comprises at least one SA that binds EGFR and the cancer disease is an epithelial tumor.

13. The bacterial strain for use according to any one of claims 11 to 12, wherein the treatment comprises the use of at least two of the bacterial strains, and wherein each of the bacterial strains expresses a different cargo protein.

14. The bacterial strain for use according to claim 13, wherein the treatment comprises the use of (a) a bacterial strain expressing ExoA or a fragment thereof and (b) a bacterial strain expressing TccC3 or a fragment thereof.

15. The bacterial strain for use according to any one of claims 11 to 14, wherein the subject is a human subject.


SEQUENCE LISTING

<110> Consejo Superior de Investigaciones Científicas(CSIC)

<120> Recombinant bacteria and uses thereof

<130> EP1641.1637

<160> 71

<170> BiSSAP 1.3.6

<210> 1
<211> 5472
<212> DNA
<213> Artificial Sequence

<220>
<223> Sequence of eLEE operon 1

<400> 1
cctgtagtac gggaagctgc ttagaacact atatataggg ggtatgtttg ggattcactg 60

caagatagtg tgaaaatgac cctcttgcaa gtgcataact ttgtggataa ctcaggaagg 120

aaaaagtggc tttcgcgcac cttaggtcag acaaggtgtc cgggaaagtc aatgggaaga 180

gaaaaatttg ttaaaaataa ctgttcggcg atttcttgaa cggcataata ggttagataa 240

attgatatag atggcttatt aatctttttg ctaatgattg caaaaattga tctaagcacg 300

agtcatcaca taatctgatg tatcagttgt ggtcaatata taagcgaatg taagagattg 360

ttgatggcga aatggttgtt ttctattcgg ttattgttaa atgctaagcc attttgttaa 420

tttaccttat attataagaa aattattcat gactttaatt tagtttaaat ataactgata 480

tccctgaagg ttatgagctc cagggctaca ctagtcgttg aggttctaga gtcgacaaga 540

aggagatata catatgcgga gattatttat tatgaatatg gaaactaatt cacatacaac 600

aagtccatac attcagctta tagagcaaat tgcagttcta cagcaggaag caaagcgact 660

gcgagagcag gaagttcaaa gtgtaattga gtcgattcag aagcagatta cttattacaa 720

tataacctta caagagctgg gatatactaa tgtgcctgat gatggactcg ctcgccggaa 780

ctcatcgaaa ggtgtttact accgcaatga agaagggcag acctggtcgg gcgtaggccg 840

acagccacgc tggcttaaag aagcactgtt gaatggaatg aagaaagaag attttcttgt 900

gaaggacact gaagaagaaa taataccgct gaaaaatatt taacataaaa taattaaatg 960

ataacgataa ctgagctgga agacgaaata ataaaaaata aagaagccgc aaatgttttt 1020

attgaaaaaa taaacgacaa aaagaacgaa atccatgaaa aaatgaaaca ccctttggat 1080

aaagttacct acaatgaagc aaaggaactg ctcattgcgt gtgatgcggc aattaggact 1140

atagagataa tgcgaatcag gattaataat aaatagagga tgagtattgt gagccaaaca 1200

agaaataaag aactattaga taaaaaaata agatcagaaa ttgaggcgat aaaaaaaata 1260

atcgccgaat ttgatgtcgt aaaagaaagc gttaatgagt taagtgaaaa agcaaaaaca 1320

gatcctcagg cggcagaaaa attaaacaaa ttaattgagg ggtacacata tggtgaagag 1380

cgaaaattat atgattctgc cctaagtaag attgaaaaac taatagagac actgagtcca 1440

gcaagatcta aaagccaatc aacaatgaat caacggaata gaaataatag aaaaatagta 1500

tgacaatttt taataaaata gactataact actataaatt aattaattat ccaatcatga 1560

tggttcatga tgaatggcta ggtgatttaa ccggtgtcaa tcaggtcagt tttcggcgtt 1620

taagagaaac tagttctact cgtaatcaac tcaataagat attgcgtcag gagatacaag 1680

ataaaatttc tggtgtggaa ttatctgaca taaataagga aggtttttta tatcagtcaa 1740

ttgggaaaat aagattgctt gctctaagta gtgcattatt tgacattcaa tgccctgatt 1800

atattttctc acgattatat cgagaaactc tgattagaga gataggatat caaaatgtga 1860

agcagttatc attctattgg cagggagggc aatgtaaacc agaatatggt gaagaacggt 1920

tttgtgctga gttaataaaa tacggtgcag gaaatttgga gtggctattt gctgataatc 1980

ctttatggac catagtcaaa tatcttttac cgaaaagtgg agaaataaaa ccaactcata 2040

ttaatgattt atttcttaac agacttaata aaattttatt gccttatgaa actttatgaa 2100

tttaaaaata tagaaattga tttagtgctt acagaagata tcataccaga ggataagtta 2160

caggaaatta ttcaatctga tgatattatc aaactggctc gaaaaaaaac atatgaacat 2220

ttactgaggg cgaggaggaa atcgaaagaa ttaaagatag aatccagaaa aaaaattgcc 2280

agaaaaatga ttgcaatgag agaaaggatt cgaaaaaata ataaaataaa attagataaa 2340

gaggttaacc aatcgataaa atgggttaaa gatatccagg ctatcgagtt ggtcttaatg 2400

caggacataa tgaataaggt tcatttgtca ctgactaatg cattacattc tttagataca 2460

tcatcaagaa ttaattggga tgatttactt aatgaagttg taagggaaac tctatctaat 2520

aacaatattg tcggagctat taagatcact aaaaatccag acattaaact tgatcctggc 2580

gaggccaaca atatacagct tattaatgat gcaaacacac cgcataataa aattataata 2640

gaaaatgaat atattagaat aactttagac ccattagagc aaataagtat tttattgaat 2700

tcatttaaag ataattatct tagcattatt caggaatgat gatatgtctc aattaatgac 2760

cattggctca cagccaatat ttttaattat tgtatttttt ctgttatcat tactgccaat 2820

atttgttgtt attggtactt cattcctgaa aatttctatt gtactgggga tattgaagaa 2880

cgcattaggc attcaacagg taccaccaaa catggcgcta acatcagtgt ctttgatact 2940

gacaatgttt attatgtctc cgataatatt acagataaat gataatattt ctcaggaacc 3000

aatcaattat accgactctg atttttttca aaaagttgat gagaaaatat tatcaccata 3060

tcgcggtttt ttagaaaaaa atacagagaa agacaatgta gagttttttg aacgtgcagc 3120

tcaaaaaaaa ttgggtaatg aaactatctt aaaaaaagac tctctattta tactgttacc 3180

ggctttcacg atggggcagc ttgaagctgc attcaagata ggttttttgc tctatttacc 3240

ctttattgcg atagatttga tcatttccaa tatcttattg gccttgggta tgatgatggt 3300

atcgccagta acaatttcga ttccttttaa gatcttgtta ttcattcttg ttggaggctg 3360

gcaaaaatta ttcgaatttc tgttggtggt gaattgatgg atactggata ttttgttcaa 3420

ttatgtgtgc aaacgttctg gataatattt atcctctcat tgcctacagt catagcggcc 3480

tctgttatcg gtattattat tagtctggtc caggctataa cgcagttaca ggatcaaaca 3540

ttgccttttt tgctaaaaat aatagcagtg tttgctacgc ttgccctgac ttatcactgg 3600

atgggaacaa caatcatcaa cttctcgtcg ataatatttg aaatgattcc gaaggtgaac 3660

ggctaatgaa tgagataatg acggtcatag tatcatcatt ttattgtata ctcaggccgc 3720

tggggatgtt tattatccta ccaatatttt caacaggtgt tttgctcagt aactttattc 3780

gcaactcaat tatgattgcg tttacgcttc cgatcatagt agagaactat acattttcgg 3840

aaaaattgcc atctggaatc tttcagttga caggaatagt gctaaaagaa ataagtattg 3900

gttttttcat tgggttatca tttactattc ttttttgggc aatagatgcg gctgggcaga 3960

ttattgatac tctaagaggt tcaacaatat cttcaatttt taacccgtcc ataagtgatt 4020

catcttctat cactggcgtt attttgtacc aatttatctc tgtgatcttt gttattcatg 4080

gtgggataca aagcattctg gataagctat atttatccta cgagatatta ccattacaag 4140

ccgatattgc attcaatcgt gctttaatag attttttgtt ttctctatgg gattcattta 4200

ttaaactgat gttatcattt tcagttccca tgattatcgg tatattctta tgtgatatgg 4260

ggtttgggtt tcttaacaaa acagcgcctc aactaaacgt attcacatta tcactgccag 4320

taaaaagttt gatagcaatc tttatattgc tattagttat tcatgttttt cctgatttta 4380

ttacggcaaa tattcattct gacataattg atagatcgtt accgagcatg attaatgagt 4440

gaaaaaacag aaaagcccac acccaaaaaa ctgagggatc taaaaaagaa gggcgatgta 4500

acaaaaagtg aagaggtaat ggctgcagtg cagtcattaa tcttattttc atttttttct 4560

ttatatggca tgagtttttt tgttgatata gttgggttag ttaatacgac aatagactcg 4620

ctaaatagac cgtttttgta tgccattcga gaaatattag gtgcggtgtt aaatatattt 4680

ttattatata ttttgccaat ttctttgatt gtctttgttg gaactgttac gactggtgta 4740

tcacaaatag gattcatctt tgcggttgaa aaaataaaac catcggctca gaagattagt 4800

gtaaaaaata acctgaaaaa tattttttct gtaaagagca tttttgagct acttaaatca 4860

gtatttaagt tagtgataat tgttctcatt ttttatttta tggggcattc atatgcaaat 4920

gagtttgcta atttcacagg actgaacgca tatcaagctc ttgtcgttgt tgcctttttt 4980

gtttttcttt tatggaaagg cgtgctattc ggatatctac tcttttcagt atttgatttc 5040

tggttccaga agcatgaggg actgaagaaa atgaaaatga gtaaagatga ggtgaaacga 5100

gaagccaagg atactgatgg taaccctgaa attaaagggg agcgccgtcg ccttcattcc 5160

gagatacaaa gtggaagttt ggcgaataac atcaaaaaat caaccgttat tgttaaaaac 5220

ccgactcaca ttgcgatttg cctatactat aaacttgggg agactccatt acctttagtt 5280

attgaaacag gaaaagatgc caaagctcta cagatcatta aactggctga actctatgat 5340

attccagtga ttgaagatat tcctttagca agaagtctct ataagaatat acataaagga 5400

caatatataa cagaagactt ttttgaacct gtggcacaat tgattcgtat tgcgatagac 5460

cttgattatt aa 5472

<210> 2
<211> 4623
<212> DNA
<213> Artificial Sequence

<220>
<223> Sequence of eLEE operon 2

<400> 2
ctcgagggcg atcgcgccag cggagttccc tttattccgt tgcgtgaggt tgctggctgg 60

gaacacgatc ttcatgctgc aatgaacaac attcaggaag aaatcgaatt ggttggcgag 120

aatgcggcga gcattgatgc ttatgcagcc agcgatcctg ctgaatgttt tgccgtactt 180

tctgaatatt tctttagcgc cccagaactt tttgctcctc gtttcccttc attgtggcaa 240

cgtttctgcc aattttatca acaagatcct ttgcagagac tgcatcacgc taatgataca 300

gactcgtttt cggcgacgaa tgttcattaa ttaacaactt tgcagattaa ttaaccaatt 360

gaaatgactt atgaaattta gtgttgacag acaaggtacc gctaagtaat attcgccccg 420

ttcacacgat tcctctgtag ttcagtcggt agaacggcgg actgttaatc cgtatgtcac 480

tggttcgagt ccagtcagag gagccaaatt ctaaaaattc gctgagctct tctgaaatga 540

gctgttgaca attaatcatg ggctcgtata atgtgtggaa ttgtgagcgg ataacaattc 600

tagagtcgac ctgcaggttt gcaagcttaa gaaggagata tacatatgga tgcattatgc 660

tattgcctct ctcatgaaaa gagactaaca gtaaatatga ataatattaa tcaatcggaa 720

aacattaata ttcagttaaa taaagcatcc caaacaaatg tcgttgatga gcatatcccg 780

ttagcatcaa ccccctctgc agccggagcg gctcagtttc ttgatcaatt actccctaaa 840

acggcgggag tgtcttcccc agaacaagtg ttgattgagg aaattaaaaa gcgacatctt 900

gcaacaatga acaccgatct cagtttcgat gcactatctg caggtgggct ctcgcccgaa 960

gatgtactca ctttacaaaa gaatgtgctc aacgcaaacg tcaatgttga tgtcgtatct 1020

aagttagcaa gcctcctttc aacatcggtt acaaaattag tttcgatgca ataaaaatga 1080

aaaaacacat taaaaacctt tttttattgg ctgctatttg tttgacagtg gcatgtaaag 1140

agcaattgta tacaggatta acagaaaaag aagctaatca gatgcaagca ctgttgctat 1200

ccaatgatgt caatgtttcc aaagaaatgg acaaaagtgg aaatatgaca ctcagtgtgg 1260

aaaaagagga ttttgttcgc gcaataacga ttttaaataa taacggcttc ccgaaaaaga 1320

aatttgcaga tatcgaggtt attttcccac ccagccagtt agtggcatcg ccatcacaag 1380

aaaatgcaaa aatcaactat ctcaaagaac aagacatcga aaggttgcta agtaaaatac 1440

ccggtgttat cgattgcagc gtttcattaa atgttaacaa taacgaaagt caaccatcct 1500

cagcagccgt tttagtcatc agctcgccag aggttaactt agcaccatcg gtcattcaga 1560

taaaaaacct ggttaagaat agtgtcgacg atctcaaatt agaaaatatt agtgttgtta 1620

tcaagtcatc ctcaggacag gacgggtaat atgtatgaac aataataatg gcatagcaaa 1680

gaatgattgt gactggctaa cggcccttga ttttgtaaag gatgtcaacg gttcaccaac 1740

gcatctcact ttttatatct accaaaaaaa tgctttcttg cacgatttgg gaaactactg 1800

ggttctatat atcgagctta gtggtgattt tcgtcagatt ccaacagata catttatcag 1860

actctgcaat atcctggccg ttagtaaaga atataaacaa atgggtattt tcctgtctaa 1920

taagaagtgg tatctttgcc aaatttttca taaagataat aatcatcgcg caaacatgtc 1980

aaaagcaata atgcaacata ctctcgcctc gttgctcgac aagcaatttg ataaactaga 2040

gcaatcatct tcatcagata caatgatgcc gccaacacac ttgttttctg atataggacg 2100

aattgtgtaa tgaaaaaaat aagttttttt atttttacag cactattttg ctgcagtgca 2160

caagctgccc cgtcctctct ggaaaaaaga ttaggcaaga acgaatattt tattattact 2220

aaaagttctc cagtacgtgc aatccttaat gactttgctg ctaactacag cattcctgtt 2280

ttcatcagta gttccgtgaa tgatgatttc tctggcgaaa taaaaaatga aaaacctgta 2340

aaagtactgg aaaaactgtc caaactctat catctcacat ggtattatga tgagaacatc 2400

ttgtatattt ataaaacaaa tgaaatatca agaagtataa ttacaccaac atatctggat 2460

attgactcac tgctaaaata tctgagtgat acaatctcag taaacaaaaa ctcttgcaat 2520

gtgagaaaaa ttacgacatt taactcgatt gaagttcggg gtgttcctga atgtataaaa 2580

tatattacat ctttatcaga aagtcttgat aaagaggcac agtctaaagc caaaaataaa 2640

gatgttgtta aggtctttaa actgaactat gcgtcagcga cagatataac atacaaatac 2700

cgtgaccaaa atgttgtcgt cccaggtgtt gtctccattc tgaagacaat ggcaagtaat 2760

ggttcgcttc ccagtaccgg taaaggtgcc gtcgagcgaa gcggtaacct gtttgataac 2820

agtgtaacga tttccgctga tccacgtctt aatgcagttg ttgtaaaaga ccgcgaaata 2880

acgatggata tctatcagca attaatttct gagcttgata ttgaacaaag gcaaattgaa 2940

atttctgtct caattattga tgttgatgca aacgatcttc agcagctcgg cgtaaactgg 3000

tccggtacgt taaatgcagg acaaggtacg attgccttta atagctctac cgcacaggct 3060

aatatcagct cttctgtgat aagtaatgcc agtaatttta tgattagggt gaatgcgtta 3120

caacagaact cgaaagctaa aatactctct cagccgtcaa ttataacctt aaataatatg 3180

caggcgattc ttgataaaaa cgtaacgttt tatacaaaag tctccggaga aaaggtggcg 3240

tctctggaaa gtattacaag cggaacattg ctgagagtga caccacgtat acttgatgac 3300

agctcgaata gtcttaccgg aaaacgtcgg gaacgagttc gtttgttact tgatattcag 3360

gatggcaatc aatcgactaa ccaaagcaat gctcaggatg ccagcagcac cttacctgaa 3420

gtacaaaatt cagaaatgac aacagaagcg acattaagtg caggagaaag tttattactt 3480

ggcggattta tccaagataa agaaagttca agtaaagatg gtataccgct gttaagcgac 3540

attcctgtta ttggtagttt attctccagt acggtaaaac aaaaacatag tgttgttcga 3600

ctctttttaa ttaaagcaac cccgataaaa tctgcatcta gcgaataata aatgaggctt 3660

tatgagcagg aaatttagct ctctagagga tatttatgat ttctaccagg atggtggcac 3720

attagcgtca ttaacaaatc tgacacaaca agatctcaat gaccttcatt cttatgccta 3780

tacagcatat caatctggtg atgtaataac cgcaagaaat ctattccatt tgctcacata 3840

tctggaacac tggaattatg actacacctt atctctgggc ttatgccatc agcgtttatc 3900

aaatcatgaa gatgcacaac tgtgtttcgc acgctgtgca actttagtta tgcaagatcc 3960

cagggcatct tattattctg gaattagcta cttactcgtc ggaaataaga aaatggccaa 4020

gaaagccttt aaggcttgtt taatgtggtg taatgaaaaa gaaaaataca ctacatataa 4080

agaaaatatt aaaaaattgt taggtaatac agagtaaact agtttttccg ttataatttc 4140

ttaaagagtg ttccattcgg ggcactcttt tttacattcc ctctacagaa ctagcccttc 4200

tctcacagaa aatgaataaa taaaaatgcg gcaccgccag aatcgcgttc gatgtgacta 4260

tgatcatttt cggtgagtac tgctttgatc taaaggctgg ttttctgtga tgattgacca 4320

gcctcagagt attgatgaca ccaccgttca taacagatcg gcattactat gcataagtac 4380

tgtctcatat tctcgccaaa acacttttta atgcctttgc gaattcatcc agacttatat 4440

tgtcaatttt ttgcccaacg acgactgact ccgagacatc ggccattgcg ggccatcgcg 4500

ttttgtaatc agcataaatt gcaacaacag gggtattata ggcacttgcc atatgtaata 4560

ttgaggtgtc aggagttatt gcgatataag catcttttat gattgctgct gaacgtttaa 4620

tcg 4623

<210> 3
<211> 6485
<212> DNA
<213> Artificial Sequence

<220>
<223> Sequence of eLEE operon 3

<400> 3
cttgggctga acgaaccggg agaaagcctg caaccggcct gccatatcag tcaacttgat 60

gccagaacac agcaacatga gatgttaaaa accgcgggtc gccccgtgac tcgtgggatc 120

accttaggcc tgaaggatat tctcaatgcc cgcgaagttt tgttactggt gactggcgaa 180

ggaaagcagg atgcgacaga tcgttttctc acggctaaag tctctaccgc tatcccggct 240

tcatttttat ggctgcacag taactttatt tgtttaatta acacctaacc agatgaaaaa 300

tttgctcaaa atgtttaaat taacttatgt aacagtcacg cattatatta aataacattt 360

gactgggttg aacataacgc cgatagcaaa aggagttatg ttcaacaatt taattttaga 420

tatacatata acttgttcta ttaataaatc acctcgtatt ctgactcgcc ccctggcgca 480

gcaaagtcag gcatttatac tctgagctct tctgaaatga gctgttgaca attaatcatg 540

ggctcgtata atgtgtggaa ttgtgagcgg ataacaattc tagagtcgac aagaaggaga 600

tatacatatg aatcttttag ttaaaagaaa tgttgaagag tttttaagat tgttgggaaa 660

tgatttttat ttatttgata accgtgttga aattgatttt aatgggtttt ctttttttat 720

tgaaataatt gataataatg tttttgttac gtttgcttta gagtataacg aaaacgcatt 780

tttctctttc tttagtgccc ttgctcctga gcgtacgcag ggagtgattg aacatatttt 840

tgtctacgat aataaattgt gcttaagttg tttgttaacc aatatcgatg ttttttttct 900

aatgaatact tttcaacagc atgtgcagat tattgagcgc gttcgcagga tgacatcatg 960

aataaactct taaatatatt taaaaaagca gagtcatatc acgatcttat tctggctctc 1020

ttcttcttta tggctgtaat gatgatgatt attccactac caacagttgt ggtggatatc 1080

attatcgcga ttaatatttc gacagcttta cttttattaa tgctctcaat ctatataaaa 1140

aatcctcttg aactaacttc tttccccaca atcctgttga ttacgacgtt gatgcgcctg 1200

tcgctcagtg ttagtacaac cagacttatt ctgctgcatc acgatgcggg tgacattatt 1260

tactccttcg gtaactttgt tgttggcggt aatattgttg ttggcctggt tatttttact 1320

attatcacca ttgttcaatt tatggttatt acaaaagggg ccgagcgtgt tgctgaagtg 1380

agtgctcgtt tttcccttga tggtatgcca ggaaagcaaa tgagtattga tggtgatatg 1440

cgcgcaggcg ttattgaccc gcttgaggca aaagttcttc gctcccgagt gcaaaaggaa 1500

agccagtttt atggttcaat ggacggggcg atgaagtttg taaaagggga cgctattgct 1560

ggtatcatta ttgttttagt taaccttttt ggtggcgtgc tcattggtat gtggcaattt 1620

gacatgccat ttagtgaggc acttagcctg ttttctgtat tgtctgtcgg tgatgctttg 1680

gttgcccaga tccctgcgct tattatttct gtcaccgcgg gcgtggttgt tactcgtgtg 1740

cccggtgaaa gcgaaaaaga agaaaacctt gcaggtgata ttgttcaaca ggtttctgtt 1800

aatagtcgcc cttttttaat cagtgccgca ctaatgctcg taatggcgat tattccgggc 1860

tttcctacat tggtcttctt atttctggcg gtttgcctgt tggggatagc ctggaaatta 1920

cagaagaaaa gaacatttgg agctggcaat aataaggatg ctatgggagc tgatttgtct 1980

aatagccaaa acatctcacc gggcgctgag ccattgattt taaacttaag tagtaatatt 2040

tatagttcag acattacaca gcaaattgag gtcatgcgtt ggaatttctt tgaggaaagt 2100

ggaattccat tgcctaagat tattgttaat ccggttaaaa ataatgatag cgcaatagaa 2160

tttttgctct atcaagagtc aatatacaaa gatactctta tagatgatac tgtctatttt 2220

gaggctgggc atgcagagat atcattcgaa tttgtccagg aaaagctttc gactaactct 2280

atcgtatata agacgaataa gactaatcaa caactcgctc accttacagg tatggatgtt 2340

tatgcaacaa caaatgataa gataacgttt ttgctaaaga aacttgtatt gtctaatgcc 2400

aaagagttca tcggcgtaca agaaacgcgt tatttgatgg acatcatgga gagaaaatat 2460

aacgagcttg tgaaagagct gcagcgccag cttggtttga gcaaaattgt tgacattcta 2520

caacgtctcg tagaggaaaa tgtctcaatt agagatctga gaactatctt tgagacgctt 2580

attttttggt caacaaaaga aaaggatgtg gttatcttgt gcgaatacgt tcgtatcgcc 2640

ctgcgtcggc atattttagg tcgctatagc gttagcggta cacttctgaa cgtttggctt 2700

attggctctg atattgaaaa tgagctacga gagtcgatca gacaaacgtc atcaggttcg 2760

tatctgaata tctcaccgga gcgaactgag cagataattg gcttcttaaa aaatatcatg 2820

aatccaacgg ggaacggcgt cattctgacc gctttagata tcaggcgcta tgtgaagaaa 2880

atgattgaag gttcgttccc gtcagtcccc gtgctctctt ttcaggaggt tgggaataat 2940

atcgaactta aagtattagg aacggtaaat gatttcagag catgattctg tattggaaaa 3000

atacccacgt attcagaaag tgctcaatag cactgtgccg gcattatcat taaattcgtc 3060

taccagatat gaaggcaaga ttatcaatat cggcgggacg attattaagg cgcgtttacc 3120

aaaagcgcgt attggcgcct tttacaagat agaacccagt caacgtttag ccgaggtgat 3180

cgctattgat gaagatgagg ttttcttgtt gccttttgag catgtttccg gcatgtactg 3240

tggtcagtgg ttaagttatc agggggatga gttcaagatt cgtgttggag acgcactact 3300

tgggcggctt attgatggca taggcagacc aatggagagt aatattgttg ccccctatct 3360

tccgtttgaa cgcagcttgt atgctgaacc accagatcct ttattaaggc aggttattga 3420

tcagccattt atacttggcg tcagggcaat tgatgggcta ctaacttgcg gtattgggca 3480

gcgtatcggt atttttgctg gttcaggcgt tggtaaaagt acgcttttgg ggatgatttg 3540

taatggcgca tccgcagata ttattgtcct tgctcttatc ggcgaacgtg gtcgcgaagt 3600

aaatgaattc ctcgcgcttt tacctcaatc cacgctttct aaatgtgtgc tggtcgtcac 3660

aacgtcagac cgccccgcgc tggaaaggat gaaagccgca tttacggcga cgactattgc 3720

agagtacttc cgcgatcaag gtaaaaatgt attattaatg atggattctg taactcgtta 3780

tgcccgagcc gcgcgtgatg ttgggcttgc atcgggagag cctgatgtaa gggggggatt 3840

ccctccgagt gttttttctt cgttacccaa attattagag cgagcggggc ctgcgccaaa 3900

aggttcaata accgcgattt atacggtgtt gttagaaagc gataatgtta atgatcctat 3960

tggcgatgaa gtccgctcta ttcttgatgg gcacatcgta cttacccgag aacttgcaga 4020

ggaaaaccat ttccctgcaa ttgatattgg tttaagtgcc agtcgtgtta tgcataacgt 4080

tgttacatcg gagcatttgc gtgcggcggc agaatgcaaa aagcttattg caacttataa 4140

aaatgttgag ctgcttattc gtattggtga gtacacgatg gggcaagatc ctgaagcaga 4200

taaggcaata aaaaatagga aattaattca gaactttata caacaaagca ccaaagatat 4260

cagtagttac gaaaaaacga ttgaaagcct attcaaagtg gttgcctgag tatgttggac 4320

agaattttat ctattcgtaa aagcagagcg aaccgattga gagaatcaat ggctaagatt 4380

aactctcaga taaaagaggt ggatggtaaa ctcgatgact gcgagcaatc cattaaagag 4440

tcgattgcct ctaagcaggc gtattgtgca tcgctggtga atttggataa agtgtcgttg 4500

tataaatatc agattaaaaa caatgcattt gatgagcaaa agcagcgatt gtatgagaaa 4560

aaatcttcac tttcgaagga aaagcgttca ttgctagatt cacaaaaacg aacaaaagag 4620

aatctgcaac atgttaataa atcagtagaa aaattatctt ttgccataaa ggaacattac 4680

tttgactaga gtttctctaa aaagaaattt gatagagcct gtttcttttc ggccatcaga 4740

tgattggccg caaggagaag aaagttttaa tgatattttt tatcatataa aaaaaagatc 4800

gcctgatacg ttgttcatac gaatggcttc tttatggaag agattgtttt actcctcggg 4860

ccgaagacga cgctactttg aagaagggga gcactcgttt agtattcttt gtggaagatt 4920

acgaggagtt gttttaacaa ttaaatgtag taacggaatc atttatctgt ctattaaaat 4980

aagccaaaat aatagaaatc atgttttttt atatcataaa aaagactatg ttttcgataa 5040

gttaaaagaa atctttcctg atgaagccat tgagttcaca attgaatatg aaaattaacg 5100

atttttattt gccgttgttg ccagttatcg gaaccggtcg cctctatatt acgtccgaag 5160

gtcatgcttg ccatgcatat tttcgtgagg tcagcggtca tggtattcgt ttcacgttga 5220

catactcggg ctatgagggg cgtttctgga tatcagaaga acagttcatt cagtggtgcc 5280

aagagttatt cccgtatagt gaaagccggc tgataccgga agatatgata aaactcatga 5340

tcttatgggt aatgcagact gccttaccgg aaggtgatgt gtcagttgat gatgttcaat 5400

ttacgatgct aaataaagat gtttatccgg taatagaaaa taataatgga gaaaacagac 5460

ttaatgtaat tatactcgaa acaacggtac aaagtttaca acatctgatt aatgataatt 5520

ggcaatttgt tccacattct aatgctcttt tttttgatgg ctatatcgta cctgggtgga 5580

ctgattatcc agttacagag ttgagagttg gtgatagtct gagactctat catgttgatg 5640

atagtcaaga gagaaggtgc tggattgtta ttaataaccc tttggcaacc gtaaagctga 5700

gcgataataa tttatctgtc acggatgttc tggctgcaga tccgttatgt actatcagca 5760

gcgagactgt gatgaatcgc atttattgtg tgattggcac agtgcatatt gatatacata 5820

tgttgcgtaa tgtccaaaaa gatgacatta tcgattcaac gggatatcat ctttttggtg 5880

ggtgccagtt agtacggaat aataccacaa tcgcatacgg gagtattata aaaatcaacg 5940

aagattttta ttttactatt agcgtagtat gtgattaaac tagtcatggc accgtcaata 6000

tgcgcgcctg gccagttaat ctggtcggtg gtgagctgga aacaggaaaa tttcagggca 6060

cagccaccat taccgtcatc gtgcggtaaa agaagacgtt gcgttttgat taacgcaacg 6120

cttcggcact gttacccctg ctgctccagc gcatacttat acagcgcatt tttcttcacg 6180

ccgtgaattt ctgcggccag cgccgccgct tttttcagcg gcagttctgc ctgtagcagc 6240

gccagcgtgc gcagggcatc ggcgggtaag tcttcttcct gtgctttatg accttcgaca 6300

atcagcacca tttcgccttt gcgacggttt tcatcttcct ttacccacgc cagcagctcg 6360

ccaacgggcg cgccgtgaat ggtttcccag gttttggtca gctcacgcgc cagaaccacg 6420

tagcgggatt cgcctaatac cgcaacgata tcttccaggc tatctaacag acggtgggta 6480

gattc 6485

<210> 4
<211> 5898
<212> DNA
<213> Artificial Sequence

<220>
<223> Sequence of eLEE operon 4

<400> 4
cgctgttagc gtcaatcgtt ttattgttac ctgccaacct gttgcctatt tctatcattt 60

atctgaacgg aggacggcag gaagatacaa ttctttccgg aattatgtcg ctggcaagta 120

gcaacattgc ggttgcagga atcgtgttta tcgccagtat tctggtaccg tttactaaag 180

tgatcgtcat gttcacttta ctgttgagca ttcattttaa atgccagcaa ggtttacgca 240

cacgcattct gttactgcgg atggtgacct ggattggtcg ctggtcgatg ctcgacctgt 300

ttgtcatatc tttaaccatg tcgctgatta atcgcgatca gatcctcgct tttactatgg 360

gaccggctgc gttttatttc ggcgcagcgg taattttgac tattcttgct gtggaatggc 420

tggacagccg cttactttgg gatgcacatg agtcaggaaa cgcccgcttc gacgactgaa 480

gcgcagatta aaaataaacg gagctcttct gaaatgagct gttgacaatt aatcatgggc 540

tcgtataatg tgtggaattg tgagcggata acaattctag agtcgacaag aaggagatat 600

acatatggct aatggtattg aatttaatca aaaccccgca tctgttttta attctaattc 660

attagatttt gaattagaat ctcagcaatt aacgcaaaaa aattcttcta atacttcttc 720

gccattaata aatttgcaaa atgaattagc aatgattact agctcatcgc tttctgaaac 780

gattgaaggg ctaagcctgg gatatcgcaa aggtagcgca aggaaagaag aggaaggcac 840

gacaatcgag aagctgctca atgaaatgca agagctgctt actcttaccg atagtgataa 900

aataaaagaa ctatcattaa aaaacagcgg gttgctggaa caacacgatc ctactttggc 960

gatgtttggc aacatgccaa agggggaaat tgttgcgcta atatcttctt tattacaatc 1020

taaattcgtt aagattgaat taaaaaagaa atacgccaaa ttattattgg atttattagg 1080

cgaagacgat tgggagctgg ctctgctttc ctggttaggg gtgggtgagt taaaccagga 1140

aggtatccag aagatcaaga agctttatga aaaggctaag gatgaggatt ctgaaaatgg 1200

cgcctcttta cttgactggt ttatggagat taaggatctt cctgagcgtg aaaagcactt 1260

aaaggttatt attagggcgc tatcgttcga tctgtcttat atgtcttctt ttgaagacaa 1320

ggtaagaaca tcttcaatta ttagtgattt atgtcggata atcatttttt tatcacttaa 1380

taactatacg gatattattg caatctccat taagaaagat aaagatgtca ttttaaatga 1440

aatgctatct attattgaac atgtatggtt aacagaagac tggctactgg agagcccttc 1500

tcgggtatcg attgtcgaag ataaacatgt ttattatttc catttattga aagaattttt 1560

tgcatcatta cctgatgctt gttttattga taacgagcag aggagtaata cattattaat 1620

gattggtaaa gtgatagatt ataaggagga tgttatgtga tattgttttt tttatagttt 1680

ttgtcatgct aagaaagatt atgaagaggt atatacatgg atacatcaac tacagcatca 1740

gttgctagtg cgaatgcgag tacttcgaca tcaatggcct atgatttagg gagcatgtcg 1800

aaagatgacg ttattgatct atttaataaa ctcggtgttt ttcaggctgc aattctcatg 1860

tttgcctata tgtatcaggc acaaagcgat ctgtcgattg caaagtttgc tgatatgaat 1920

gaggcatcta aggagtcaac cactgcccaa aaaatggcta atcttgtaga tgctaaaatt 1980

gctgacgttc agagtagctc tgacaagaat gcgaaagctc aacttcctga tgaagtgatt 2040

tcatatataa atgatcctcg caatgacatt acaataagtg gtattgacaa tataaatgct 2100

caattaggcg ctggtgattt gcaaacggtg aaagcagcta tttcagctaa agcgaataat 2160

ttgacaacga cggtcaataa tagccagctt gaaatacagc aaatgtcaaa tacgttaaac 2220

ctattaacga gtgcacgttc tgatatgcag tcactgcaat atagaactat ttcaggaata 2280

tcccttggta aataaccgga gataactatg cttaatgtaa ataacgatat ccagtctgtg 2340

aggtctggag ccagtgctgc tacggctact tcaggtatta atcagtctga agtgacttcc 2400

gcgctggatt tacaactggt taaatccacg gccccatcag caagttggac agaaagtacc 2460

gctttaccaa cgccaccggc aggtcactca ttagtgacgc cctctgctgc tgaggatgtc 2520

ctgagtaagc tgtttggtgg tattagtggt gaagttacga gtcgaacaga gggaacagaa 2580

ccacagcgca gcactcagaa tgcaagctct ggctatcctt atctttctca ggtgaataac 2640

gttgaccctc aggccatgat gatgatggcc actctgttat ccctggatgc ctcagcgcag 2700

agagttgcga gtatgaaaaa ctccaacgag atctacgcgg atggacaaaa taaagcgctg 2760

gataataaaa cgctggagtt taaaaagcag ctcgaagaac aacaaaaagc cgaagagaaa 2820

gcacaaaaaa gtaaaattgt tggtcaggtc tttggttggt taggggttgc agcaactgct 2880

attgcagcta tttttaaccc ggcactctgg gctgttgttg ctattagtgc aacagcaatg 2940

gcactgcaaa ctgcagttga tgttatgggc gatgatgctc cacaggcgtt aaaaacagca 3000

gcgcaagctt ttggcggtct atctcttgcc gcaggtattc tgacagccgg cattggcggg 3060

gtgtcttcac ttatatctaa agttggcgat gtcgctaaca aagttggttc aaacatcgta 3120

aaagtagtca ctacgctggc cgacactttt gttgataatg ttgcatcgaa aatttcggca 3180

gtggcaaatg gtttaactac ttcttcgcgc tctattggta caactgtgct gaataatgac 3240

gcagcgtatt ataatgtgtt atcacaggtt tctgcttttg ctgtggaaaa tttaactcga 3300

caaagtgagt acttaagtca gagtgcgaag gcagagctgg aaaaagctac tctcgagctg 3360

caaaaccagg cgaattatat acagagtgct tctcagctga tgtctgattc tgcgcgagtg 3420

aatatccgta ttgtcagcgg tcgagtttaa ttattaaaga ggcgtttatt atgaatacta 3480

tcgataataa caatgcggca atcgcagtta attctgtttt gagcagcacg actgacagca 3540

ccagttcaac aactacatca acttcgtcaa tcagttcatc tttacttact gatggcagag 3600

tggatatttc taaacttctg ctggaggttc aaaaactgtt gcgtgagatg gtcaccacat 3660

tgcaagatta tcttcagaag caattggcgc aaagctatga catccaaaaa gccgtttttg 3720

agagccaaaa taaagctatt gatgagaaaa aagccggtgc gacagccgct ctgattggtg 3780

gtgctatctc atctgtcctg ggatttttag gctcttttgc tgccattaat agcgcaacta 3840

aaggcgcgag tgatgtcgct cagcaagccg cttctacttc tgcgaagtct atcggtacag 3900

tctctgaagc ttcaactaaa gcactggcga aggcttccga aggtattgca gatgcagcag 3960

atgatgcagc tggcgcaatg cagcaaacta tcgcgacagc tgcaaaagcg gccagtcgta 4020

catccggtat cactgatgat gttgctactt cggctcagaa agcttctcag gtagctgaag 4080

aggctgctga tgctgctcaa gaattagcac agaaggcagg attattaagt cgctttactg 4140

ctgctgccgg aaggatttcc ggttcaacgc catttattgt tgttaccagc cttgctgaag 4200

gtacgaaaac attgccaaca acgatatctg aatctgtcaa gtccaatcat gatatcaatg 4260

agcagcgtgc taaatctgta gaaaatcttc aggcgtcgaa tttagatacc tacaaacagg 4320

atgttcgcag agcgcaggat gatatctcta gccgtctacg tgatatgaca acgacagccc 4380

gtgatcttac tgaccttatt aatcgcatgg ggcaagcggc tcgcttagct gggtaattga 4440

tcatggtcga tacgtttaat gatgaagtgt ttaattacta tcttgaacaa aaagggtaca 4500

caatacagaa ggagtttctt tgtggcagtg ccttttttat cggatggcgg attgagacac 4560

cttttttttc attagcgtac agactggatg aacaagaact gattttgtgc tcttttgaag 4620

cacgtaacca aacaggcttt aacggccctg ttttatcact gactcgtttg ctcgaagagt 4680

tgtatcacca cttttcgggt attaagaaaa tcagtgcgat gaaatctaag attggttcag 4740

attcagaacg tcaaaagcgc gaagagttgt ttaattactt catcagaaag ggtgccgtgc 4800

agcaagaaac agaagacgga atttggttcg taatgaacgt aaatagttaa aaaaaaatta 4860

ttttattaac ttctgaggga aatttaatga atttatctga aattactcaa caaatgggtg 4920

aagtaggtaa aacgctgagc gattctgtgc cagagttact taatagcacc gatttggtta 4980

atgaccctga aaaaatgtta gagttgcagt ttgcggttca gcaatattct gcttatgtta 5040

acgtagaaag tggaatgttg aaaacgataa aagatctggt ctcaaccatt tctaaccgta 5100

gtttttaatt tttatggtta atgatatttc tgctaataag atactggtgt gggcggctgt 5160

agctgcggca aaccataagc ttcccaaata tgcggaagct atcctgaatg tattcccaca 5220

aattatacct gataaaaaag atatcgcaca tttagaattt attatcttat ttggattaaa 5280

tagaaaaaat gatgcgataa aggctttgga gggctgtatg gatgatgagt cgagtcagtt 5340

attgtacagt ttgttccatg agaatgttag tggttgggta cgaggatttt aaactagtgg 5400

tacgccactg gcaccgaaag cccaggaagg caaacacttc ctgttgcagg aaagtgaacc 5460

gaaagagtgg cgtgaatggg gaactgcgct tcccaaataa tgcccactgc tccggcgtgc 5520

ctgcgccgga gcgtttatgc taaactgcgc gcctgttttt ttgccagtgg tacatgctcg 5580

tggcccaaca caccgtttat ttcccggacg cctttctgac acaaatgcgc gaagcgatgc 5640

cttcgacgct ctcatttgat gattttcttg ccgcctgtca gcgcccgttg cgccgcagca 5700

ttcgcgttaa tacgctgaaa atctccgttg ctgatttcct gcaattaacc gctccttatg 5760

gctggacgct tacgccaatt ccgtggtgtg aagaaggttt ctggattgaa cgcgacaatg 5820

aagatgcatt gccattgggt agtaccgccg agcatttaag tggcctgttt tatattcagg 5880

aagccagttc aatgttgc 5898

<210> 5
<211> 3434
<212> DNA
<213> Artificial Sequence

<220>

<223> >Sequence of eLEE operon 5 (EscD)

<400> 5
ggcagcggat atctgtcaac ctcactacta tcgaagactc tccccatcta taaataaaac 60

acctttaact ttcattcgga aagtattctt ctgcgcgtat cttgattttg cccaaagtta 120

catgttgaaa tgaataatgt ctgtttgaca ctcattatca tttattaata cagataatgg 180

agaaaataga gttgaataat caggcaggac gtaattgaaa cttgattaaa ttctggctat 240

agtgtttata tattatatcg acaaaagcgg gttaatgcca atcgcattat atgttcattg 300

tttgtttttt aagctattgg cattcattgt tgatatgctt cctgtttttt ctaaatatat 360

atttcgtgtt tttttagaag atccttaaga taatctttcc cctatcaata tattaattaa 420

atctacattt actcattagt aaaattaaat tgaatcaggg gagtgaaaac atcactattc 480

gaccccacac caacgaatac gagctcttct gaaatgagct gttgacaatt aatcatgggc 540

tcgtataatg tgtggaattg tgagcggata acaattctag agtcgacaag aaggagatat 600

acatatgtta tcctcatata aaataaaact tctcaatggt gcgatgcgta atcgggaatt 660

acaacttccg atgggaaacc taacgctagg tacggaagat aacgatattg tttactttcc 720

tcttgaacaa ggcctcaatc agtttttact tgatatacgt gaggaaggtg ttttcctttt 780

atctccggtt gaattttgga ttgatggcaa acccacgccc tatgaagcag ataaaccatt 840

acctgtcgga aagattattg atatagcggg atgttgcttc atcattggtg atattgacca 900

ttccttgcca ttgtctgatg tgccagaaag gttttctgcc aaaaatcaac gcaaaaaacg 960

ccttatcctg gcgagcgtga ttggtgctac ttttgcgttg tctggtgcca tcggcagtta 1020

cgttttgctt tccccaaaag ctgaaccccc aacgtttacc agagctgatg tatatcagca 1080

gctaaaagaa aacaaacttc atgcaatcac tctggtatgg catggaaaaa atatcgcgct 1140

ttatgggcgt tgcgaatcaa ccacggattt aactccattt tttaattacc tgaaagaaaa 1200

aaatattttt tattacaata aaatcatttg caataaccaa atcatatccg caattaatga 1260

tgtcttaacg gaatacggtt acaaagatat cattattaca aaaggtaata agcctgggtt 1320

ctttctgtta tcgggataca ttcccccttc acccaaatgg agcgaggttg aaaatctgtt 1380

actgaatacg ccaggggttg caggatggga aatacacaac aacagtaaca ataaaatcaa 1440

tgaactggca tcagagttta aaaagaataa actaataaac tatgtcaata ttttcaaaaa 1500

gaatgatgtc atcattgtcg cgggggaagt ttctcaacag aacgagagta agatccttgc 1560

cattataaat gcaatgaaca aaaatagtaa cgtcaagata ctctttcaga acattcagcc 1620

gtacatttca gcagatattt ttcccggaaa aatactcaga atcagcggta cgatgaagaa 1680

tcctacaatt gctcttgata atggtaccag cctcgggatt ggtagcattc tgaaaggagg 1740

ctatgtcatc gacgcaattg atcctaaaga tggtatcaac atctcccgcc cagacgaata 1800

tatacatatt ccactgtcgt attaaactag tgaagttcct atactttcta gagaatagga 1860

acttcggaat aggaacttca tgagtaaact tggtctgaca gttaccaatg cttaatcagt 1920

gaggcaccta tctcagcgat ctgtctattt cgttcatcca tagttgcctg actccccgtc 1980

gtgtagataa ctacgatacg ggagggctta ccatctggcc ccagtgctgc aatgataccg 2040

cgagacccac gctcaccggc tccagattta tcagcaataa accagccagc cggaagggcc 2100

gagcgcagaa gtggtcctgc aactttatcc gcctccatcc agtctattaa ttgttgccgg 2160

gaagctagag taagtagttc gccagttaat agtttgcgca acgttgttgc cattgctaca 2220

ggcatcgtgg tgtcacgctc gtcgtttggt atggcttcat tcagctccgg ttcccaacga 2280

tcaaggcgag ttacatgatc ccccatgttg tgcaaaaaag cggttagctc cttcggtcct 2340

ccgatcgttg tcagaagtaa gttggccgca gtgttatcac tcatggttat ggcagcactg 2400

cataattctc ttactgtcat gccatccgta agatgctttt ctgtgactgg tgagtactca 2460

accaagtcat tctgagaata gtgtatgcgg cgaccgagtt gctcttgccc ggcgtcaata 2520

cgggataata ccgcgccaca tagcagaact ttaaaagtgc tcatcattgg aaaacgttct 2580

tcggggcgaa aactctcaag gatcttaccg ctgttgagat ccagttcgat gtaacccact 2640

cgtgcaccca actgatcttc agcatctttt actttcacca gcgtttctgg gtgagcaaaa 2700

acaggaaggc aaaatgccgc aaaaaaggga ataagggcga cacggaaatg ttgaatactc 2760

atactcttcc tttttcaata ttattgaagc atttatcagg gttattgtct catgagcgga 2820

tacatatttg aatgtattta gaaaaataaa caaatagggg ttccgcgcac atttccccga 2880

gaagttccta tactttctag agaataggaa cttcggaata ggaacttcac tagtggacaa 2940

acagttacac gatatggtcg tgcgtgttga taccagcagt atgcagccaa tcgattctac 3000

ttgccggttc cagtacatac tgaatgagtt gtagccctta gatacaccag gcaggggcat 3060

tcgcccctgc cattacccgt acctggcgca acgcgctcaa ctcctcaggg caactccggc 3120

ggcagccact cttcgacttc aatcaacacc aacaacgccg catcaccgcc atactctttt 3180

ggtgcctgat gaaaggccat cacatgcgga tgttgtgcca gccacagcgg tgtttgttgc 3240

ttcaaaatat gcttcccgtg cccatgcatc acgcaggcgc aaaacacatg ttcacggcgg 3300

caggcggcaa tcaacgcccc cagttcctgc ttggcctgca gctgcgtcag accgtgtaaa 3360

tccaaaaaca actccggcga ataatcgcca cggcgcagtt tcttcgcctc aaaatggctg 3420

acatccgggc gaac 3434

<210> 6
<211> 69
<212> DNA
<213> Artificial Sequence

<220>
<223> >tac promoter

<400> 6
ttctgaaatg agctgttgac aattaatcat gggctcgtat aatgtgtgga attgtgagcg     60

gataacaat                                                             69


<210> 7
<211> 30
<212> PRT
<213> Artificial Sequence


<220>
<223> >Tir30

<400> 7
Met Pro Ile Gly Asn Leu Gly Asn Asn Val Asn Gly Asn His Leu Ile
1               5                   10                  15
Pro Pro Ala Pro Pro Leu Pro Ser Gln Thr Asp Gly Ala Ala
            20                  25                  30

<210> 8
<211> 100
<212> PRT
<213> Artificial Sequence


<220>
<223> Tir100

<400> 8
Met Pro Ile Gly Asn Leu Gly Asn Asn Val Asn Gly Asn His Leu Ile
1               5                   10                  15
Pro Pro Ala Pro Pro Leu Pro Ser Gln Thr Asp Gly Ala Ala Arg Gly
            20                  25                  30
Gly Thr Gly His Leu Ile Ser Ser Thr Gly Ala Leu Gly Ser Arg Ser
        35                  40                  45
Leu Phe Ser Pro Leu Arg Asn Ser Met Ala Asp Ser Val Asp Ser Arg
    50                  55                  60
Asp Ile Pro Gly Leu Pro Thr Asn Pro Ser Arg Leu Ala Ala Ala Thr
65                  70                  75                  80
Ser Glu Thr Cys Leu Leu Gly Gly Phe Glu Val Leu His Asp Lys Gly
                85                  90                  95
Pro Leu Asp Ile
            100

<210> 9
<211> 20
<212> PRT
<213> Artificial Sequence


<220>
<223> >EspF20

<400> 9
Met Leu Asn Gly Ile Ser Asn Ala Ala Ser Thr Leu Gly Arg Gln Leu
1               5                   10                  15
Val Gly Ile Ala
            20

<210> 10
<211> 156
<212> PRT
<213> Escherichia coli

<220>
<223> >CesT sequence protein

<400> 10
Met Ser Ser Arg Ser Glu Leu Leu Leu Asp Arg Phe Ala Glu Lys Ile
1               5                   10                  15
Gly Val Gly Ser Ile Ser Phe Asn Glu Asn Arg Leu Cys Ser Phe Ala
            20                  25                  30
Ile Asp Glu Ile Tyr Tyr Ile Ser Leu Ser Asp Ala Asn Asp Glu Tyr
        35                  40                  45
Met Met Ile Tyr Gly Val Cys Gly Lys Phe Pro Thr Asp Asn Pro Asn
    50                  55                  60
Phe Ala Leu Glu Ile Leu Asn Ala Asn Leu Trp Phe Ala Glu Asn Gly
65                  70                  75                  80
Gly Pro Tyr Leu Cys Tyr Glu Ser Gly Ala Gln Ser Leu Leu Leu Ala
                85                  90                  95
Leu Arg Phe Pro Leu Asp Asp Ala Thr Pro Glu Lys Leu Glu Asn Glu
            100                 105                 110
Ile Glu Val Val Val Lys Ser Met Glu Asn Leu Tyr Leu Val Leu His
        115                 120                 125
Asn Gln Gly Ile Thr Leu Glu Asn Glu His Met Lys Ile Glu Glu Ile
    130                 135                 140
Ser Ser Ser Asp Asn Lys His Tyr Tyr Ala Gly Arg
145                 150                 155

<210> 11
<211> 360
<212> PRT
<213> Escherichia coli

<220>
<223> > lacI protein

<400> 11
Met Lys Pro Val Thr Leu Tyr Asp Val Ala Glu Tyr Ala Gly Val Ser
1               5                   10                  15
Tyr Gln Thr Val Ser Arg Val Val Asn Gln Ala Ser His Val Ser Ala
            20                  25                  30
Lys Thr Arg Glu Lys Val Glu Ala Ala Met Ala Glu Leu Asn Tyr Ile
        35                  40                  45
Pro Asn Arg Val Ala Gln Gln Leu Ala Gly Lys Gln Ser Leu Leu Ile
    50                  55                  60
Gly Val Ala Thr Ser Ser Leu Ala Leu His Ala Pro Ser Gln Ile Val
65                  70                  75                  80
Ala Ala Ile Lys Ser Arg Ala Asp Gln Leu Gly Ala Ser Val Val Val
                85                  90                  95
Ser Met Val Glu Arg Ser Gly Val Glu Ala Cys Lys Ala Ala Val His
            100                 105                 110
Asn Leu Leu Ala Gln Arg Val Ser Gly Leu Ile Ile Asn Tyr Pro Leu
        115                 120                 125
Asp Asp Gln Asp Ala Ile Ala Val Glu Ala Ala Cys Thr Asn Val Pro
    130                 135                 140
Ala Leu Phe Leu Asp Val Ser Asp Gln Thr Pro Ile Asn Ser Ile Ile
145                 150                 155                 160
Phe Ser His Glu Asp Gly Thr Arg Leu Gly Val Glu His Leu Val Ala

                165                     170                     175
Leu Gly His Gln Gln Ile Ala Leu Leu Ala Gly Pro Leu Ser Ser Val
            180                     185                     190
Ser Ala Arg Leu Arg Leu Ala Gly Trp His Lys Tyr Leu Thr Arg Asn
        195                     200                     205
Gln Ile Gln Pro Ile Ala Glu Arg Glu Gly Asp Trp Ser Ala Met Ser
    210                     215                     220
Gly Phe Gln Gln Thr Met Gln Met Leu Asn Glu Gly Ile Val Pro Thr
225                     230                     235                     240
Ala Met Leu Val Ala Asn Asp Gln Met Ala Leu Gly Ala Met Arg Ala
                245                     250                     255
Ile Thr Glu Ser Gly Leu Arg Val Gly Ala Asp Ile Ser Val Val Gly
            260                     265                     270
Tyr Asp Asp Thr Glu Asp Ser Ser Cys Tyr Ile Pro Pro Leu Thr Thr
        275                     280                     285
Ile Lys Gln Asp Phe Arg Leu Leu Gly Gln Thr Ser Val Asp Arg Leu
    290                     295                     300
Leu Gln Leu Ser Gln Gly Gln Ala Val Lys Gly Asn Gln Leu Leu Pro
305                     310                     315                     320
Val Ser Leu Val Lys Arg Lys Thr Thr Leu Ala Pro Asn Thr Gln Thr
                325                     330                     335
Ala Ser Pro Arg Ala Leu Ala Asp Ser Leu Met Gln Leu Ala Arg Gln
            340                     345                     350
Val Ser Arg Leu Glu Ser Gly Gln
        355                     360

<210> 12
<211> 360
<212> PRT
<213> Artificial Sequence


<220>
<223> > lacI W220F protein

<400> 12
Met Lys Pro Val Thr Leu Tyr Asp Val Ala Glu Tyr Ala Gly Val Ser
1               5                   10                  15
Tyr Gln Thr Val Ser Arg Val Val Asn Gln Ala Ser His Val Ser Ala
            20                  25                  30
Lys Thr Arg Glu Lys Val Glu Ala Ala Met Ala Glu Leu Asn Tyr Ile
        35                  40                  45
Pro Asn Arg Val Ala Gln Gln Leu Ala Gly Lys Gln Ser Leu Leu Ile
    50                  55                  60
Gly Val Ala Thr Ser Ser Leu Ala Leu His Ala Pro Ser Gln Ile Val
65                  70                  75                  80
Ala Ala Ile Lys Ser Arg Ala Asp Gln Leu Gly Ala Ser Val Val Val
                85                  90                  95
Ser Met Val Glu Arg Ser Gly Val Glu Ala Cys Lys Ala Ala Val His
            100                 105                 110
Asn Leu Leu Ala Gln Arg Val Ser Gly Leu Ile Ile Asn Tyr Pro Leu
        115                 120                 125
Asp Asp Gln Asp Ala Ile Ala Val Glu Ala Ala Cys Thr Asn Val Pro
    130                 135                 140
Ala Leu Phe Leu Asp Val Ser Asp Gln Thr Pro Ile Asn Ser Ile Ile
145                 150                 155                 160
Phe Ser His Glu Asp Gly Thr Arg Leu Gly Val Glu His Leu Val Ala
                165                 170                 175
Leu Gly His Gln Gln Ile Ala Leu Leu Ala Gly Pro Leu Ser Ser Val
            180                 185                 190
Ser Ala Arg Leu Arg Leu Ala Gly Trp His Lys Tyr Leu Thr Arg Asn
        195                 200                 205
Gln Ile Gln Pro Ile Ala Glu Arg Glu Gly Asp Phe Ser Ala Met Ser

210                 215                 220
Gly Phe Gln Gln Thr Met Gln Met Leu Asn Glu Gly Ile Val Pro Thr
225                 230                 235                 240
Ala Met Leu Val Ala Asn Asp Gln Met Ala Leu Gly Ala Met Arg Ala
                245                 250                 255
Ile Thr Glu Ser Gly Leu Arg Val Gly Ala Asp Ile Ser Val Val Gly
            260                 265                 270
Tyr Asp Asp Thr Glu Asp Ser Ser Cys Tyr Ile Pro Pro Leu Thr Thr
        275                 280                 285
Ile Lys Gln Asp Phe Arg Leu Leu Gly Gln Thr Ser Val Asp Arg Leu
    290                 295                 300
Leu Gln Leu Ser Gln Gly Gln Ala Val Lys Gly Asn Gln Leu Leu Pro
305                 310                 315                 320
Val Ser Leu Val Lys Arg Lys Thr Thr Leu Ala Pro Asn Thr Gln Thr
                325                 330                 335
Ala Ser Pro Arg Ala Leu Ala Asp Ser Leu Met Gln Leu Ala Arg Gln
            340                 345                 350
Val Ser Arg Leu Glu Ser Gly Gln
        355                 360

<210> 13
<211> 49
<212> DNA
<213> Artificial Sequence

<220>
<223> > PR promoter

<400> 13
taacaccgtg cgtgttgact attttacctc tggcggtgat aatggttgc                49

<210> 14
<211> 56
<212> DNA
<213> Artificial Sequence

<220>
<223> >Ptet promoter (complete, bidirectional)

<400> 14
ttctctatca ctgataggga gtggtaaaat aactctatca atgatagagt gtcaac        56

<210> 15
<211> 207
<212> PRT
<213> Escherichia coli

<220>
<223> > TetR protein

<400> 15
Met Ser Arg Leu Asp Lys Ser Lys Val Ile Asn Ser Ala Leu Glu Leu
1               5                   10                  15
Leu Asn Glu Val Gly Ile Glu Gly Leu Thr Thr Arg Lys Leu Ala Gln
            20                  25                  30
Lys Leu Gly Val Glu Gln Pro Thr Leu Tyr Trp His Val Lys Asn Lys
        35                  40                  45
Arg Ala Leu Leu Asp Ala Leu Ala Ile Glu Met Leu Asp Arg His His

50                  55                  60
Thr His Phe Cys Pro Leu Glu Gly Glu Ser Trp Gln Asp Phe Leu Arg
65                  70                  75                  80
Asn Asn Ala Lys Ser Phe Arg Cys Ala Leu Leu Ser His Arg Asp Gly
                85                  90                  95
Ala Lys Val His Leu Gly Thr Arg Pro Thr Glu Lys Gln Tyr Glu Thr
            100                 105                 110
Leu Glu Asn Gln Leu Ala Phe Leu Cys Gln Gln Gly Phe Ser Leu Glu
        115                 120                 125
Asn Ala Leu Tyr Ala Leu Ser Ala Val Gly His Phe Thr Leu Gly Cys
    130                 135                 140
Val Leu Glu Asp Gln Glu His Gln Val Ala Lys Glu Glu Arg Glu Thr
145                 150                 155                 160
Pro Thr Thr Asp Ser Met Pro Pro Leu Leu Arg Gln Ala Ile Glu Leu
                165                 170                 175
Phe Asp His Gln Gly Ala Glu Pro Ala Phe Leu Phe Gly Leu Glu Leu
            180                 185                 190
Ile Ile Cys Gly Leu Glu Lys Gln Leu Lys Cys Glu Ser Gly Ser
        195                 200                 205

<210> 16
<211> 237
<212> PRT
<213> Enterobacteria phage lambda


<220>
<223> >cl protein

<400> 16

<210> 17
<211> 237

<212> PRT
<213> Enterobacteria phage lambda

<220>
<223> >cI+ ind- protein

<400> 17
Met Ser Thr Lys Lys Lys Pro Leu Thr Gln Glu Gln Leu Glu Asp Ala
1               5                   10                  15
Arg Arg Leu Lys Ala Ile Tyr Glu Lys Lys Lys Asn Glu Leu Gly Leu
            20                  25                  30
Ser Gln Glu Ser Val Ala Asp Lys Met Gly Met Gly Gln Ser Gly Val
        35                  40                  45
Gly Ala Leu Phe Asn Gly Ile Asn Ala Leu Asn Ala Tyr Asn Ala Ala
    50                  55                  60
Leu Leu Ala Lys Ile Leu Lys Val Ser Val Glu Glu Phe Ser Pro Ser
65                  70                  75                  80
Ile Ala Arg Glu Ile Tyr Glu Met Tyr Glu Ala Val Ser Met Gln Pro
                85                  90                  95
Ser Leu Arg Ser Glu Tyr Glu Tyr Pro Val Phe Ser His Val Gln Ala
            100                 105                 110
Gly Met Phe Ser Pro Lys Leu Arg Thr Phe Thr Lys Gly Asp Ala Glu
        115                 120                 125
Arg Trp Val Ser Thr Thr Lys Lys Ala Ser Asp Ser Ala Phe Trp Leu
    130                 135                 140
Glu Val Glu Gly Asn Ser Met Thr Ala Pro Thr Gly Ser Lys Pro Ser
145                 150                 155                 160
Phe Pro Asp Gly Met Leu Ile Leu Val Asp Pro Glu Gln Ala Val Glu
                165                 170                 175
Pro Gly Asp Phe Cys Ile Ala Arg Leu Gly Gly Asp Glu Phe Thr Phe
            180                 185                 190
Lys Lys Leu Ile Arg Asp Ser Gly Gln Val Phe Leu Gln Pro Leu Asn
        195                 200                 205
Pro Gln Tyr Pro Met Ile Pro Cys Asn Glu Ser Cys Ser Val Val Gly
    210                 215                 220
Lys Val Ile Ala Ser Gln Trp Pro Glu Glu Thr Phe Gly
225                 230                 235

<210> 18
<211> 103
<212> DNA
<213> Artificial Sequence

<220>
<223> > T0 terminator

<400> 18
cttggactcc tgttgataga tccagtaatg acctcagaac tccatctgga tttgttcaga      60

acgctcggtt gccgccgggc gttttttatt ggtgagaatc cag                         103

<210> 19
<211> 87
<212> DNA
<213> Artificial Sequence

<220>
<223> >T1 terminator

<400> 19
atttgtccta ctcaggagag cgttcaccga caaacaacag ataaaacgaa aggcccagtc     60

tttcgactga gcctttcgtt ttatttg                                         87


<210> 20
<211> 87
<212> DNA
<213> Artificial Sequence


<220>
<223> >RBS 0030 sequence

<400> 20
atttgtccta ctcaggagag cgttcaccga caaacaacag ataaaacgaa aggcccagtc     60

tttcgactga gcctttcgtt ttatttg                                         87


<210> 21
<211> 11
<212> PRT
<213> Artificial Sequence


<220>
<223> AAV-ssrA tag

<400> 21
Ala Ala Asn Asp Glu Asn Tyr Ala Ala Ala Val
1               5                   10

<210> 22
<211> 11
<212> PRT
<213> Artificial Sequence


<220>
<223> ASV-ssrA tag

<400> 22
Ala Ala Asn Asp Glu Asn Tyr Ala Ala Ala Val
1               5                   10

<210> 23
<211> 11
<212> PRT
<213> Artificial Sequence


<220>
<223> LVA-ssrA tag

<400> 23
Ala Ala Asn Asp Glu Asn Tyr Ala Leu Val Ala
1               5                   10

<210> 24
<211> 11
<212> PRT

<213> Artificial Sequence

<220>
<223> LAA-ssrA tag

<400> 24
Ala Ala Asn Asp Glu Asn Tyr Ala Leu Ala Ala
1               5                   10

<210> 25
<211> 825
<212> PRT
<213> Artificial Sequence

<220>
<223> Synthetic adhesin full

<400> 25
Met Ile Thr His Gly Cys Tyr Thr Arg Thr Arg His Lys His Lys Leu
1               5                   10                  15
Lys Lys Thr Leu Ile Met Leu Ser Ala Gly Leu Gly Leu Phe Phe Tyr
            20                  25                  30
Val Asn Gln Asn Ser Phe Ala Asn Gly Glu Asn Tyr Phe Lys Leu Gly
        35                  40                  45
Ser Asp Ser Lys Leu Leu Thr His Asp Ser Tyr Gln Asn Arg Leu Phe
    50                  55                  60
Tyr Thr Leu Lys Thr Gly Glu Thr Val Ala Asp Leu Ser Lys Ser Gln
65                  70                  75                  80
Asp Ile Asn Leu Ser Thr Ile Trp Ser Leu Asn Lys His Leu Tyr Ser
                85                  90                  95
Ser Glu Ser Glu Met Met Lys Ala Ala Pro Gly Gln Gln Ile Ile Leu
            100                 105                 110
Pro Leu Lys Lys Leu Pro Phe Glu Tyr Ser Ala Leu Pro Leu Leu Gly
        115                 120                 125
Ser Ala Pro Leu Val Ala Ala Gly Gly Val Ala Gly His Thr Asn Lys
    130                 135                 140
Leu Thr Lys Met Ser Pro Asp Val Thr Lys Ser Asn Met Thr Asp Asp
145                 150                 155                 160
Lys Ala Leu Asn Tyr Ala Ala Gln Gln Ala Ala Ser Leu Gly Ser Gln
                165                 170                 175
Leu Gln Ser Arg Ser Leu Asn Gly Asp Tyr Ala Lys Asp Thr Ala Leu
            180                 185                 190
Gly Ile Ala Gly Asn Gln Ala Ser Ser Gln Leu Gln Ala Trp Leu Gln
        195                 200                 205
His Tyr Gly Thr Ala Glu Val Asn Leu Gln Ser Gly Asn Asn Phe Asp
    210                 215                 220
Gly Ser Ser Leu Asp Phe Leu Leu Pro Phe Tyr Asp Ser Glu Lys Met
225                 230                 235                 240
Leu Ala Phe Gly Gln Val Gly Ala Arg Tyr Ile Asp Ser Arg Phe Thr
                245                 250                 255
Ala Asn Leu Gly Ala Gly Gln Arg Phe Phe Leu Pro Ala Asn Met Leu
            260                 265                 270
Gly Tyr Asn Val Phe Ile Asp Gln Asp Phe Ser Gly Asp Asn Thr Arg
        275                 280                 285
Leu Gly Ile Gly Gly Glu Tyr Trp Arg Asp Tyr Phe Lys Ser Ser Val
    290                 295                 300
Asn Gly Tyr Phe Arg Met Ser Gly Trp His Glu Ser Tyr Asn Lys Lys
305                 310                 315                 320
Asp Tyr Asp Glu Arg Pro Ala Asn Gly Phe Asp Ile Arg Phe Asn Gly
                325                 330                 335
Tyr Leu Pro Ser Tyr Pro Ala Leu Gly Ala Lys Leu Ile Tyr Glu Gln

340                 345                 350
Tyr Tyr Gly Asp Asn Val Ala Leu Phe Asn Ser Asp Lys Leu Gln Ser
        355                 360                 365
Asn Pro Gly Ala Ala Thr Val Gly Val Asn Tyr Thr Pro Ile Pro Leu
    370                 375                 380
Val Thr Met Gly Ile Asp Tyr Arg His Gly Thr Gly Asn Glu Asn Asp
385                 390                 395                 400
Leu Leu Tyr Ser Met Gln Phe Arg Tyr Gln Phe Asp Lys Ser Trp Ser
                405                 410                 415
Gln Gln Ile Glu Pro Gln Tyr Val Asn Glu Leu Arg Thr Leu Ser Gly
            420                 425                 430
Ser Arg Tyr Asp Leu Val Gln Arg Asn Asn Asn Ile Ile Leu Glu Tyr
        435                 440                 445
Lys Lys Gln Asp Ile Leu Ser Leu Asn Ile Pro His Asp Ile Asn Gly
    450                 455                 460
Thr Glu His Ser Thr Gln Lys Ile Gln Leu Ile Val Lys Ser Lys Tyr
465                 470                 475                 480
Gly Leu Asp Arg Ile Val Trp Asp Asp Ser Ala Leu Arg Ser Gln Gly
                485                 490                 495
Gly Gln Ile Gln His Ser Gly Ser Gln Ser Ala Gln Asp Tyr Gln Ala
            500                 505                 510
Ile Leu Pro Ala Tyr Val Gln Gly Gly Ser Asn Ile Tyr Lys Val Thr
        515                 520                 525
Ala Arg Ala Tyr Asp Arg Asn Gly Asn Ser Ser Asn Asn Val Gln Leu
    530                 535                 540
Thr Ile Thr Val Leu Ser Asn Gly Gln Val Val Asp Gln Val Gly Val
545                 550                 555                 560
Thr Asp Phe Thr Ala Asp Lys Thr Ser Ala Lys Ala Asp Asn Ala Asp
                565                 570                 575
Thr Ile Thr Tyr Thr Ala Thr Val Lys Lys Asn Gly Val Ala Gln Ala
            580                 585                 590
Asn Val Pro Val Ser Phe Asn Ile Val Ser Gly Thr Ala Thr Leu Gly
        595                 600                 605
Ala Asn Ser Ala Lys Thr Asp Ala Asn Gly Lys Ala Thr Val Thr Leu
    610                 615                 620
Lys Ser Ser Thr Pro Gly Gln Val Val Val Ser Ala Lys Thr Ala Glu
625                 630                 635                 640
Met Thr Ser Ala Leu Asn Ala Ser Ala Val Ile Phe Phe Asp Gly Ala
                645                 650                 655
Pro Val Pro Tyr Pro Asp Pro Leu Glu Pro Ala Gln Pro Ala Met Ala
            660                 665                 670
Gln Val Gln Leu Gln Glu Ser Gly Gly Gly Leu Val Gln Ala Gly Gly
        675                 680                 685
Ser Leu Leu Leu Ser Cys Ala Ala Ser Gly Arg Thr Phe Ser Ser Tyr
    690                 695                 700
Ala Met Gly Trp Phe Arg Gln Ala Pro Gly Lys Glu Arg Glu Phe Val
705                 710                 715                 720
Ala Ala Ile Asn Trp Ser Gly Gly Ser Thr Ser Tyr Ala Asp Ser Val
                725                 730                 735
Lys Gly Arg Phe Thr Ile Ser Arg Asp Asn Thr Lys Asn Thr Val Tyr
            740                 745                 750
Leu Gln Met Asn Ser Leu Lys Pro Glu Asp Thr Ala Ala Phe Tyr Cys
        755                 760                 765
Ala Ala Thr Tyr Asn Pro Tyr Ser Arg Asp His Tyr Phe Pro Arg Met
    770                 775                 780
Thr Thr Glu Tyr Asp Tyr Trp Gly Gln Gly Thr Gln Val Thr Val Ser
785                 790                 795                 800
Ser Glu Pro Lys Thr Pro Lys Pro Gln Pro Ala Ala Ala Glu Gln Lys
                805                 810                 815
Leu Ile Ser Glu Glu Asp Ala Ala Ala
            820                 825

<210> 26

<211> 5384
<212> DNA
<213> Artificial Sequence

<220>
<223> >Regulatory circuit complete

<400> 26
ccggtctcga ggtgactgga aactggtgtt accttcgctg gcgcttgggc tgatatttgg 60

ttacgcaatg aaaaacagtg gcctgtggct ggcggcgcgt agtgcaaaga cggcgcaccg 120

tgagcaggaa atcaaaaata aagcgtgagg ggcactcacg ctttcgctta aacagtaaaa 180

tgccggatga taattccggc ttttttatct gtcaggattc cggtggaacc gacatatggc 240

ggtatttcac cagaatgtca ttctgccgtt ctgctttatt ttgcaaatcc cacagaccac 300

ggtcgatacc atcattaatc aggaaaatga cccctgtttc gatagccgac atcaggcaca 360

gcataacagg ttcgttcgag gtgtaaccca cttccccttc aagcaagcgc tggtagtcaa 420

taaagcggaa aaccccggcc tgaacttcat aggaaagtat cgtcttactg gtgttcaccg 480

aagaaaggat ctcgccggta ctcacattga cgacgcgcag gttcacggca atctgagagc 540

tccaaataaa acgaaaggct cagtcgaaag actgggcctt tcgttttatc tgttgtttgt 600

cggtgaacgc tctcctgagt aggacaaatg gatcctcagc caaacgtctc ttcaggccac 660

tgactagcga taactttccc cacaacggaa caactctcat tgcatgggat cattgggtac 720

tgtgggttta gtggttgtaa aaacacctga ccgctatccc tgatcagttt cttgaaggta 780

aactcatcac ccccaagtct ggctatgcag aaatcacctg gctcaacagc ctgctcaggg 840

tcaacgagaa ttaacattcc gtcaggaaag cttggcttgg agcctgttgg tgcggtcatg 900

gaattacctt caacctcaag ccagaatgca gaatcactgg cttttttggt tgtgcttacc 960

catctctccg catcaccttt ggtaaaggtt ctaagcttag gtgagaacat ccctgcctga 1020

acatgagaaa aaacagggta ctcatactca cttctaagtg acggctgcat actaaccgct 1080

tcatacatct cgtagatttc tctggcgatt gaagggctaa attcttcaac gctaactttg 1140

agaatttttg caagcaatgc ggcgttataa gcatttaatg cattgatgcc attaaataaa 1200

gcaccaacgc ctgactgccc catccccatc ttgtctgcga cagattcctg ggataagcca 1260

agttcatttt tctttttttc ataaattgct ttaaggcgac gtgcgtcctc aagctgctct 1320

tgtgttaatg gtttcttttt tgtgctcatc tagtatttct cctctttaat ctgcagttct 1380

ctatcactga tagggagtgg taaaataact ctatcaatga tagagtgtca acaaaaatta 1440

ggaattaatg atgtccagat tagataaaag taaagtgatt aacagcgcat tagagctgct 1500

taatgaggtc ggaatcgaag gtttaacaac ccgtaaactc gcccagaagc taggtgtaga 1560

gcagcctaca ttgtattggc atgtaaaaaa taagcgggct ttgctcgacg ccttagccat 1620

tgagatgtta gataggcacc atactcactt ttgcccttta gaaggggaaa gctggcaaga 1680

ttttttacgt aataacgcta aaagttttag atgtgcttta ctaagtcatc gcgatggagc 1740

aaaagtacat ttaggtacac ggcctacaga aaaacagtat gaaactctcg aaaatcaatt 1800

agccttttta tgccaacaag gtttttcact agagaatgca ttatatgcac tcagcgctgt 1860

ggggcatttt actttaggtt gcgtattgga agatcaagag catcaagtcg ctaaagaaga 1920

aagggaaaca cctactactg atagtatgcc gccattatta cgacaagcta tcgaattatt 1980

tgatcaccaa ggtgcagagc cagccttctt attcggcctt gaattgatta tatgcggatt 2040

agaaaaacaa cttaaatgtg aaagtgggtc ttaagtcgac cttggactcc tgttgataga 2100

tccagtaatg acctcagaac tccatctgga tttgttcaga acgctcggtt gccgccgggc 2160

gttttttatt ggtgagaatc cagccgcggt aacaccgtgc gtgttgacta ttttacctct 2220

ggcggtgata atggttgcga attcattaaa gaggagaaat actagatgaa accagtaacg 2280

ttatacgatg tcgcagagta tgccggtgtc tcttatcaga ccgtttcccg cgtggtgaac 2340

caggccagcc acgtttctgc gaaaacgcgg gaaaaagtgg aagcggcgat ggcggagctg 2400

aattacattc ccaaccgcgt ggcacaacaa ctggcgggca aacaaagctt gctgattggc 2460

gttgccacct ccagtctggc cctgcacgcg ccgtcgcaaa ttgtcgcggc gattaaatct 2520

cgcgccgatc aactgggtgc cagcgtggtg gtgtcgatgg tagaacgaag cggcgtcgaa 2580

gcctgtaaag cggcggtgca caatcttctc gcgcaacgcg tcagtgggct gatcattaac 2640

tatccgctgg atgaccagga tgccattgct gtggaagctg cctgcactaa tgttccggcg 2700

ttatttcttg atgtctctga ccagacaccc atcaacagta ttattttctc ccatgaagac 2760

ggtacgcgac tgggcgtgga gcatctggtc gcattgggtc accagcaaat cgcgctgtta 2820

gcgggcccat taagttctgt ctcggcgcgt ctgcgtctgg ctggctggca taaatatctc 2880

actcgcaatc aaattcagcc gatagcggaa cgggaaggcg acttcagtgc catgtccggt 2940

tttcaacaaa ccatgcaaat gctgaatgag ggcatcgttc ccactgcgat gctggttgcc 3000

aacgatcaga tggcgctggg cgcaatgcgc gccattaccg agtccgggct gcgcgttggt 3060

gcggatatct cggtagtggg atacgacgat accgaagaca gctcatgtta tatcccgccg 3120

ttaaccacca tcaaacagga ttttcgcctg ctggggcaaa ccagcgtgga ccgcttgctg 3180

caactctctc agggccaggc ggtgaagggc aatcagctgt tgcccgtctc actggtgaaa 3240

agaaaaacca cgctagcacc caatacgcaa accgcctctc cccgcgcgtt ggccgattca 3300

ttaatgcagc tggcacgaca ggtttcccga ctggaaagcg ggcaggcagc aaacgacgaa 3360

aactacgctg catcagtttg aggtaccgcg caacgcaatt aatgtgagtt agctcactca 3420

ttaggcaccc caggcactag tctgcgcgaa ggcagttcag ggcaaagtca gacgaagcaa 3480

gaaaagaccc tttcattgcc tgctaatcaa cccattgctt tgacgaagtt gagtttaaat 3540

atttccccgg acgatcgggt gaaaatagtt gttactgttt ctgatggaca gtcacttcat 3600

ttatcacaac aatggccgcc ctcttcagaa aagtcttaat ttgttgaaat atcgagcata 3660

agatgaatct ggagagaatg gtctgctgcg aatcagccaa cctgaaagta tggataacac 3720

aaccctcaag gatgactaat cattgaggaa atagaataaa tgttcagacc ttttttaaac 3780

tctcttatgc tcggcagttt gttttttcct tttattgcca ttgctggaag caccgtgcaa 3840

gggggcgtga tccattttta tggccaaatt gtggaaccgg catgtgacgt cagcacccag 3900

tcatcacccg tagaaatgaa ctgcccacag aatggttcta ttccgggcaa aacctactcc 3960

agcaaagcgt taatgagcgg caatgtcaaa aacgcgcaaa tagcctcagg catgcaccgg 4020

gattaccctg ttatccctat tattagaaaa attcatccag catcagatga aattgcagtt 4080

tgttcatatc cggattatca atgccatatt tctgaaacag acgtttttgc aggctcgggc 4140

taaattcgcc caggcagttc cacagaatgg ccagatcctg ataacgatcc gcaatgccca 4200

cacggcccac atcaatgcag ccaatcagtt tgccttcatc gaaaatcagg ttatccaggc 4260

taaaatcgcc gtgggtcacc acgctatccg ggctaaacgg cagcagttta tgcatttctt 4320

tccacacctg ttccaccggc cagccgttac gttcatcatc aaaatcgctc gcatccacca 4380

ggccgttgtt catacggctc tgcgcctggg ccagacgaaa cacacgatcg ctgttaaacg 4440

ggcagttgca caccggaatg ctatgcagac gacgcagaaa cacggccagc gcatccacaa 4500

tgttttcgcc gctatccgga tattcttcca gcacctgaaa cgcggttttg cccggaatcg 4560

cggtggtcag cagccacgca tcatccgggg tgcgaataaa atgtttaatg gtcggcagcg 4620

gcataaattc ggtcagccag ttcagacgca ccatttcatc ggtcacatcg ttcgccacgc 4680

tgcctttgcc atgtttcaga aacagttccg gcgcatccgg tttgccatac agacgataaa 4740

tggtcgcgcc gctctgaccc acgttatcac gcgcccattt atagccatac agatccgcat 4800

ccatgttgct gttcagacgc ggacggctac agctcgtttc acgctgaata tggctcataa 4860

caccccttgt attactgttt atgtaagcag acagttttat tgttcatgat gatatatttt 4920

tatcttgtgc aatgtaacat cagagatttt gagacacaag acgtcggcgc gccgatctga 4980

agatcagcag ttcaacctgt tgatagtacg tactaagctc tcatgtttca cgtactaagc 5040

tctcatgttt aacgtactaa gctctcatgt ttaacgaact aaaccctcat ggctaacgta 5100

ctaagctctc atggctaacg tactaagctc tcatgtttca cgtactaagc tctcatgttt 5160

gaacaataaa attaatataa atcagcaact taaatagcct ctaaggtttt aagttttata 5220

agaaaaaaaa gaatatataa ggcttttaaa gcctttaagg tttaacggtt gtggacaaca 5280

agccagggat gtaacgcact gagaagccct tagagcctct caaagcaatt ttgagtgaca 5340

caggaacact taacggctga catggattac cctgttatcc ctac 5384

<210> 27
<211> 57
<212> DNA
<213> Artificial Sequence

<220>
<223> >F_XbaI_RBS_MAP

<400> 27
tcgagtctag aaagaaggag atatacatat gtttagtcca acggcaatgg taggtag          57

<210> 28
<211> 37
<212> DNA
<213> Artificial Sequence

<220>
<223> R_NotI_MAP

<400> 28
ccgatgcggc cgccagccga gtatcctgca cattgtc                                37

<210> 29
<211> 27
<212> DNA
<213> Artificial Sequence

<220>
<223> >pjA_5'

<400> 29
agctgtgcga acgtggtatt aactttc                                           27

<210> 30
<211> 27
<212> DNA
<213> Artificial Sequence

<220>
<223> ypjA_3'

<400> 30
aacaacacta tggcctgaca ctgaacg                                           27

<210> 31
<211> 25
<212> DNA
<213> Artificial Sequence

<220>
<223> >R_AraC_test

<400> 31

cgatcaactc tatttctcgc gggta                                                25

<210> 32
<211> 27
<212> DNA
<213> Artificial Sequence

<220>
<223> F_CesT_test

<400> 32
gtgggccata cctgtgttat gagtcag                                              27

<210> 33
<211> 47
<212> DNA
<213> Artificial Sequence

<220>
<223> F_SacI_RBS_CesT

<400> 33
aattcgagct caagaaggag atatacatat gtcatcaaga tccgaac                        47

<210> 34
<211> 36
<212> DNA
<213> Artificial Sequence

<220>
<223> F_SfiI_Gly_ZF6x

<400> 34
attgcggccc agccggccgg aaccggggcc gagcgc                                    36

<210> 35
<211> 34
<212> DNA
<213> Artificial Sequence

<220>
<223> >R_NotI_ZF6x

<400> 35
ccgatgcggc cgcgactagt tgtgagccgc cgcc                                      34

<210> 36
<211> 41
<212> DNA
<213> Artificial Sequence

<220>
<223> F_SfiI_GranzymeB

<400> 36
attgcggccc agccggccat gattattggc ggccatgaag c                41

<210> 37
<211> 43
<212> DNA
<213> Artificial Sequence

<220>
<223> R_NotI_GranzymeB

<400> 37
ccgatgcggc cgcatagcgt ttcatggttt ttttaatcca atg              43

<210> 38
<211> 36
<212> DNA
<213> Artificial Sequence

<220>
<223> >F_SfiI_OVA

<400> 38
attgcggccc agccggccat gggcagcatt ggcgcg                      36

<210> 39
<211> 29
<212> DNA
<213> Artificial Sequence

<220>
<223> R_NotI_OVA

<400> 39
ccgatgcggc cgccgggctc acgcagcgg                              29

<210> 40
<211> 40
<212> DNA
<213> Artificial Sequence

<220>
<223> F_SfiI_TK

<400> 40
attgcggccc agccggccat ggcttcgtac ccctgccatc                  40

<210> 41
<211> 34
<212> DNA
<213> Artificial Sequence

<220>
<223> R_NotI_TK

<400> 41
ccgatgcggc cgcgttagcc tcccccatct cccg                                    34


<210> 42
<211> 63
<212> DNA
<213> Artificial Sequence


<220>
<223> >F_SacI_RBS_EspF20

<400> 42
aattcgagct caagcttaag aaggagatat acatatgctt aatggaatta gtaacgctgc      60

ttc                                                                      63


<210> 43
<211> 38
<212> DNA
<213> Artificial Sequence


<220>
<223> >R_SpeI_Bla

<400> 43
catgcactag tttaccaatg cttaatcagt gaggcacc                                38


<210> 44
<211> 32
<212> DNA
<213> Artificial Sequence


<220>
<223> R_NotI_BIM

<400> 44
ccgatgcggc cgcatgcatg cgccacacca gg                                      32


<210> 45
<211> 37
<212> DNA
<213> Artificial Sequence


<220>
<223> F_SfiI_BIM

<400> 45
ggtgcggccc agccggccgc gaaacagccg agcgatg                                 37


<210> 46
<211> 53

<212> DNA
<213> Artificial Sequence

<220>
<223> >F_SfiI_Cas9

<400> 46
ggtgcggccc agccggccat ggataagaaa tactcaatag gcttagatat cgg 53

<210> 47
<211> 41
<212> DNA
<213> Artificial Sequence

<220>
<223> R_NotI_Cas9

<400> 47
ccgatgcggc cgcgtcacct cctagctgac tcaaatcaat g 41

<210> 48
<211> 41
<212> DNA
<213> Artificial Sequence

<220>
<223> >F_SfiI_Sox2

<400> 48
ggtgcggccc agccggccat gatggagacg gagctgaagc c 41

<210> 49
<211> 33
<212> DNA
<213> Artificial Sequence

<220>
<223> >R_NotI_Sox2

<400> 49
ccgatgcggc cgccatgtgc gacaggggca gtg 33

<210> 50
<211> 40
<212> DNA
<213> Artificial Sequence

<220>
<223> >F_SfiI_Oct4

<400> 50
ggtgcggccc agccggccat ggctggacac ctggcttcag 40

<210> 51
<211> 35
<212> DNA
<213> Artificial Sequence

<220>
<223> R_NotI_Oct4

<400> 51
ccgatgcggc cgcgtttgaa tgcatgggag agccc 35

<210> 52
<211> 21
<212> DNA
<213> Artificial Sequence

<220>
<223> >R_cargo_seq

<400> 52
gcgtcgaagc atggtgatgg t 21

<210> 53
<211> 24
<212> DNA
<213> Artificial Sequence

<220>
<223> 3' del LacI confirm

<400> 53
attgatggtg aacatgatgc cgac 24

<210> 54
<211> 39
<212> DNA
<213> Artificial Sequence

<220>
<223> R_NotI_tBID

<400> 54
ccgatgcggc cgcatccata ccattacgtg ccaggctac 39

<210> 55
<211> 24
<212> DNA
<213> Artificial Sequence

<220>
<223> >5prima_SReginteg

<400> 55
cgtttggctg aggatccatt tgtc 24

<210> 56
<211> 19
<212> DNA
<213> Artificial Sequence

<220>
<223> F_LacI_seq

<400> 56
gcgtctgcgt ctggctggc                                                    19

<210> 57
<211> 31
<212> DNA
<213> Artificial Sequence

<220>
<223> R_3prima de LacI

<400> 57
ctaatgagtg agctaactca cattaattgc g                                      31

<210> 58
<211> 58
<212> DNA
<213> Artificial Sequence

<220>
<223> >F_EcoRI_RBS0034_LacI

<400> 58
gttgcgaatt caaagaggag aaatactaga tgaaaccagt aacgttatac gatgtcgc        58

<210> 59
<211> 66
<212> DNA
<213> Artificial Sequence

<220>
<223> >R_KpnI_ssrAtag(LAA)_LacI

<400> 59
tgcgcggtac ctcaagcagc cagagcgtag ttttcgtcgt tagcagcctg cccgctttcc      60

agtcgg                                                                  66

<210> 60
<211> 44
<212> DNA
<213> Artificial Sequence

<220>

<223> F_EcoRI_RBS0034_LacI (NEW)

<400> 60
gttgcgaatt caaagaggag aaatactaga tgaaaccagt aacg                        44


<210> 61
<211> 44
<212> DNA
<213> Artificial Sequence


<220>
<223> >F_EcoRI_RBS0034_(GTG)LacI

<400> 61
gttgcgaatt caaagaggag aaatactagg tgaaaccagt aacg                        44


<210> 62
<211> 61
<212> DNA
<213> Artificial Sequence


<220>
<223> >F_EcoRI_RBSWT_(GTG)LacI

<400> 62
gttgcgaatt cagtcaattc agggtggtga atgtgaaacc agtaacgtta tacgatgtcg      60

c                                                                       61


<210> 63
<211> 32
<212> DNA
<213> Artificial Sequence


<220>
<223> R_KpnI_LacI

<400> 63
tgcgcggtac ctcactgccc gctttccagt cg                                     32


<210> 64
<211> 66
<212> DNA
<213> Artificial Sequence


<220>
<223> R_KpnI_ssrAtag(ASV)_LacI

<400> 64
tgcgcggtac ctcaaactga tgcagcgtag ttttcgtcgt ttgctgcctg cccgctttcc      60

agtcgg                                                                  66


<210> 65

<211> 66
<212> DNA
<213> Artificial Sequence

<220>
<223> R_KpnI_ssrAtag(AAV)_LacI

<400> 65
tgcgcggtac ctcaaactgc tgcagcgtag ttttcgtcgt ttgctgcctg cccgctttcc 60

agtcgg 66

<210> 66
<211> 33
<212> DNA
<213> Artificial Sequence

<220>
<223> F_SfiI_PE25

<400> 66
ggtgcggccc agccggccgg cccggcggac agc 33

<210> 67
<211> 31
<212> DNA
<213> Artificial Sequence

<220>
<223> R_NotI_PE

<400> 67
ccgatgcggc cgccttcagg tcctcgcgcg g 31

<210> 68
<211> 36
<212> DNA
<213> Artificial Sequence

<220>
<223> R_BglII_STOP_Tir

<400> 68
ttcttagatc tttaaacgaa acgtactggt cccggc 36

<210> 69
<211> 34
<212> DNA
<213> Artificial Sequence

<220>
<223> R_BglII_STOP_PE25

<400> 69

ttcttagatc ttcacttcag gtcctcgcgc ggcg 34

<210> 70
<211> 42
<212> DNA
<213> Artificial Sequence

<220>
<223> F_SfiI_TccC3

<400> 70
ggtgcggccc agccggccat gccgaccatt gcagaacgta tt 42

<210> 71
<211> 40
<212> DNA
<213> Artificial Sequence

<220>
<223> R_BglII_STOP_TccC3

<400> 71
ttcttagatc ttcaacgttt atgcggtttc acatttttca 40